(19) Europäisches Patentamt
European Patent Office
Office européen des brevets

(11) EP 3 442 526 B1

(12) # EUROPEAN PATENT SPECIFICATION

(45) Date of publication and mention of the grant of the patent:
**04.01.2023 Bulletin 2023/01**

(21) Application number: **17727439.6**

(22) Date of filing: **16.05.2017**

(51) International Patent Classification (IPC):
***A61K 31/425*** *(2006.01)* ***A61K 31/41*** *(2006.01)*
***A61P 31/04*** *(2006.01)*

(52) Cooperative Patent Classification (CPC):
**A61K 31/422; A61K 31/41; A61K 31/425; A61P 17/00; A61P 17/10; A61P 31/00; A61P 31/04**

(86) International application number:
**PCT/US2017/032805**

(87) International publication number:
**WO 2017/200979 (23.11.2017 Gazette 2017/47)**

(54) **TOPICAL FORMULATIONS OF BIARYL HETEROCYCLIC COMPOUNDS AND METHODS OF USE THEREOF**

TOPISCHE FORMULIERUNGEN VON BIARYLHETEROCYCLISCHEN VERBINDUNGEN UND VERFAHREN ZUR VERWENDUNG DAVON

FORMULATIONS TOPIQUES DE COMPOSÉS HÉTÉROCYCLIQUES BIARYLES ET LEURS PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.05.2016 US 201662337636 P**

(43) Date of publication of application:
**20.02.2019 Bulletin 2019/08**

(73) Proprietor: **Melinta Subsidiary Corp.**
**Morristown, NJ 07960 (US)**

(72) Inventors:
- **JOHNSON, Keith, Arthur**
  **Durham, NC 27707 (US)**
- **DUFFY, Erin, M.**
  **Deep River, CT 06417 (US)**

(74) Representative: **Bassil, Nicholas Charles et al**
**Kilburn & Strode LLP**
**Lacon London**
**84 Theobalds Road**
**London WC1X 8NL (GB)**

(56) References cited:
**WO-A2-2006/133397 WO-A2-2015/085143**
**US-A1- 2014 100 254**

- **JOYCE A. SUTCLIFFE: "Antibiotics in development targeting protein synthesis", ANNALS OF THE NEW YORK ACADEMY OF SCIENCES, vol. 1241, no. 1, 22 December 2011 (2011-12-22), pages 122-152, XP55120873, ISSN: 0077-8923, DOI: 10.1111/j.1749-6632.2011.06323.x**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 442 526 B1

## Description

## RELATED APPLICATION

The present application claims the benefit of priority to U.S. Provisional Application No. 62/337,636, filed May 17, 2016.

## FIELD OF THE INVENTION

The present invention relates to topical formulations of a biaryl heterocyclic compound, radezolid, for treating, preventing, or reducing the risk of acne and other skin infections caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient in need thereof. In some embodiments, the acne or other skin infection is caused or mediated by *Propionibacterium acnes, Gardnerella vaginalis,* or *Staphylococcus aureus.*

## BACKGROUND

Acne is a common inflammatory pilosebaceous disease characterized by comedones, papules, pustules, inflamed nodules, superficial pus-filled cysts, and in extreme cases, sinus formation and deep inflammation, sometimes associated with purulent sacs.

The pathogenesis of acne is complex. An interaction between hormones, keratinization, sebum, and bacteria somehow determines the course and severity of the disease. Acne usually begins at puberty when the increase of androgens causes an increase in the size and activity of the pilosebaceous glands. The earliest microscopic change is intrafollicular hyperkeratosis, which leads to restriction of the pilosebaceous follicle with consequent formation of the comedone composed of sebum, keratin, and microorganisms, particularly *Propionibacterium acnes.* Lipases from *Propionibacterium acnes* break down triglycerides in the sebum to form free fatty acids (FFA), which irritate the follicular wall. Retention of sebaceous secretions and dilation of the follicle may lead to cyst formation. Rupture of the follicle with release of the contents into the tissues induces an inflammatory reaction which heals with scarring in severe cases.

Acne tends to appear during puberty and to fade away again, usually spontaneously when growth has stopped. Occasionally it is still to be found in older adults. The face, back, and shoulders are the predominant areas affected. Particularly with the face, severe cases can cause alterations resulting in considerable disfigurement with significant psychological burdens for the afflicted person.

Over the years, many compositions have been developed for the treatment or prevention of acne. For example, acne can be treated by topical application of various lotions, salves, and the like or by, for example, localized treatment with sulfur, resorcinol, salicylic acid, benzoyl peroxide, vitamin A acids, retinoic acid, antibiotics such as erythromycin, and the like. Acne has also been treated orally with antibiotics and tretinoin. However, the effectiveness, reliability, and convenience of current treatments have not always met with patient expectations.

Salicylic acid is a long-known anti-acne active ingredient which is believed to cause a reduction in intercellular cohesion of the corneocytes (*see* C. Huber et al., Arch. Derm. Res. 257, pp. 293-297, 1977). It has also been postulated that salicylic acid works by dissolving the existing keratin plugs, as well as by preventing the formation of new ones. In order to best exert its skin benefits, the ideal anti-acne composition should deliver and retain optimal concentrations of salicylic acid in the stratum corneum with less penetration through the skin and into the general circulation. Also, compliance by the user to a regimen of treatment involving repeated applications is important. However, salicylic acid tends to be somewhat drying and irritating and can often cause peeling, thereby causing individuals to refrain from using salicylic acid products as frequently and copiously as is necessary to obtain an optimum benefit. Thus, user compliance with current salicylic acid compositions is less than ideal.

Benzoyl peroxide has been used as a keratolytic agent and an antibacterial agent in the topical treatment of skin lesions such as acne. *See e.g.,* Levine et al., Ohio State Med. J., 65, 492 (1969); U.S. Pat. No. 3,535,422, to Cox et al., issued Oct. 20, 1970; British Patent Application Nos. 1,185,685, to Fisher, published Mar. 25, 1970; 1,163,004, to Stiefel Laboratories, Inc., published Sep. 4, 1969; and 1,407,937, to Stiefel Laboratories, Inc. published Oct. 1, 1975. The topical application of benzoyl peroxide for skin lesion therapy is thoroughly detailed in the medical literature. *See* Brogdne et al., Drugs, 4, 417 (1974); Poole et al., Arch Derm., 102, 400 (1972); Eaglstein, Arch Derm., 97, 527 (1968); Pace, Can. Med. Assoc. J., 93,252 (1965); Vasarinsh, Arch. Derm., 98, 183 ( 1968); Mysliborski et al., AFP, 15, 86 (1977); Nare, Br. J. Clin. Prac,, 29, 63 (1975); Fulton et al., Arch. Derm., 1, 10, 83 (1974); and Wilkinson et al., Can. Med. Assoc. J., 95, 28 (1966).

While benzoyl peroxide can be a useful topical treatment of skin lesions from acne, seborrhea, and other conditions, it has the undesirable side effect of being a contact irritant. The irritation associated with benzoyl peroxide

therapy has also been detailed in the medical literature cited in the previous paragraph. Additionally, the redness induced by benzoyl peroxide may impair a patient's ability to perceive the improvement in acne condition initially. Accordingly, some patients are denied the benefits of benzoyl peroxide therapy because of the irritation problem. When used in the treatment of acne, benzoyl peroxide produces dryness, exfoliation, increased redness and a decrease in bacterial flora.

Other agents, such as retinoic acid, are used for the treatment of acne. However, retinoic acid can be extremely drying and irritating when applied topically and can adversely affect the structure of the skin. Also, retinoic acid can be administered orally. However, retinoic acid can be teratogenic and have other undesired side effects.

Antibiotic agents, such as erythromycin, clindamycin, tetracycline, and beta lactams have also been used both orally and topically in the treatment of acne. However, as is a problem with the use of most antibiotic agents in general, bacteria can become resistant making the underlying condition more difficult to treat. Resistant strains of *Propionibacterium acnes, Staphylococcus aureus, Propionibacterium granulosum, Gardnerella vaginalis* and other bacteria involved in acne have begun to emerge. *See, e.g.,* Eady E.A., et al. The effects of acne treatment with a combination of benzoyl peroxide and erythromycin on skin carriage of erythromycinresistant propionibacteria. Br. J. Dermatol. 1996; 134:107-113; McLean, N. W.; McGroarty, J. A. Appl. Environ. Microbiol. 1996, 62(3), 1089-1092; Nagaraja, P. Indian J. Med. Microbiol. 2008, 26(2), 155-157; Tomusiak, A. et al. Ginekol. Pol. 2011, 82(12), 900-904; Eschenbach, D. A. Clin. Infectious Dis. 2007, 44(2), 220-221. Furthermore, bacteria, including resistant strains of bacteria, can also cause further complications such as skin infections, eye infections, bone and joint infections, central nervous system infections, and endocarditis. Therefore, with antibiotic treatments for acne, it would be highly desirable to have effective treatments which are also useful against resistant bacteria. See Tan, A.W. and Tan, H.H., "Acne vulgaris,: a review of antibiotic therapy", Expert Opin. Pharmacother., 2005 Mar 6(3), 409-418; Oprica, C. et al., "European surveillance study on the antibiotic susceptibility of Propionibacterium acnes ", Clinical Microbiology and Infection, Volume 11, Number 3, March 2005, pp. 204-312; and Goldstein, E., et al., "Comparative In Vitro Activities of retapamulin (SB-275833) against 141 Clinical isolates of propionibacterium spp., Including 117 P. acnes Isolates", Antimicrobial Agents and Chemotherapy, Jan. 2006, p. 379-381, vol. 50, no. 1.

Bacterial vaginosis (BV) is one of the most common vaginal diseases in women of reproductive age. Since BV is caused by an imbalance of normal vaginal microorganisms, there are multiple risk factors, including the use of intrauterine devices, the use of douches, and new sexual partners. *Gardnerella vaginalis* is a causative agent of BV. BV may present with a burning sensation when urinating and white or gray discharge with a fish-like odor. Additionally, BV can increase the risk of contracting sexually transmitted diseases, including HIV/AIDS. Treatment of BV with current antibiotics has high rates of failure and recurrence. Thus, it is important for there to be safe, effective, and convenient alternatives for treating, preventing, or reducing the risk of BV.

From the foregoing, and especially in view of the development of resistant strains of bacteria, it is seen that there is a continuing need for safe, effective, and convenient means for treating, preventing, or reducing the risk of acne, BV, and other skin infections caused or mediated by *Propionibacterium acnes, Staphylococcus aureus,* or *Gardnerella vaginalis.*

Skin infections, including acne and BV, have been treated by a variety of methods, including administration of topical formulations. However, these topical formulations often have undesired side effects and suboptimal efficacy. Accordingly, there is a need for improved topical formulations to treat, prevent, and reduce the risk of acne, BV, and other skin infections caused or mediated by *Propionibacterium acnes, Staphylococcus aureus,* or *Gardnerella vaginalis.*

## SUMMARY OF THE INVENTION

The present invention relates to topical formulations a of biaryl heterocyclic compound, radezolid, for treating, preventing, and/or reducing the risk of acne and other skin infections caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient in need thereof. In certain embodiments, the acne or other skin infection is caused or mediated by *Propionibacterium acnes, Gardnerella vaginalis,* or *Staphylococcus aureus.* For example, the topical formulations for use are administered in a safe and effective amount. The topical formulation can include, e.g., an oxazolidinone compound.

In addition, the present invention provides the use of an antibiotic compound in the manufacture of a medicament useful for topically treating, preventing, or reducing the risk of acne and other skin infections caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)). In certain embodiments, the acne or other skin infected is caused or mediated by *Propionibacterium acnes, Gardnerella vaginalis,* or *Staphylococcus aureus.*

In one aspect, the present invention relates to a topical formulation comprising radezolid, or a pharmaceutically acceptable salt, tautomer, and a penetration enhancer.

In another aspect, the present invention relates to a topical formulation comprising radezolid or a pharmaceutically acceptable salt, tautomer, or prodrug thereof; a penetration enhancer; a buffering agent; a preservative; an emulsion stabilizer; a moisturizing agent; a cosmesis enhancer; a pH modifier; a nonionic surfactant; and water.

In another aspect, the present invention relates to a topical formulation comprising radezolid or a pharmaceutically acceptable salt, tautomer, or prodrug thereof; a penetration enhancer; a buffering agent; a preservative; a moisturizing agent; a thickener; a pH modifier; and water.

In another aspect, the present invention relates to a topical formulation comprising radezolid or a pharmaceutically acceptable salt, tautomer, or prodrug thereof; a penetration enhancer; a buffering agent; a preservative; an emulsifier; a moisturizing agent; a pH modifier; a thickener; a salt; and water.

In another aspect, the present invention relates to a topical formulation comprising radezolid or a pharmaceutically acceptable salt, tautomer, or prodrug thereof; a penetration enhancer; an emulsion stabilizer; a moisturizing agent; a cosmesis enhancer; a preservative; a buffering agent;
a pH modifier; and water.

In another aspect, the present invention relates to a topical formulation comprising about 0.1 to about 10 wt.% radezolid mesylate salt, about 0.01 to about 15 wt.% citric acid monohydrate, about 0.01 to about 10 wt.% methylparaben, about 0.01 to about 10 wt.% propylparaben, about 5 to about 25 wt.% propylene glycol, about 1 to about 25 wt.% glycerin, about 0.1 to about 30 wt.% cetostearyl alcohol, about 0.1 to about 25 wt.% cetomacrogol 1000, about 0.5 to about 7.5 wt.% cyclomethicone, about 0.5 to about 7.5 wt.% dimethicone, q.s. 1.0 N NaOH to about pH 3.5 to about pH 4.5, and q.s. purified water to 100%.

In another aspect, the present invention relates to a topical formulation comprising about 0.1 to about 10 wt.% radezolid mesylate salt, about 0.01 to about 15 wt.% citric acid monohydrate, about 0.01 to about 10 wt.% methylparaben, about 0.01 to about 10 wt.% propylparaben, about 5 to about 25 wt.% propylene glycol, about 1 to about 25 wt.% glycerin, about 0.1 to about 20 wt.% Natrosol HXX, q.s. 1.0 N NaOH to about pH 3.5 to about pH 4.5, and q.s. purified water to 100%.

In another aspect, the present invention relates to a topical formulation comprising about 0.1 to about 10 wt.% micronized radezolid hydrochloride salt, about 0.01 to about 15 wt.% trolamine, about 0.01 to about 10 wt.% methylparaben, about 0.01 to about 10 wt.% propylparaben, about 5 to about 25 wt.% propylene glycol, about 1 to about 25 wt.% glycerin, about 0.01 to about 20 wt.% polysorbate 80, about 0.1 to about 10 wt.% NaCl, about 0.1 to about 20 wt.% Natrosol HXX, q.s. 1.0 N HCl to about pH 7.5 to about pH 8.0, and q.s. purified water to 100%.

In another aspect, the present invention relates to a topical formulation comprising about 0.1 to about 10 wt.% radezolid mesylate salt, about 5 to about 25 wt.% propylene glycol, about 0.1 to about 30 wt.% cetostearyl alcohol, about 1 to about 25 wt.% glycerin, about 0.5 to about 7.5 wt.% cyclomethicone, about 0.1 to about 30 wt.% polyoxyl 20 cetostearyl ether, about 0.5 to about 7.5 wt.% dimethicone, about 0.01 to about 10 wt.% methylparaben, about 0.01 to about 15 wt.% anhydrous citric acid, about 0.01 to about 10 wt.% propylparaben, q.s. NaOH to about pH 3.8 to 4.2, and q.s. purified water to 100%.

In another aspect, the present invention relates to a topical formulations disclosed herein for treating, preventing, or reducing the risk of a skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient. In certain embodiments, the skin infection is caused or mediated by *Propionibacterium acnes, Gardnerella vaginalis,* or *Staphylococcus aureus.*

In another aspect, the present invention relates to the topical formulations disclosed herein when used to treat, prevent, or reduce the risk of a skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient. In certain embodiments, the skin infection is caused or mediated by *Propionibacterium acnes, Gardnerella vaginalis,* or *Staphylococcus aureus.*

The foregoing and other aspects and embodiments of the present invention can be more fully understood by reference to the following detailed description and claims. Certain features of the invention, which are, for clarity, described in the context of separate embodiments, may also be provided in combination in a single embodiment. All combinations of the embodiments are specifically embraced by the present invention and are disclosed herein just as if each and every combination was individually and explicitly disclosed. Conversely, various features of the invention, which are, for brevity, described in the context of a single embodiment, may also be provided separately or in any suitable sub-combination. All sub-combinations of features listed in the embodiments are also specifically embraced by the present invention and are disclosed herein just as if each and every such sub-combination was individually and explicitly disclosed herein.

## BRIEF DESCRIPTION OF THE FIGURES

**FIG 1.** shows radezolid safety in a long-term rat study, as indicated by average body weight over time.

**FIG 2.** shows linezolid safety in a long-term rat study, as indicated by average body weight over time.

**FIG 3.** shows bacterial burden in pouch over time when treated with radezolid.

**FIG 4.** shows radezolid levels in pouch and plasma over time when treated with radezolid.

**FIG 5.** shows radezolid hydrochloride salt formulation stability testing results at (i) zero time, (ii) 3 months at 2-8 °C, (iii) 1 month at 25 °C, (iv) 3 months at 25 °C, (v) 6 months at 25 °C, (vi) 1 month at 40 °C, (vii) 3 months at 40 °C, and (viii) 6 months at 40 °C.

**FIG 6.** shows radezolid mesylate salt formulation stability testing results at (i) zero time, (ii) 1 month at 25 °C, (iii) 3 months at 25 °C, (iv) 6 months at 25 °C, (v) 1 month at 40 °C, (vi) 3 months at 40 °C, and (vii) 6 months at 40 °C.

**FIG 7.** shows the comparative delivered dose (outliers included) of different radezolid formulations for transdermal and dermal delivery.

**FIG 8.** shows the comparative delivered dose (outliers excluded) of different radezolid formulations for transdermal and dermal delivery.

## DETAILED DESCRIPTION OF THE INVENTION

The present invention relates to topical formulations of biaryl heterocyclic compounds and methods of use thereof for treating, preventing, or reducing the risk of acne and other skin infections caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient in need thereof, wherein the topical formulation comprises a safe and effective amount of an antibiotic, *i.e.,* an antimicrobial compound such as, *e.g.,* an oxazolidinone compound as described herein. The topical formulations disclosed herein are useful for treating, preventing, or reducing the risk of a variety of skin or mucosal infections which can be managed topically and are caused by Gram-negative bacteria such as *Klebsiella, Enterobacter, Haemophilus, Moraxella, Corynebacterium* and *Proteus* species or Gram-positive organisms such as *Propionibacterium acnes, Streptococcus* or *Staphylococcus* species including MRSA. In certain embodiments, the present invention relates to topical formulations of biaryl heterocyclic compounds and methods of use thereof for treating, preventing, or reducing the risk of acne and other skin infections caused or mediated by *Propionibacterium acnes, Gardnerella vaginalis,* or *Staphylococcus aureus.*

### *1. Definitions*

The term "patient," as used herein, means the human or animal (in the case of an animal, more typically a mammal) subject that would be considered to be in need of the methods of treating, preventing, or reducing the risk of a skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient. In certain embodiments, the skin infection is caused or mediated by *Propionibacterium acnes, Gardnerella vaginalis,* or *Staphylococcus aureus.*

As used herein, the phrase "pharmaceutically acceptable" refers to those compounds, materials, compositions, carriers, formulations, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "treating," as used herein, means to cure, arrest the development of, relieve the symptoms or effects of, ameliorate, or cause the regression of an already present acne outbreak or other skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient or subject. In certain embodiments, the acne outbreak or other skin infection is caused or mediated by *Propionibacterium acnes, Gardnerella vaginalis,* or *Staphylococcus aureus.*

The term "preventing," as used herein means, to completely or almost completely stop an acne outbreak or other skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) from occurring in a patient

or subject, especially when the patient or subject is predisposed to such. In certain embodiments, the acne outbreak or other skin infection is caused by *Propionibacterium acnes, Gardnerella vaginalis,* or *Staphylococcus aureus.* Preventing can also include inhibiting the acne outbreak or other skin infection.

The term "reducing the risk of," as used herein, means to lower the likelihood or probability of an acne outbreak or other skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) from occurring, especially when the patient or subject is predisposed to such occurrence. In certain embodiments, the acne outbreak or other skin infection is caused by *Propionibacterium acnes, Gardnerella vaginalis,* or *Staphylococcus aureus.*

As used herein, the term "pharmaceutically effective amount" refers to an amount of a compound or formulation effective when administered alone or in combination with other active ingredients useful to treat, prevent, or reduce the risk of acne or other skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)). In certain embodiments, the acne or other skin infection is caused or mediated by *Propionibacterium acnes, Gardnerella vaginalis,* or *Staphylococcus aureus.* For example, a pharmaceutically effective amount refers to an amount of the compound present in a formulation or on a medical device given to a recipient patient or subject sufficient to elicit biological activity, for example, activity against acne or other skin infection caused or mediated by *Propionibacterium acnes, Gardnerella vaginalis,* or *Staphylococcus aureus.* The combination of compounds optionally is a synergistic combination. Synergy, as described, for example, by Chou and Talalay, Adv. Enzyme Regul. vol. 22, pp. 27-55 (1984), occurs when the effect of the compounds when administered in combination is greater than the additive effect of the compounds when administered alone as a single agent. In general, a synergistic effect is most clearly demonstrated at sub-optimal concentrations of the compounds. Synergy can be in terms of lower cytotoxicity, increased anti-proliferative and/or anti-infective effect, or some other beneficial effect of the combination compared with the individual components.

The term "prophylactically effective amount" means an effective amount of a compound or formulation that is administered to prevent or reduce the risk of an acne outbreak or other skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)). In certain embodiments, the acne outbreak or other skin infection is caused by *Propionibacterium acnes, Gardnerella vaginalis,* or *Staphylococcus aureus.* In other words, an amount that can be administered to provide a preventative or prophylactic effect.

As used herein, "topically applying" and "topical administration" means that compounds or formulations are applied to the skin of the patient directly laying or spreading said compounds or formulations on the outer skin, scalp, hair, fur, feathers, scales, shells, eyes, or ears of the patient, e.g., by use of the hands or an applicator such as a wipe, roller, or spray. Topically applying and topical administration can also include administering the compounds or formulations of the present application intravaginally, *e.g*., by use of an applicator such as a wipe, roller, or spray.

As used herein, the term "topical formulation" refers to a formulation comprising one or more biaryl heterocyclic compounds (*e.g*., radezolid) that is applied to the skin of a patient in need thereof. In some embodiments, the topical formulations disclosed herein are directly laid or spread on the outer skin, scalp, hair, fur, feathers, scales, shells, eyes, or ears of the patient, e.g., by use of the hands or an applicator such as a wipe, roller, or spray. In further embodiments, the topical formulations disclosed herein are applied intravaginally, such as by the use of an applicator such as a wipe, roller, or spray. In some embodiments, the topical formulations disclosed herein are in the form of a gel, a cream, or a lotion.

As used herein, the term "preservative-free" refers to a composition which includes less than 0.005% preservatives by weight. In some embodiments, the preservative-free formulations disclosed herein include less than 0.005% by weight of a preservative selected from the group consisting of methylparaben, propyl paraben, benzyl alcohol, benzalkonium chloride, cetylpyridinium chloride, benzoic acid, sodium benzoate, potassium sorbate, and combinations thereof. In further embodiments, the preservative-free formulations disclosed herein include less than 0.005% total by weight of all of methylparaben, propyl paraben, benzyl alcohol, benzalkonium chloride, cetylpyridinium chloride, benzoic acid, sodium benzoate, potassium sorbate. In still further embodiments, the preservative-free formulations disclosed herein include less than 0.005% by weight of each of methylparaben, propyl paraben, benzyl alcohol, benzalkonium chloride, cetylpyridinium chloride, benzoic acid, sodium benzoate, potassium sorbate.

As used herein, "dermatologically acceptable" means that the ingredients the term describes are suitable for use in contact with tissues (e.g., the skin or hair) without undue toxicity, incompatibility, instability, irritation, allergic response, or the like.

With respect to the antibiotic compounds useful in the topical formulations disclosed herein, the following terms can be applicable, however, it should be kept in mind that more specific definitions are also given in the references referred to and incorporated by reference herein:

The chemical compounds described herein can have asymmetric centers. Compounds disclosed herein containing an asymmetrically substituted atom can be isolated in optically active or racemic forms. It is well known in the art how to prepare optically active forms, such as by resolution of racemic forms or by synthesis from optically active starting materials. Many geometric isomers of olefins, C=N double bonds, and the like can also be present in the compounds described herein, and all such stable isomers are contemplated in the present invention. *Cis* and *trans* geometric isomers of the compounds disclosed herein are described and can be isolated as a mixture of isomers or as separated isomeric forms. All chiral, diastereomeric, racemic, and geometric isomeric forms of a structure are intended, unless the specific stereochemistry or isomeric form is specifically indicated. All processes used to prepare compounds disclosed herein and intermediates made therein are, where appropriate, considered to be part of the present invention. All tautomers of shown or described compounds are also, where appropriate, considered to be part of the present invention.

When a bond to a substituent is shown to cross a bond connecting two atoms in a ring, then such substituent can be bonded to any atom in the ring. When a substituent is listed without indicating the atom via which such substituent is bonded to the rest of the compound of a given formula, then such substituent can be bonded via any atom in such substituent. Combinations of substituents and/or variables are permissible, but only if such combinations result in stable compounds.

As used herein, "pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines, alkali or organic salts of acidic residues such as carboxylic acids, and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 2-acetoxybenzoic, 2-hydroxyethane sulfonic, acetic, ascorbic, benzene sulfonic, benzoic, bicarbonic, carbonic, citric, edetic, ethane disulfonic, ethane sulfonic, fumaric, glucoheptonic, gluconic, glutamic, glycolic, glycollyarsanilic, hexylresorcinic, hydrabamic, hydrobromic, hydrochloric, hydroiodic, hydroxymaleic, hydroxynaphthoic, isethionic, lactic, lactobionic, lauryl sulfonic, maleic, malic, mandelic, methane sulfonic, napsylic, nitric, oxalic, pamoic, pantothenic, phenylacetic, phosphoric, polygalacturonic, propionic, salicylic, stearic, subacetic, succinic, sulfamic, sulfanilic, sulfuric, tannic, tartaric, toluene sulfonic, and the commonly occurring amine acids, e.g., glycine, alanine, phenylalanine, arginine, etc.

The pharmaceutically acceptable salts of the compounds disclosed herein can be synthesized from a parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are preferred. Lists of suitable salts are found in Remington: The Science and Practice of Pharmacy, 22nd ed. (Pharmaceutical Press, 2012). For example, salts can include, but are not limited to, the hydrochloride and acetate salts of the aliphatic amine-containing, hydroxyl amine-containing, and imine-containing compounds disclosed herein.

Additionally, the compounds disclosed herein, for example, the salts of the compounds, can exist in either hydrated or unhydrated (the anhydrous) form or as solvates with other solvent molecules. Nonlimiting examples of hydrates include monohydrates, dihydrates, etc. Nonlimiting examples of solvates include ethanol solvates, acetone solvates, etc.

The compounds disclosed herein can also be prepared as prodrugs, for example pharmaceutically acceptable prodrugs. Since prodrugs are known to enhance numerous desirable qualities of pharmaceuticals (*e.g.*, solubility, bioavailability, manufacturing, etc.) the compounds disclosed herein can be delivered in prodrug form. Thus, the present invention is intended to cover topical formulations containing prodrugs of the compounds disclosed herein, and methods of delivering the same. "Prodrugs" are intended to include any covalently bonded carriers that release an active parent drug of the compounds disclosed herein *in vivo* when such prodrug is administered to a mammalian subject. Prodrugs of the compounds disclosed herein are prepared by modifying functional groups present in the compound in such a way that the modifications are cleaved, either in routine manipulation or *in vivo,* to the parent compound. Prodrugs include compounds disclosed herein wherein a hydroxy, amino, or sulfhydryl group is bonded to any group that, when the prodrug of the compounds disclosed herein is administered to a mammalian subject, cleaves to form a free hydroxyl, free amino, or free sulfhydryl group, respectively. Examples of prodrugs include, but are not limited to, acetate, formate, and benzoate derivatives of alcohol and amine functional groups in the compounds disclosed herein.

"Stable compound" and "stable structure" are meant to indicate a compound that is sufficiently robust to survive isolation, and as appropriate, purification from a reaction mixture, and formulation into an efficacious therapeutic agent.

As used herein, "skin infection" includes but is not limited to acne vulgaris, rosacea, impetigo, otitis externa, bacterial conjunctivitis, and bacterial vaginosis.

As used herein, "treating acne" refers to a mitigating, reducing, preventing, improving, or eliminating the presence or signs of disorders resulting from the actions of hormones and other substances on the sebaceous glands and hair

follicles, typically leading to clogged pores and the formation of inflammatory or non-inflammatory lesions on the skin. Specifically, it relates to the treatment or prevention of blemishes, lesions, or pimples, preemergent pimples, blackheads, and/or whiteheads.

As used herein, "treating bacterial vaginosis" refers to mitigating, reducing, preventing, improving, or eliminating the presence or signs of disorders resulting from the actions of *Gardnerella vaginalis* in the vagina.

In the specification, the singular forms also include the plural, unless the context clearly dictates otherwise. Unless defined otherwise, all technical and scientific terms used herein have the same meaning as commonly understood by one of ordinary skill in the art to which this invention belongs. In the case of conflict, the present specification will control.

All percentages and ratios used herein, unless otherwise indicated, are by weight.

Throughout the description, where compositions are described as having, including, or comprising specific components, it is contemplated that compositions also consist essentially of, or consist of, the recited components. Similarly, where methods or processes are described as having, including, or comprising specific process steps, the processes also consist essentially of, or consist of, the recited processing steps. Further, it should be understood that the order of steps or order for performing certain actions is immaterial so long as the invention remains operable. Moreover, two or more steps or actions can be conducted simultaneously.

### *2. Methods of the Invention*

It is to be understood that any reference to a method of treatment of a human or animal body is to be regarded as referring; to a composition for use in such a method.

The present invention relates to methods for treating acne and other skin infections caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient in need thereof by administering a safe and effective amount of a topical formulation disclosed herein. In certain embodiments, the acne or other skin infection is caused by *Propionibacterium acnes, Gardnerella vaginalis,* or *Staphylococcus aureus*. In certain embodiments, the treating is curing an acne outbreak or other skin infection in a patient or subject. In certain embodiments, the treating is arresting the development of an acne outbreak or other skin infection in a patient or subject. In certain embodiments, the treating is relieving the symptoms or effects of an acne outbreak or other skin infection in a patient or subject. In certain embodiments, the treating is ameliorating an acne outbreak or other skin infection in a patient or subject. In certain embodiments, the treating is causing the regression of an acne outbreak or other skin infection in a patient or subject.

The present invention relates to methods for preventing acne and other skin infections caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient in need thereof by administering a safe and effective amount of a topical formulation disclosed herein. In certain embodiments, the acne or other skin infection is caused or mediated by *Propionibacterium acnes, Gardnerella vaginalis,* or *Staphylococcus aureus.* In certain embodiments, the preventing is completely or almost completely stopping an acne outbreak or other skin infection from occurring in a patient or subject. In certain embodiments, the preventing is inhibiting an acne outbreak or other skin infection in a patient or subject.

The present invention relates to methods for reducing the risk of acne and other skin infections caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient by administering a safe and prophylactically effective amount of a topical formulation disclosed herein. In certain embodiments, the acne or other skin infection is caused or mediated by *Propionibacterium acnes, Gardnerella vaginalis,* or *Staphylococcus aureus.*

As discussed above, acne and other skin infections can be caused or mediated by *Propionibacterium acnes, Gardnerella vaginalis,* or *Staphylococcus aureus.* It has been found that the topical formulations disclosed herein are useful for treating, preventing, or reducing the risk of these microbial infections and the associated acne or other skin infections caused or mediated by *Propionibacterium acnes, Gardnerella vaginalis,* or *Staphylococcus aureus.* The methods disclosed herein can be usefully applied to patients, whether human or animal.

As discussed above, bacterial vaginosis can be caused or mediated by *Gardnerella vaginalis.* It has been found that the topical formulations disclosed herein are useful for treating, preventing, or reducing the risk of bacterial vaginosis caused or mediated by *Gardnerella vaginalis.* The methods disclosed herein can be usefully applied to patients, whether human or animal.

In practicing the methods disclosed herein, it is desired that the tissue level, or sometimes the blood level in the patient or subject, of the topical formulation used to provide the effect be of an appropriate level for a sufficient time interval. Also, because it often takes a finite amount of time to achieve such blood or tissue levels, it is important that

the topical formulation is administered at some appropriate time. The appropriate time for administration of the topical composition will depend upon the pharmacokinetic profile of the compound and its formulation, time required for completing administration, patient characteristics, desired clinical outcome, etc.

The formulations disclosed herein are provided to the patient or subject by topical administration, including, but not limited to administration to the skin, hair, fur, feathers, ears, eyes, or vagina.

### *3. Antibiotic Compounds Useful in the Topical Formulations Disclosed Herein*

The antibiotic, *i.e.,* antimicrobial, compounds or antibiotic agents useful in the topical formulations and methods of use disclosed herein are those that are particularly effective against bacteria that cause or mediate or are involved in acne and other undesirable skin infections caused or mediated by *Propionibacterium acnes, Gardnerella vaginalis,* or *Staphylococcus aureus,* and that are especially effective against resistant strains of such bacteria.

The antibiotic compounds useful in the topical formulations disclosed herein can include their pharmaceutically acceptable salts, esters, tautomers, or prodrugs thereof. The invention also provides topical formulations comprising an effective amount of one or more of the antibiotic compounds disclosed herein and a penetration enhancer. The present invention further provides methods for making these topical formulations.

An antibiotic compound particularly useful in the invention is radezolid (structure shown below), or a pharmaceutically acceptable salt, tautomer, thereof:

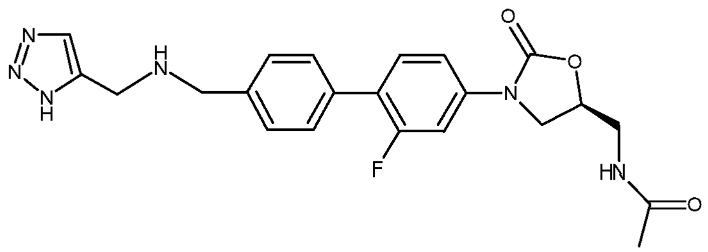

Other names for the compound radezolid include:

RX-1741;
N-[3-(2-Fluoro-4'-{[(3H-[1,2,3]triazol-4-ylmethyl)-amino]-methyl}-biphenyl-4-yl)-2-oxo-oxazolidin-5-(S)-ylmethyl]-acetamide;
N-[3-(2-Fluoro-4'-{[(1H-[1,2,3]triazol-4-ylmethyl)-amino]-methyl}-biphenyl-4-yl)-2-oxo-oxazolidin-5-(S)-ylmethyl]-acetamide;
N-{[(5S)-3-(2-fluoro-4'-{[(1H-1,2,3-triazol-5-ylmethyl)amino]methyl}biphenyl-4-yl)-2-oxo-1,3-oxazolidin-5-yl]methyl} acetamide;
869884-78-6;
UNII-53PC6LO35W; and
(S)-N-((3-(4'-((((1H-1,2,3-triazol-5-yl)methyl)amino)methyl)-2-fluoro-[1,1'-biphenyl]-4-yl)-2-oxooxazolidin-5 -yl)methyl)acetamide.

As used herein, the term radezolid can also be used to refer to tautomers of radezolid, for example, the compound (S)-N-((3-(4'-((((1H-1,2,3-triazol-4-yl)methyl)amino)methyl)-2-fluoro-[1,1'-biphenyl]-4-yl)-2-oxooxazolidin-5-yl)methyl)acetamide or the compound N-((3-(4'-((((2H-1,2,3-triazol-4-yl)methyl)amino)methyl)-2-fluoro-[1,1'-biphenyl] -4-yl)-2-oxooxazolidin-5 -yl)methyl)acetamide.

### *4. Formulation and Administration*

The methods disclosed herein can be practiced by administering the topical formulations disclosed herein to a patient or subject in need thereof. The dose of the topical formulation will depend upon the intended patient or subject and the targeted microorganism, e.g., the target bacterial organism. The formulations typically include biaryl heterocyclic antibiotic compounds (e.g., radezolid) in association with a pharmaceutically acceptable carrier.

The carrier(s) should be "acceptable" in the sense of being compatible with compounds disclosed herein and not deleterious to the recipient. Pharmaceutically acceptable carriers, in this regard, are intended to include any and all solvents, dispersion media, coatings, absorption delaying agents, and the like, compatible with pharmaceutical administration. The use of such media and agents for pharmaceutically active substances is known in the art. Except insofar as any conventional media or agent is incompatible with the active compound, use thereof in the topical formulations

disclosed herein is contemplated. Supplementary active compounds (identified or designed as disclosed herein and/or known in the art) also can be incorporated into the topical formulations disclosed herein. The formulations can conveniently be presented in dosage unit form and can be prepared by any of the methods well known in the art of pharmacy/microbiology. In general, some formulations are prepared by bringing the compound into association with a liquid carrier or a finely divided solid carrier or both, and then, if necessary, shaping the product into the desired formulation.

In some embodiments, the methods, uses and topical formulations for use disclosed herein can further include an additional active agent. In some embodiments, the additional active agent is selected from the group consisting of salicylic acid, benzoyl peroxide, glycolic acid, lactic acid, citric acid, lactobionic acid, triclosan, triclocarban and combinations thereof. Other exemplary agents which can be used in accordance with the present disclosure include those discussed in Decker, A.; Graber, E. M. J. Clin. Aesthet. Dermatol. 2012, 5(5), 32-40, the contents of which are hereby incorporated by reference in their entirety.

A topical formulation disclosed herein can be formulated to be compatible with its intended route of administration. These can include solids and semisolids. Solutions or suspensions can include the following components: a sterile diluent such as water, saline solution, fixed oils, polyethylene glycols, glycerine, propylene glycol or other synthetic solvents; antibacterial agents such as benzyl alcohol or methylparabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The pH can be adjusted with acids or bases, such as hydrochloric acid or sodium hydroxide.

A wide variety of formulations and administration methods can be found in S. K. Niazi, ed., Handbook of Pharmaceutical Formulations, Vols. 1-6 [Vo1. 1 Compressed Solid Products, Vol. 2 Uncompressed Drug Products, Vol. 3 Liquid Products, Vol. 4 Semi-Solid Products, Vol. 5 Over the Counter Products, and Vol. 6 Sterile Products], CRC Press, April 27, 2004.

Formulations suitable for topical administration, including eye treatment, include liquid or semi-liquid preparations such as liniments, lotions, gels, applicants, oil-in-water or water-in-oil emulsions such as creams, ointments or pastes; foams; or solutions or suspensions such as drops. Formulations for topical administration to the skin surface can be prepared by dispersing the drug with a dermatologically acceptable carrier such as a lotion, cream, ointment or soap. Useful are carriers capable of forming a film or layer over the skin to localize application and inhibit removal. For topical administration to internal tissue surfaces, the agent can be dispersed in a liquid tissue adhesive or other substance known to enhance adsorption to a tissue surface. For example, hydroxypropylcellulose or fibrinogen/thrombin solutions can be used to advantage. Alternatively, tissue-coating solutions, such as pectin-containing formulations can be used.

Additionally, the carrier for a topical formulation can be in the form of a hydroalcoholic system (e.g. liquids and gels), an anhydrous oil or silicone based system, or an emulsion system, including, but not limited to, oil-in-water, water-in-oil, water-in-oil-in-water, and oil-in-water-in-silicone emulsions. The emulsions can cover a broad range of consistencies including thin lotions (which can also be suitable for spray or aerosol delivery), creamy lotions, light creams, heavy creams, and the like. The emulsions can also include microemulsion systems. Other suitable topical carriers include anhydrous solids and semisolids (such as gels and sticks); and aqueous based mousse systems. Nonlimiting examples of the topical carrier systems useful in the topical formulations disclosed herein are described in the following four references, all of which are incorporated herein by reference in their entirety: "Sun Products Formulary", Cosmetics & Toiletries, vol. 105, pp. 122-139 (December 1990); "Sun Products Formulary", Cosmetics & Toiletries, vol. 102, pp. 117-136 (March 1987); U.S. Pat. No. 4,960,764 to Figueroa et al., issued Oct. 2, 1990; and U.S. Pat. No. 4,254,105 to Fukuda et al., issued Mar. 3, 1981.

The pharmaceutically-acceptable topical carriers, in total, typically comprise from about 0.1% to about 99.8% by weight of the compositions useful in the topical formulations disclosed herein, alternatively from about 0.1% to about 99.9%, alternatively from about 0.1% to about 99%, alternatively from about 5% to about 99%, alternatively from about 10% to about 99%, alternatively from about 20% to about 99%, alternatively about 50% to about 99%, alternatively from about 80% to about 99%, and alternatively from about 85% to about 95%.

Generally, an effective amount of dosage of active compound will be in the range of from about 0.01 to about 1500 mg. The amount administered will likely depend on such variables as the condition to be treated, the severity of the condition, the age and overall health status of the patient, the relative biological efficacy of the compound delivered, the formulation of the compound, and the presence and types of excipients in the formulation. Also, it is to be understood that the initial dosage administered can be increased beyond the above upper level in order to rapidly achieve the desired tissue level or blood level, or the initial dosage can be smaller than the optimum.

Nonlimiting doses of active compound comprise from about 0.1 to about 1500 mg per dose. Nonlimiting examples of doses, which can be formulated as a unit dose for convenient administration to a patient include: about 0.10 mg, about 0.15 mg, about 0.20 mg, about 0.25 mg, about 0.30 mg, about 0.35 mg, about 0.40 mg, about 0.45 mg, about 0.50 mg, about 0.75 mg, about 1 mg, about 2 mg, about 2.5 mg, about 3 mg, about 4 mg, about 5 mg, about 7.5 mg, about 10 mg, about 12.5 mg, about 15, mg, about 20 mg, about 25 mg, about 30 mg, about 35 mg, about 40 mg, about 45 mg, about 50 mg, about 60 mg, about 70 mg, about 75 mg, about 80 mg, about 90 mg, about 100 mg, about 110 mg, about

120 mg, about 125 mg, about 130 mg, about 140 mg, about 150 mg, about 160 mg, about 170 mg, about 175 mg, about 180 mg, about 190 mg, about 200 mg, about 210 mg, about 220 mg, about 225 mg, about 230 mg, about 240 mg, about 250 mg, about 275 mg, about 300 mg, about 325, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050, mg, about 1075 mg, about 1100 mg, about 1125 mg, about 1150 mg, about 1175 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, and about 1500 mg. The foregoing doses are useful for administering the compounds of the topical formulations disclosed herein according to the methods disclosed herein.

Alternatively, the amount of active ingredient in the topical formulations disclosed herein can be described on a weight percentage basis. Nonlimiting amounts of active ingredients include about 0.01%, about 0.015%, about 0.02%, about 0.025% about 0.03%, about 0.035% about 0.04%, about 0.045%, about 0.05%, about 0.055%, about 0.06%, about 0.065%, about 0.07%, about 0.075%, about 0.080%, about 0.085%, about 0.090%, about 0.095%, about 0.1%, about 0.15%, about 0.2%, about 0.25%, about 0.3%, about 0.35%, about 0.4%, about 0.45%, about 0.5%, about 0.55%, about 0.6%, about 0.65%, about 0.7%, about 0.75%, about 0.8%, about 0.85%, about 0.9%, about 0.95%, about 1%, about 1.25%, about 1.5%, about 1.75%, about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5%, about 10%, about 10.5%, about 11%, about 11.5%, about 12%, about 12.5%, about 13%, about 13.5%, about 14%, about 14.5%, about 15%, about 15.5%, about 16%, about 16.5%, about 17%, about 17.5%, about 18%, about 18.5%, about 19%, about 19.5%, about 20%, about 21%, about 22%, about 23%, about 24%, about 25%, about 26%, about 27%, about 28%, about 29%, about 30%, about 31%, about 32%, about 33%, about 34%, about 35%, about 36%, about 37%, about 38%, about 39%, about 40%, about 41%, about 42%, about 43%, about 44%, about 45%, about 46%, about 47%, about 48%, about 49%, about 50%, about 51%, about 52%, about 53%, about 54%, about 55%, about 56%, about 57%, about 58%, about 59%, about 60%, about 61%, about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, about 99.1%, about 99.2%, about 99.3%, about 99.4%, about 99.5%, about 99.6%, about 99.7%, about 99.8%, and about 99.9%.

In some embodiments, the pH of the formulation is between about 2.5 and about 5.5. In some embodiments, the pH is between about 3.5 and about 4.5. In some embodiments, the pH is about 4.0. In some embodiments, the topical formulation includes a radezolid mesylate salt and is in the form of a solution. In some embodiments, the radezolid mesylate salt solution has a pH between about 3.5 and about 4.5. In some embodiments, the radezolid mesylate salt solution has a pH of about 4.0.

In some embodiments, the formulation is in the form of a lotion.

In some embodiments, the formulation comprises a cosmesis enhancer *(i.e.,* an excipient that enhances the feel of the topical formulation). In some embodiments, the cosmesis enhancer is silicone-based (*e.g*., cyclomethicone and/or dimethicone).

In some embodiments, the formulation is in the form of a gel.

In some embodiments, the formulation comprises a thickener. In some embodiments, the thickener is a cellulose derivative (*i.e.,* a polysaccharide consisting of glucose units, where one or more hydroxyl groups have been partially reacted with various reagents such that it is a different functional group, *e.g.,* an ester or ether). In some embodiments, the thickener is a nonionic non-cellulose polymer (*i.e.,* a polymer other than cellulose that is not ionic).

In some embodiments, the formulation is in the form of a cream.

In some embodiments, the pH of the formulation is between about 7.0 and about 9.0. In some embodiments, the pH of the formulation is between about 7.0 and about 8.0. In some embodiments, the pH of the formulation is between about 7.5 and about 8.0. In some embodiments, the pH of the formulation is about 7.5. In some embodiments, the topical formulation includes a radezolid HCl salt and is in the form of a suspension. In some embodiments, the radezolid HCl salt suspension has a pH between about 7.5 and about 8.0. In some embodiments, the radezolid HCl salt suspension has a pH of about 7.5.

In some embodiments, the formulation comprises moisturizing agents. In some embodiments, the moisturizing agent is glycerin.

In some embodiments, the formulation comprises a penetration enhancer. In some embodiments, the penetration enhancer is propylene glycol.

In some embodiments, the formulation comprises a thickener. In some embodiments, the thickener is a cellulose derivative.

In some embodiments, the formulation comprises additional salts. In some embodiments, the salt is NaCl. In

some embodiments, the salt is $CaCl_2$.

**Optional Components**

In addition to the required components of the topical formulations, a variety of optional components can also be incorporated.

*Anti-Acne Agents*

The topical formulations disclosed herein can also contain anti-acne agents in addition to the antibiotics. These other anti-acne agents can comprise from about 0.01% to about 20% by weight of the topical formulations, or alternatively from about 0.01% to about 10%, and yet alternatively from about 0.1% to about 5%. Mixtures of these additional anti-acne actives may also be used. Examples of these other anti-acne agents include conventional antibiotic agents such as erythromycin, agents such as tretinoin, keratolytics such as sulfur, lactic acid, glycolic, pyruvic acid, urea, resorcinol, and N-acetylcysteine; retinoids such as retinoic acid and its derivatives (*e.g., cis* and *trans*); antibiotics, antimicrobials, antibacterials, antifungals, antiprotozoals, and antivirals (*e.g*., benzoyl peroxide, octopirox, erythromycin, tetracycline, triclosan, azelaic acid and its derivatives, phenoxy ethanol and phenoxy propanol, ethyl acetate, clindamycin and meclocycline, triclosan, chlorhexidine, tetracycline, neomycin, miconazole hydrochloride, octopirox, parachlorometaxylenol, nystatin, tolnaftate, clotrimazole, and the like); sebostats such as flavonoids; hydroxy acids; antipruritic drugs including, for example, pharmaceutically-acceptable salts of methdilizine and trimeprazine; and bile salts such as scymnol sulfate and its derivatives, deoxycholate, and cholate. Other exemplary agents which can be used in accordance with the present disclosure include those discussed in Decker, A.; Graber, E. M. J. Clin. Aesthet. Dermatol. 2012, 5(5), 32-40, the contents of which are hereby incorporated by reference in their entirety.

Also useful are non-steroidal anti-inflammatory drugs (NSAIDS). The NSAIDS can be selected from the following categories: propionic acid derivatives; acetic acid derivatives; fenamic acid derivatives; biphenylcarboxylic acid derivatives; and oxicams. All of these NSAIDS are fully described in the U.S. Pat. No. 4,985,459 to Sunshine et al., issued Jan. 15, 1991, incorporated by reference herein. Most preferred are the propionic NSAIDS including but not limited to aspirin, acetaminophen, ibuprofen, naproxen, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen and bucloxic acid. Also useful are the steroidal anti-inflammatory drugs including hydrocortisone and the like.

**Other Components for Topical Formulations**

The following components are especially useful for the topical formulations disclosed herein.

*Humectants, Moisturizers, and Skin Conditioners*

The topical formulations disclosed herein can optionally comprise one or more humectants/moisturizers/skin conditioners. A variety of these materials can be employed and each can be present at a level of from about 0.1% to about 20%, alternatively from about 1% to about 10% and yet alternatively from about 2% to about 5%. These materials include urea; guanidine; glycolic acid and glycolate salts (*e.g.* ammonium and quaternary alkyl ammonium); lactic acid and lactate salts (*e.g.* ammonium and quaternary alkyl ammonium); aloe vera in any of its variety of forms (*e.g.,* aloe vera gel ); polyhydroxy alcohols such as sorbitol, glycerol, hexanetriol, propylene glycol, hexylene glycol and the like; polyethylene glycol; sugars and starches; sugar and starch derivatives (*e.g.*, alkoxylated glucose); hyaluronic acid; lactamide monoethanolamine; acetamide monoethanolamine; and mixtures thereof.

In certain embodiments, humectants/moisturizers/skin conditioners useful herein are the $C_3$-$C_6$ diols and triols, and also aloe vera gel. Especially preferred is the triol, glycerol (aka "glycerin" or "glycerine"), and also aloe vera gel.

*Surfactants*

The topical formulations disclosed herein can optionally comprise one or more surfactants. The surfactants can be present at a level from about 0.1% to about 10%, alternatively from about 0.2% to about 5%, and yet alternatively from about 0.2% to about 2.5%. Suitable surfactants include, but are not limited to, nonionic surfactants such as polyalkylene glycol ethers of fatty alcohols, and anionic surfactants such as taurates and alkyl sulfates. Nonlimiting examples of these surfactants include isoceteth-20, sodium methyl cocoyl taurate, sodium methyl oleoyl taurate, polyethylene glycol hexadecyl ether, PEG-20 lanolate, PEG-50 lanolate, PEG-25 propylene glycol stearate, Laureth-4, Laureth-23, Ceteth-10, Ceteth-20, Steareth-10, Steareth-20, Steareth-21, Steareth-100, Oleth-10, Oleth-20, PEG-8 steareth, PEG-40 steareth, PEG-50 steareth, PEG-100 steareth, Polysorbate, Polysorbate 20, Polysorbate 21, Polysorbate 40, Polysorbate 60, Polysorbate 61, Polysorbate 65, Polysorbate 80, Polysorbate 81, and Polysorbate 85, and sodium lauryl sulfate. *See* U.S. Pat. No. 4,800,197, to Kowcz et al., issued Jan. 24, 1989, which is incorporated herein by reference in its entirety. Examples of a broad variety of additional surfactants useful herein are described in McCutcheon's, Detergents and Emulsifiers, North American Edition (1986), published by Allured Publishing Corporation, which is incorporated herein by reference in its entirety.

*Emollients*

The topical formulations disclosed herein can optionally comprise one or more emollients. Examples of suitable emollients include, but are not limited to, volatile and nonvolatile silicone oils, highly branched hydrocarbons, and non-polar carboxylic acid and alcohol esters, and mixtures thereof. Emollients useful in the topical formulations disclosed herein are further described in U.S. Pat. No. 4,919,934, to Deckner et al., issued Apr. 24, 1990, which is incorporated herein by reference in its entirety.

Emollients useful in the topical formulations disclosed herein can typically comprise in total from about 1% to about 50%, preferably from about 1% to about 25%, and more preferably from about 1% to about 10% by weight of the topical formulations.

*Sunscreens*

The topical formulations disclosed herein can also optionally comprise at least one sun screening agent. A wide variety of one or more sun screening agents are suitable for use in the topical formulations disclosed herein and are described in U.S. Pat. No. 5,087,445, to Haffey et al., issued Feb. 11, 1992; U.S. Pat. No. 5,073,372, to Turner et al., issued Dec. 17, 1991; U.S. Pat. No. 5,073,371, to Turner et al. issued Dec. 17, 1991; and Segarin, et al., at Chapter VIII, pages 189 et seq., of Cosmetics Science and Technology.

Exemplary sunscreens which are useful in the topical formulations disclosed herein include those selected from the group consisting of 2-ethylhexyl p-methoxycinnamate (aka "octyl methoxycinnamate"), octocrylene, octyl salicylate, oxybenzone, and mixtures thereof.

Other useful sunscreens include the solid physical sunblocks such as titanium dioxide (micronized titanium dioxide, 0.03 microns), zinc oxide, silica, iron oxide and the like. Without being limited by theory, it is believed that these inorganic materials provide a sun screening benefit through reflecting, scattering, and absorbing harmful UV, visible, and infrared radiation.

Still other useful sunscreens are those disclosed in U.S. Pat. No. 4,937,370, to Sabatelli, issued Jun. 26, 1990; and U.S. Pat. No. 4,999,186, to Sabatelli et al., issued Mar. 12, 1991. The sun screening agents disclosed therein have, in a single molecule, two distinct chromophore moieties which exhibit different ultra-violet radiation absorption spectra. One of the chromophore moieties absorbs predominantly in the UVB radiation range and the other absorbs strongly in the UVA radiation range. These sun screening agents provide higher efficacy, broader UV absorption, lower skin penetration and longer lasting efficacy relative to conventional sunscreens.

Generally, the sunscreens can comprise from about 0.5% to about 20% of the topical formulations disclosed herein. Exact amounts will vary depending upon the sunscreen chosen and the desired Sun Protection Factor (SPF). SPF is a commonly used measure of photoprotection of a sunscreen against erythema. *See* Federal Register, Vol. 43, No. 166, pp. 38206-38269, Aug. 25, 1978.

**Delivery Methods for Topical Formulations**

The topical formulations disclosed herein can also be delivered from a variety of delivery devices. The following are two nonlimiting examples.

*Medicated Cleansing Pads*

The topical formulations disclosed herein can be incorporated into a medicated cleansing pad. Preferably, these pads comprise from about 50% to about 75% by weight of one or more layers of nonwoven fabric material and from about 20% to about 75% by weight (on dry solids basis) of a water soluble polymeric resin. These pads are described in detail in U.S. Pat. No. 4,891,228, to Thaman et al., issued Jan. 2, 1990 and U.S. Pat. No. 4,891,227, to Thaman et al., issued Jan. 2, 1990.

*Dispensing Devices*

The topical formulations disclosed herein can also be incorporated into and delivered from a soft-tipped or

flexible dispensing device. These devices are useful for the controlled delivery of the compositions to the skin surface and have the advantage that the treatment composition itself never need be directly handled by the user. Nonlimiting examples of these devices comprise a fluid container including a mouth, an applicator, means for holding the applicator in the mouth of the container, and a normally closed pressure-responsive valve for permitting the flow of fluid from the container to the applicator upon the application of pressure to the valve. The valve can include a diaphragm formed from an elastically fluid impermeable material with a plurality of non-intersecting arcuate slits therein, where each slit has a base which is intersected by at least one other slit, and where each slit is out of intersecting relation with its own base, and wherein there is a means for disposing the valve in the container inside of the applicator. Examples of these applicator devices are described in U.S. Pat. No. 4,693,623, to Schwartzman, issued Sep. 15, 1987; U.S. Pat. No. 4,620,648, to Schwartzman, issued Sep. 15, 1987; U.S. Pat. No. 3,669,323, to Harker et al., issued Jun. 13, 1972; U.S. Pat. No. 3,418,055, to Schwartzman, issued Dec. 24, 1968; and U.S. Pat. No. 3,410,645, to Schwartzman, issued Nov. 12, 1968. Examples of applicators useful herein are commercially available from Dab-O-Matic, Mount Vernon, N.Y.

### *4. Embodiments*

In one aspect, the present invention relates to a topical formulation comprising radezolid, or a pharmaceutically acceptable salt, tautomer, or prodrug thereof, and a penetration enhancer.

In certain embodiments, the formulation comprises about 0.1% to about 10% by weight of the radezolid, or a pharmaceutically acceptable salt thereof. In certain embodiments, the formulation comprises about 0.2% to about 5% by weight of the radezolid, or a pharmaceutically acceptable salt thereof. In certain embodiments, the formulation comprises about 0.3% to about 3% by weight of the radezolid, or a pharmaceutically acceptable salt thereof. In certain embodiments, the formulation comprises about 0.5% to about 2% by weight of the radezolid, or a pharmaceutically acceptable salt thereof.

In certain embodiments, the formulation comprises about 5% to about 25% by weight penetration enhancer. In certain embodiments, the formulation comprises about 7.5% to about 15% by weight penetration enhancer. In certain embodiments, the penetration enhancer is selected from the group consisting of propylene glycol, hexylene glycol, diethylene glycol monoethyl ether, dimethyl sulfoxide, ethanol, isopropanol, oleic acid, laurocapram, d-limonene, ethyl acetate, and mixtures thereof. In certain embodiments, the penetration enhancer is propylene glycol.

In another aspect, the present invention relates to a topical formulation comprising radezolid or a pharmaceutically acceptable salt, tautomer, or prodrug thereof; a penetration enhancer; a buffering agent; a preservative; an emulsion stabilizer; a moisturizing agent; a cosmesis enhancer; a pH modifier; a nonionic surfactant; and water.

In another aspect, the present invention relates to a topical formulation comprising radezolid or a pharmaceutically acceptable salt, tautomer, or prodrug thereof; a penetration enhancer; a buffering agent; a preservative; a moisturizing agent; a thickener; a pH modifier; and water.

In another aspect, the present invention relates to a topical formulation comprising radezolid or a pharmaceutically acceptable salt, tautomer, or prodrug thereof; a penetration enhancer; a buffering agent; a preservative; an emulsifier; a moisturizing agent; a pH modifier; a thickener; a salt; and water.

In another aspect, the present invention relates to a topical formulation comprising radezolid or a pharmaceutically acceptable salt, tautomer, or prodrug thereof; a penetration enhancer; an emulsion stabilizer; a moisturizing agent; a cosmesis enhancer; a preservative; a buffering agent;
a pH modifier; and water.

In certain embodiments, the topical formulation further comprises a moisturizing agent. In certain embodiments, the formulation comprises about 1% to about 25% by weight moisturizing agent. In certain embodiments, the formulation comprises about 1% to about 15 % by weight moisturizing agent. In certain embodiments, the moisturizing agent is selected from the group consisting of glycerin, urea, lactic acid, glycolic acid, hyaluronic acid, pyrrolidone carboxylic acid, and mixtures thereof. In certain embodiments, the moisturizing agent is glycerin.

In certain embodiments, the formulation is preservative-free.

In certain embodiments, the formulation further comprises a preservative. In certain embodiments, the formulation comprises about 0.01% to about 10% by weight preservative. In certain embodiments, the formulation comprises about 0.01% to about 1% by weight preservative. In certain embodiments, the preservative is selected from the group consisting of methylparaben, propyl paraben, benzyl alcohol, benzalkonium chloride, cetylpyridinium chloride, benzoic acid, sodium benzoate, potassium sorbate, and mixtures thereof. In certain embodiments, the preservative is methylparaben. In certain embodiments, the preservative is propyl paraben. In certain embodiments, the preservative is a mixture of methylparaben and propyl paraben.

In certain embodiments, the formulation further comprises a pH modifier. In certain embodiments, the pH modifier is selected from the group consisting of HCl, phosphoric acid, sulfuric acid, acetic acid, boric acid, citric acid, hydrofluoric acid, nitric acid, oxalic acid, HBr, HI, HClO, $HClO_2$, $HClO_3$, $HClO_4$, $H_2S$, $HNO_2$, $H_2SO_3$, $H_3PO_3$, $H_2CO_3$, $H_2SiO_3$, NaOH, KOH, ammonia, LiOH, $Na_2CO_3$, $NaHCO_3$, $K_2CO_3$, $KHCO_3$, $NaH_2PO_4$, $Na_2HPO_4$, $Na_3PO_4$, $KH_2PO_4$,

$K_2HPO_4$, $K_3PO_4$, megluamine, $Ca(OH)_2$, $Mg(OH)_2$, $Zn(OH)_2$, $Al(OH)_3$, pyridoxine, triethanolamine, ammonium hydroxide, cytosine, diethylamine, meglumine, ornithine, glycine, lysine, arginine, valine, proline, aspartic acid, alanine, asparagine, isoleucine, leucine, methionine, threonine, choline hydroxide, procaine, diethylethanolamine, glucosamine, guanine, nicotinamide, piperazine, guanidine, olamine, piperidine, triethylamine, tromethamine, benzathine, benzathine, and adenine. In certain embodiments, the pH modifier is NaOH.

In certain embodiments, the pH modifier is HCl.

In certain embodiments, the formulation further comprises a buffering agent. In certain embodiments, the formulation comprises about 0.01% to about 15% by weight buffering agent. In certain embodiments, the formulation comprises about 0.01% to about 1.0% by weight buffering agent. In certain embodiments, the buffering agent is selected from the group consisting of citric acid, tartaric acid, lactic acid, phosphoric acid, acetic acid, boric acid, trolamine, ammonia, Tris(hydroxymethyl)aminomethane (TRIS), and pharmaceutically acceptable salts thereof. In certain embodiments, the buffering agent is trolamine or a pharmaceutically acceptable salt thereof. In certain embodiments, the buffering agent is citric acid or a pharmaceutically acceptable salt thereof.

In certain embodiments, the formulation further comprises a thickener. In certain embodiments, the formulation comprises about 0.01% to about 20% by weight thickener. In certain embodiments, the formulation comprises about 0.01% to about 5% by weight thickener. In certain embodiments, the thickener is selected from the group consisting of a cellulose derivative, nonionic non-cellulose polymer, and mixtures thereof. In certain embodiments, the thickener is a cellulose derivative selected from the group consisting of hydroxyethyl cellulose, hydroxypropyl cellulose, methyl cellulose, and hydroxypropyl methylcellulose. In certain embodiments, the cellulose derivative is hydroxyethylcellulose. In certain embodiments, the hydroxyethylcellulose is Natrosol HXX. In certain embodiments, the thickener is a nonionic non-cellulose polymer selected from the group consisting of polyvinylpyrrolidone (povidone), and polyvinylalcohol.

In certain embodiments, the formulation further comprises an emulsifier. In certain embodiments, the formulation comprises about 0.01% to about 20% by weight emulsifier. In certain embodiments, the formulation comprises about 0.01% to about 5.0 % by weight emulsifier. In certain embodiments, the emulsifier is selected from the group consisting of polysorbates, poloxamers, fatty acid esters, fatty acid ethers, and mixtures thereof. In certain embodiments, the emulsifier is a polysorbate selected from the group consisting of Tween, Polysorbate, Polysorbate 20, Polysorbate 21, Polysorbate 40, Polysorbate 60, Polysorbate 61, Polysorbate 65, Polysorbate 80, Polysorbate 81, Polysorbate 85, and mixtures thereof. In certain embodiments, the emulsifier is polysorbate 80. In certain embodiments, the emulsifier is a poloxamer. In certain embodiments, the emulsifier is a fatty acid ester. In certain embodiments, the fatty acid ester is Myrj. In certain embodiments, the emulsifier is a fatty acid ether. In certain embodiments, the fatty acid ether is Brij.

In certain embodiments, the formulation further comprises a salt. In certain embodiments, the formulation comprises about 0.1% to about 10.0% by weight salt. In certain embodiments, the formulation comprises about 0.1% to about 2.0% by weight salt. In certain embodiments, the salt is selected from the group consisting of NaCl, $CaCl_2$, KCl, and mixtures thereof. In certain embodiments, the salt is NaCl.

In certain embodiments, the formulation further comprises a cosmesis enhancer. In certain embodiments, the formulation comprises about 0.5% to about 7.5% by weight cosmesis enhancer. In certain embodiments, the formulation comprises about 0.5% to about 3.0% by weight cosmesis enhancer. In certain embodiments, the cosmesis enhancer is silicone-based. In certain embodiments, the silicone-based cosmesis enhancer is selected from the group consisting of cyclomethicone, dimethicone, and mixtures thereof. In certain embodiments, the silicone-based cosmesis enhancer is cyclomethicone. In certain embodiments, wherein the silicone-based cosmesis enhancer is dimethicone. In certain embodiments, the silicone-based cosmesis enhancer is a mixture of cyclomethicone and dimethicone.

In certain embodiments, the topical formulation further comprises an emulsion stabilizer. In certain embodiments, the formulation comprises about 0.1% to about 30% by weight emulsion stabilizer. In certain embodiments, the formulation comprises about 0.1% to about 15% by weight emulsion stabilizer. In certain embodiments, the emulsion stabilizer is selected from the group consisting of cetostearyl alcohol, cetyl alcohol, stearyl alcohol, myristyl alcohol, glyceryl monostearate, propylene glycol stearate, polyoxyl 20 cetostearyl ether, and mixtures thereof. In certain embodiments, the emulsion stabilizer is cetostearyl alcohol. In certain embodiments, the emulsion stabilizer is polyoxyl 20 cetostearyl ether. In certain embodiments, the emulsion stabilizer is a mixture of cetostearyl alcohol and polyoxyl 20 cetostearyl ether.

In certain embodiments, the topical formulation further comprises a nonionic surfactant. In certain embodiments, the formulation comprises about 0.1% to about 25% by weight nonionic surfactant. In certain embodiments, the formulation comprises about 0.1% to about 5% by weight nonionic surfactant. In certain embodiments, the nonionic surfactant is selected from the group consisting of a polyethylene glycol hexadecyl ether, PEG-20 lanolate, PEG-50 lanolate, PEG-25 propylene glycol stearate, Laureth-4, Laureth-23, Ceteth-10, Ceteth-20, Steareth-10, Steareth-20, Steareth-21, Steareth-100, Oleth-10, Oleth-20, PEG-8 steareth, PEG-40 steareth, PEG-50 steareth, PEG-100 steareth, Polysorbate, Polysorbate 20, Polysorbate 21, Polysorbate 40, Polysorbate 60, Polysorbate 61, Polysorbate 65, Polysorbate 80, Polysorbate 81, and Polysorbate 85, and mixtures thereof. In certain embodiments, the nonionic surfactant is a polyethylene glycol hexadecyl ether. In certain embodiments, the polyethylene glycol hexadecyl ether is Cetomacrogol 1000.

In certain embodiments, the topical formulation has a pH of about 7.5 to about 8.0. In certain embodiments, the topical formulation has a pH of about 7.5. In certain embodiments, the topical formulation has a pH of about 3.5 to about 4.5. In certain embodiments, the topical formulation has a pH of about 4.

In certain embodiments, the radezolid is in the form of a suspension.

In certain embodiments, the radezolid is in the form of a solution.

In certain embodiments, the radezolid is a pharmaceutically acceptable salt selected from the group consisting of acetate, ascorbate, benzoate, citrate, esylate, ethanedisulfonate, fumarate, hydrochloride, lactate, maleate, mesylate, phosphate, pyroglutamate, salicylate, succinate, sulfate, tartrate, and tosylate. In certain embodiments, the radezolid is a radezolid acetate salt. In certain embodiments, the radezolid is a radezolid ascorbate salt. In certain embodiments, the radezolid is a radezolid benzoate salt. In certain embodiments, the radezolid is a radezolid citrate salt. In certain embodiments, the radezolid is a radezolid esylate salt. In certain embodiments, the radezolid is a radezolid ethanedisulfonate salt. In certain embodiments, the radezolid is a radezolid fumarate salt. In certain embodiments, the radezolid is a radezolid lactate salt. In certain embodiments, the radezolid is a radezolid maleate salt. In certain embodiments, the radezolid is a radezolid phosphate salt. In certain embodiments, the radezolid is a radezolid pyroglutamate salt. In certain embodiments, the radezolid is a radezolid salicylate salt. In certain embodiments, the radezolid is a radezolid succinate salt. In certain embodiments, the radezolid is a radezolid sulfate salt. In certain embodiments, the radezolid is a radezolid tartrate salt. In certain embodiments, the radezolid is a radezolid tosylate salt. In certain embodiments, the radezolid is a radezolid mesylate salt. In certain embodiments, the radezolid is a radezolid hydrochloride salt.

In another aspect, the present invention relates to a topical formulation comprising about 0.1 to about 10 wt.% radezolid mesylate salt, about 0.01 to about 15 wt.% citric acid monohydrate, about 0.01 to about 10 wt.% methylparaben, about 0.01 to about 10 wt.% propylparaben, about 5 to about 25 wt.% propylene glycol, about 1 to about 25 wt.% glycerin, about 0.1 to about 30 wt.% cetostearyl alcohol, about 0.1 to about 25 wt.% cetomacrogol 1000, about 0.5 to about 7.5 wt.% cyclomethicone, about 0.5 to about 7.5 wt.% dimethicone, q.s. 1.0 N NaOH to about pH 3.5 to about pH 4.5, and q.s. purified water to 100%.

In certain embodiments, the topical formulation comprises about 0.5 to about 2 wt.% radezolid mesylate salt, about 0.01 to about 10 wt.% citric acid monohydrate, about 0.01 to about 1 wt.% methylparaben, about 0.01 to about 1 wt.% propylparaben, about 7.5 to about 15 wt.% propylene glycol, about 1 to about 15 wt.% glycerin, about 0.1 to about 15 wt.% cetostearyl alcohol, about 0.1 to about 5 wt.% cetomacrogol 1000, about 0.5 to about 3 wt.% cyclomethicone, about 0.5 to about 3 wt.% dimethicone, q.s. 1.0 N NaOH to about pH 4, and q.s. purified water to 100%.

In certain embodiments, the topical formulation comprises about 1.22 wt.% radezolid mesylate salt, about 0.11 wt.% citric acid monohydrate, about 0.15 wt.% methylparaben, about 0.05 wt.% propylparaben, about 10.0 wt.% propylene glycol, about 2.0 wt.% glycerin, about 4.0 wt.% cetostearyl alcohol, about 1.0 wt.% cetomacrogol 1000, about 2.0 wt.% cyclomethicone, about 1.0 wt.% dimethicone, q.s. 1.0 N NaOH to about pH 4, and q.s. purified water to 100%.

In another aspect, the present invention relates to a topical formulation comprising about 0.1 to about 10 wt.% radezolid mesylate salt, about 0.01 to about 15 wt.% citric acid monohydrate, about 0.01 to about 10 wt.% methylparaben, about 0.01 to about 10 wt.% propylparaben, about 5 to about 25 wt.% propylene glycol, about 1 to about 25 wt.% glycerin, about 0.1 to about 20 wt.% Natrosol HXX, q.s. 1.0 N NaOH to about pH 3.5 to about pH 4.5, and q.s. purified water to 100%.

In certain embodiments, the topical formulation comprises about 0.5 to about 2 wt.% radezolid mesylate salt, about 0.01 to about 10 wt.% citric acid monohydrate, about 0.01 to about 1 wt.% methylparaben, about 0.01 to about 1 wt.% propylparaben, about 7.5 to about 15 wt.% propylene glycol, about 1 to about 15 wt.% glycerin, about 0.1 to about 5 wt.% Natrosol HXX, q.s. 1.0 N NaOH to about pH 4, and q.s. purified water to 100%.

In certain embodiments, the topical formulation comprises about 1.22 wt.% radezolid mesylate salt, about 0.22 wt.% citric acid monohydrate, about 0.15 wt.% methylparaben, about 0.05 wt.% propylparaben, about 10.0 wt.% propylene glycol, about 2.0 wt.% glycerin, about 1.75% Natrosol HXX, q.s. 1.0 N NaOH to about pH 4, and q.s. purified water to 100%.

In certain embodiments, the topical formulation comprises about 1.22 wt.% radezolid mesylate salt, about 0.11 wt.% citric acid monohydrate, about 0.15 wt.% methylparaben, about 0.05 wt.% propylparaben, about 10.0 wt.% propylene glycol, about 2.0 wt.% glycerin, about 1.75% Natrosol HXX, q.s. 1.0 N NaOH to about pH 4, and q.s. purified water to 100%.

In another aspect, the present invention relates to a topical formulation comprising about 0.1 to about 10 wt.% radezolid hydrochloride salt, about 0.01 to about 15 wt.% trolamine, about 0.01 to about 10 wt.% methylparaben, about 0.01 to about 10 wt.% propylparaben, about 5 to about 25 wt.% propylene glycol, about 1 to about 25 wt.% glycerin, about 0.01 to about 20 wt.% polysorbate 80, about 0.1 to about 10 wt.% NaCl, about 0.1 to about 20 wt.% Natrosol HXX, q.s. 1.0 N HCl to about pH 7.5 to about pH 8.0, and q.s. purified water to 100%.

In certain embodiments, the topical formulation comprises about 0.5 to about 2 wt.% radezolid hydrochloride salt, about 0.01 to about 10 wt.% trolamine, about 0.01 to about 1 wt.% methylparaben, about 0.01 to about 1 wt.% propylparaben, about 7.5 to about 15 wt.% propylene glycol, about 1 to about 15 wt.% glycerin, about 0.01 to about 5 wt.% polysorbate 80, about 0.1 to about 2 wt.% NaCl, about 0.1 to about 5 wt.% Natrosol HXX, q.s. 1.0 N NaOH to about

pH 7.5, and q.s. purified water to 100%.

In certain embodiments, the topical formulation comprises about 1.08 wt.% radezolid hydrochloride salt, about 0.45 wt.% trolamine, about 0.15 wt.% methylparaben, about 0.05 wt.% propylparaben, about 10.0 wt.% propylene glycol, about 2.0 wt.% glycerin, about 0.01 wt.% polysorbate 80, about 0.5 wt.% NaCl, about 1.75 wt.% Natrosol HXX, q.s. 1.0 N HCl to about pH 7.5, q.s. purified water to 100%.

In certain embodiments, the topical formulation comprises about 1.08 wt.% radezolid hydrochloride salt, about 0.45 wt.% trolamine, about 0.15 wt.% methylparaben, about 0.05 wt.% propylparaben, about 10.0 wt.% propylene glycol, about 2.0 wt.% glycerin, about 0.01 wt.% polysorbate 80, about 1.0 wt.% NaCl, about 1.25 wt.% Natrosol HXX, q.s. 1.0 N HCl to about pH 7.5, q.s. purified water to 100%.

In certain embodiments, the topical formulation comprises about 1.08 wt.% radezolid hydrochloride salt, about 0.45 wt.% trolamine, about 0.15 wt.% methylparaben, about 0.05 wt.% propylparaben, about 10.0 wt.% propylene glycol, about 2.0 wt.% glycerin, about 0.01 wt.% polysorbate 80, about 1.0 wt.% NaCl, about 1.75 wt.% Natrosol HXX, q.s. 1.0 N HCl to about pH 7.5, q.s. purified water to 100%.

In another aspect, the present invention relates to a topical formulation comprising about 0.1 to about 10 wt.% radezolid mesylate salt, about 5 to about 25 wt.% propylene glycol, about 0.1 to about 30 wt.% cetostearyl alcohol, about 1 to about 25 wt.% glycerin, about 0.5 to about 7.5 wt.% cyclomethicone, about 0.1 to about 30 wt.% polyoxyl 20 cetostearyl ether, about 0.5 to about 7.5 wt.% dimethicone, about 0.01 to about 10 wt.% methylparaben, about 0.01 to about 15 wt.% anhydrous citric acid, about 0.01 to about 10 wt.% propylparaben, q.s. NaOH to about pH 3.8 to 4.2, and q.s. purified water to 100%.

In certain embodiments, the topical formulation comprises about 0.2 to about 5 wt.% radezolid mesylate salt, about 7.5 to about 15 wt.% propylene glycol, about 0.1 to about 15 wt.% cetostearyl alcohol, about 1 to about 15 wt.% glycerin, about 0.5 to about 3.0 wt.% cyclomethicone, about 0.1 to about 15 wt.% polyoxyl 20 cetostearyl ether, about 0.5 to about 3.0 wt.% dimethicone, about 0.01 to about 1 wt.% methylparaben, about 0.01 to about 1.0 wt.% anhydrous citric acid, about 0.01 to about 1 wt.% propylparaben, q.s. NaOH to about pH 3.8 to 4.2, and q.s. purified water to 100%.

In certain embodiments, the topical formulation comprises about 0.61 wt.% radezolid mesylate salt, about 10 wt.% propylene glycol, about 4 wt.% cetostearyl alcohol, about 2 wt.% glycerin, about 2 wt.% cyclomethicone, about 1 wt.% polyoxyl 20 cetostearyl ether, about 1 wt.% dimethicone, about 0.15 wt.% methylparaben, about 0.10 wt.% anhydrous citric acid, about 0.05 wt.% propylparaben, q.s. NaOH to about pH 3.8 to 4.2, and q.s. purified water to 100%.

In certain embodiments, the topical formulation comprises about 0.91 wt.% radezolid mesylate salt, about 10 wt.% propylene glycol, about 4 wt.% cetostearyl alcohol, about 2 wt.% glycerin, about 2 wt.% cyclomethicone, about 1 wt.% polyoxyl 20 cetostearyl ether, about 1 wt.% dimethicone, about 0.15 wt.% methylparaben, about 0.10 wt.% anhydrous citric acid, about 0.05 wt.% propylparaben, q.s. NaOH to about pH 3.8 to 4.2, and q.s. purified water to 100%.

In certain embodiments, the topical formulation comprises about 1.22 wt.% radezolid mesylate salt, about 10 wt.% propylene glycol, about 4 wt.% cetostearyl alcohol, about 2 wt.% glycerin, about 2 wt.% cyclomethicone, about 1 wt.% polyoxyl 20 cetostearyl ether, about 1 wt.% dimethicone, about 0.15 wt.% methylparaben, about 0.10 wt.% anhydrous citric acid, about 0.05 wt.% propylparaben, q.s. NaOH to about pH 3.8 to 4.2, and q.s. purified water to 100%.

In certain embodiments, the topical formulation comprises about 2.20 wt.% radezolid mesylate salt, about 10 wt.% propylene glycol, about 4 wt.% cetostearyl alcohol, about 2 wt.% glycerin, about 2 wt.% cyclomethicone, about 1 wt.% polyoxyl 20 cetostearyl ether, about 1 wt.% dimethicone, about 0.15 wt.% methylparaben, about 0.10 wt.% anhydrous citric acid, about 0.05 wt.% propylparaben, q.s. NaOH to about pH 3.8 to 4.2, and q.s. purified water to 100%.

In certain embodiments, the topical formulation is in the form of a gel.

In certain embodiments, the topical formulation is in the form of a cream.

In certain embodiments, the topical formulation is in the form of a lotion.

In another aspect, the present invention relates to the topical formulations disclosed herein for treating, preventing, or reducing the risk of a skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient. In certain embodiments, the skin infection is caused or mediated by *Propionibacterium acnes, Gardnerella vaginalis,* or *Staphylococcus aureus.*

In another aspect, the present invention relates to a method of treating, preventing, or reducing the risk of a skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)) in a patient using the topical formulations disclosed herein. In certain embodiments, the skin infection is caused or mediated by *Propionibacterium acnes, Gardnerella vaginalis,* or *Staphylococcus aureus.*

In another aspect, the present invention relates to the topical formulations disclosed herein when used to treat, prevent, or reduce the risk of a skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes,*

*Gardnerella vaginalis,* or *Staphylococcus aureus* (including *Methicillin-resistant Staphylococcus aureus* (MRSA)) in a patient. In certain embodiments, the skin infection is caused or mediated by *Propionibacterium acnes, Gardnerella vaginalis,* or *Staphylococcus aureus.*

In certain embodiments, the use, or formulation disclosed herein is for treating a skin infection caused or mediated by *Propionibacterium acnes, Gardnerella vaginalis,* or *Staphylococcus aureus* in a patient.

In certain embodiments, the use, or formulation disclosed herein is for preventing a skin infection caused or mediated by *Propionibacterium acnes, Gardnerella vaginalis,* or *Staphylococcus aureus* in a patient.

In certain embodiments, the use, or formulation disclosed herein is for reducing the risk of a skin infection caused or mediated by *Propionibacterium acnes, Gardnerella vaginalis,* or *Staphylococcus aureus* in a patient.

In certain embodiments, the skin infection is caused or mediated by *Propionibacterium acnes.* In certain embodiments, the skin infection is caused or mediated by *Gardnerella vaginalis.* In certain embodiments, the skin infection is caused or mediated by *Staphylococcus aureus.*

In certain embodiments, the skin infection is selected from acne vulgaris, rosacea, impetigo, otitis externa, bacterial conjunctivitis, and bacterial vaginosis. In certain embodiments, the skin infection is acne vulgaris. In certain embodiments, the skin infection is bacterial vaginosis.

In certain embodiments, the patient is a mammal or domestic mammal. In certain embodiments, the patient is a human.

In certain embodiments, the topical formulation is administered once daily. In certain embodiments, the topical formulation is administered twice daily.

## EXAMPLES

The following examples further describe and demonstrate embodiments within the scope of the present claims. The examples are given solely for the purpose of illustration and are not to be construed as limitations of the present invention, as many variations thereof are possible without departing from the scope of the claims. Ingredients are identified by chemical or CTFA name.

### EXAMPLE 1: Anti-Microbial Activity of Radezolid and Comparator Agents

The *in vitro* antimicrobial activity of radezolid (compound 4267) and comparator antimicrobial agents were evaluated against several different types of bacteria. Bacterial isolates were obtained from the American Type Culture Collection (ATCC), Manassas, VA.

Susceptibility testing was performed by the agar dilution reference method (radezolid, linezolid, and clindamycin) and broth microdilution method (all agents) as described by the Clinical and Laboratory Standards Institute (CLSI).

For the agar dilution method, Brucella agar (BBL, 211086) was prepared according to the manufacturer's instructions and cooled to 48-50 °C in a water bath. Once the agar was cooled, it was supplemented with 10 mL of hemin + vitamin K1 Solution (Remel, R450951), 1 mL of 1 mg/mL vitamin K1 working solution (Sigma, V3501), and 50 mL laked sheep blood (Remel, R54004). The media was dispensed into 50 mL centrifuge tubes and held at 48-50 °C. The antimicrobial agents were reconstituted and prepared at 200X the desired concentrations for ranges of 0.03-8 μg/mL (radezolid and linezolid) and 0.06-16 μg/mL (clindamycin). The diluted antimicrobial agents were added to the dispensed media, gently mixed, and poured into sterile petri dishes. Once hardened, the plates were held in anaerobic jars at room temperature and used the following day.

For broth microdilution testing a master 96-well tray at 100X the highest desired concentration was prepared by adding compound to an appropriate well in the first column and serially diluting through the 10th column. DMSO or water was added to the 11th and 12th columns for growth and sterility controls. A 1:10 dilution was made into a 96-well tray containing Brucella Broth (BBL, 211088) supplemented with hemin + vitamin K1, and 5% lysed horse blood (1HB) (Remel, R54092). Daughter plates containing 10 μL of each well of the 10X tray were dispensed.

The anaerobic bacterial isolates were removed from frozen stocks and placed into thioglycollate broth with hemin and vitamin K1 and without indicator (THIO) (Anaerobe Systems, AS-805). The G. *vaginalis* isolates were reconstituted from the ATCC vials using Brucella Broth (BRU-Broth) (Anaerobe Systems, AS-105) and plated onto Brucella Blood Agar plates (BRU) (Anaerobe Systems, AS-141) and chocolate agar plates (CA) (BD BBL, 221169). THIO and BRU were incubated for 48 hours in an anaerobic jar at 35 °C; CA plates were incubated at 35 °C for 48 hours in 5% $CO_2$. The S. *aureus* isolate was removed from a frozen stock, streaked onto TSA with 5% sheep blood (BA) (BD BBL, 221261), and incubated overnight at 35 °C in ambient air. The day before testing the anaerobes and G. *vaginalis* were subcultured to fresh BRU and THIO and S. *aureus* was subcultured to BA; all were incubated as required.

On the day of testing, a 0.5 McFarland equivalent of each isolate was prepared in Brucella broth with hemin + vitamin K1. For the agar dilution method, 2 μL spots were inoculated onto each agar plate containing antibiotic in ascending concentration order, as well as BRU and CA plates before and after each set of compound as growth control

(beginning) and to assess any drug carryover (end). BRU plates and those containing compound were incubated at 35 °C in an anaerobic environment for 24 hours, read, and re-incubated for an additional 24 hours followed by a final reading. CA plates were incubated at 35 °C in 5% $CO_2$ for 48 hours as a negative control (note that some anaerobes are aerotolerant and may grow in $CO_2$). Following incubation, MICs were determined as the lowest concentration showing a marked reduction in growth or no growth compared to the growth control plate.

For the broth microdilution method, 0.5 mL of the 0.5 McFarland equivalent was placed into 4.5 mL of Brucella Broth with hemin, vitamin K1, and 5% 1HB. This intermediate suspension was diluted further (1.1 mL into 8.9 mL) in Brucella Broth with hemin, vitamin K1, and 5% 1HB for a final concentration of approximately $10^5$ CFU/well. Trays were incubated at 35 °C in an anaerobic environment for 24 hours, read, and re-incubated for an additional 24 hours followed by a final reading. To confirm the inoculum, a sample from a growth control well was diluted and spread onto BRU and CA. The plates were incubated for 48 hours at 35 °C in an anaerobic (BRU) or 5% $CO_2$ (CA) environment; colonies were counted and the inoculum was determined. Following incubation, the MIC endpoint was determined as the lowest concentration showing no growth or significantly reduced growth. If growth was poor, the MIC endpoint was not determined.

Agar and broth microdilution MICs for radezolid, linezolid, and clindamycin against the ten isolates are shown below in Table 1, and broth microdilution MICs for the remaining comparators are shown in Table 2. The "reference" agar dilution method is recommended by CLSI for anaerobic susceptibility testing. Due to limited space, only three compounds were tested by this method: radezolid, linezolid, and clindamycin, and all isolates grew well.

The broth microdilution method is recommended by CLSI for *B. fragilis* group isolates only, but all isolates were tested by this method. Of the three *P. acnes* isolates, two did not grow in broth, and a third isolate grew poorly. The remaining isolates grew well in broth.

Comparing broth and agar dilution MICs when values were obtained for both methods, MICs were mostly identical or within 2-fold for each antimicrobial agent. Only *F. magna* had MICs that differed by 4-fold, for radezolid.

**Table 1. Agar Dilution and Broth Microdilution MICs for Radezolid, Linezolid, and Clindamycin**

| | | Radezolid | | Linezolid | | Clindamycin | |
|---|---|---|---|---|---|---|---|
| **Organism** | **ATCC No.** | **Agar** | **Broth** | **Agar** | **Broth** | **Agar** | **Broth** |
| *B. fragilis* | 25285 | 4 | 2 | 4 | 2 | 2 | 1 |
| *B. thetaiotaomicron* | 29741 | >8 | 8 | 4 | 4 | 8 | 8 |
| *F. magna* | 15794 | 0.5 | 0.125 | 2 | 1 | 1 | 0.5 |
| *P. acnes* | 11828 | 0.125 | No growth | 0.5 | No growth | 0.125 | No growth |
| *P. acnes* | 29399 | 0.125 | Poor growth | 0.5 | Poor growth | ≤0.06 | Poor growth |
| *P. acnes* | 11827 | 0.125 | No growth | 0.5 | No growth | ≤0.06 | No growth |
| *P. granulosum* | 25746 | 0.25 | 0.125 | 2 | 1 | 1 | 1 |
| *G. vaginalis* | 14018 | 0.06 | 0.125 | 0.5 | 0.5 | 0.25 | 0.125 |
| *G. vaginalis* | 49145 | 0.06 | 0.06 | 0.5 | 0.5 | 0.125 | ≤0.06 |
| *S. aureus* | 29213 | 1 | 2 | 2 | 4 | 0.25 | 0.25 |

Agar dilution MICs for radezolid were more potent than those of linezolid by 4-fold for *P. acnes* and *F. magna,* and by 8-fold for *P. granulosum* and *G. vaginalis.* Radezolid and linezolid demonstrated similar activity against *S. aureus.* Clindamycin and radezolid MICs were within 2- to 4-fold of each other for most of the isolates.

**Table 2. Broth Microdilution MICs for Radezolid and Comparator Agents**

| **Organism** | **ATCC No.** | **RDZ** | **ERY** | **MET** | **TET** | **DOX** |
|---|---|---|---|---|---|---|
| *B. fragilis* | 25285 | 2 | 16 | 0.5 | 0.25 | ≤0.06 |
| *B. thetaiotaomicron* | 29741 | 8 | 16 | 4 | 8 | 2 |
| *F. magna* | 15794 | 0.125 | 4 | 0.125 | 0.25 | 0.125 |
| *P. acnes* | 11828 | No growth | No growth | No growth | No growth | No growth |
| *P. acnes* | 29399 | Poor growth | Poor growth | Poor growth | Poor growth | Poor growth |

(continued)

| Organism | ATCC No. | RDZ | ERY | MET | TET | DOX |
|---|---|---|---|---|---|---|
| *P. acnes* | 11827 | No growth | No growth | No growth | No growth | No growth |
| *P. granulosum* | 25746 | 0.125 | 16 | 1 | 0.25 | 0.25 |
| *G. vaginalis* | 14018 | 0.125 | ≤0.06 | 8 | 0.5 | 0.5 |
| *G. vaginalis* | 49145 | 0.06 | ≤0.06 | 4 | >32 | 32 |
| *S. aureus* | 29213 | 2 | 1 | >32 | 1 | 0.5 |
| Abbreviations: RDZ, radezolid; ERY, erythromycin; MET, metronidazole; TET, tetracycline; DOX, doxycycline | | | | | | |

In broth microdilution testing, radezolid was 8-fold more active than linezolid against *P. granulosum* and *G. vaginalis* 49145. Radezolid was also more active than clindamycin, erythromycin, and metronidazole against *P. granulosum,* and more active than metronidazole, tetracycline, and doxycycline against *G. vaginalis.*

The oxazolidinones and clindamycin demonstrated similar potency against *B. fragilis.* MICs for most agents were within 2-fold of radezolid MICs against *B. thetaiotaomicron;* doxycycline was 4-fold more potent.

Against *F. magna,* radezolid was more active than linezolid, clindamycin, and erythromycin; the other agents were similar in activity to radezolid. Metronidazole MICs were significantly higher than radezolid against *S. aureus;* doxycycline and clindamycin MICs were 4-fold and 8-fold lower, respectively. The MICs for the remaining comparator compounds were within 2-fold of radezolid for *S. aureus.*

In this study, radezolid demonstrated robust antimicrobial activity against most of the isolates tested, with agar dilution MICs of 0.06-1 μg/mL for all organisms. Radezolid demonstrated enhanced activity compared to the other oxazolidinones against several of the isolates.

Table 3 shows MICs for radezolid and comparator agents against ribosome-based resistance phenotypes. As shown in Table 3, radezolid exhibited similar or superior results to linezolid and azithromycin across all phenotypes tested.

**Table 3. Minimum Inhibitory Concentrations (MICs) (μg/mL) for Radezolid and Comparator Agents Against Ribosome-Based Resistance Phenotypes**

| Bacterial Strain | Resistance Phenotype | Linezolid | Azithromycin | Radezolid |
|---|---|---|---|---|
| *Enterococcus faecalis* ATCC29212 | QC | 4 | 8 | 0.25 |
| *Enterococcus faecalis* ATCC29212-P5 | Lin-R (G2576U) | 32 | 8 | 1 |
| *Enterococcus faecalis* 1069 | VanB | 4 | 128 | 0.25 |
| *Enterococcus faecium* A6349 | VanA+Lin-R (G2576U) | 16 | 128 | 0.5 |
| *Staphylococcus aureus* ATCC29213 | QC | 4 | 1 | 1 |
| *Staphylococcus aureus* A7820 | Lin-R (G2576U) +Mac-R (ErmC) | 64 | 128 | 16 |
| *Staphylococcus aureus 01A1095* | Mac-R | 4 | 128 | 1 |

**EXAMPLE 2: Safety of Radezolid Versus Comparator Agent**

Safety of radezolid versus comparator linezolid were tested in long-term rat studies (*see* **FIGs. 1-2).** Radezolid showed good safety. As shown in Figure 1, the male rats generally had higher body weights than the females, with similar body weights within each of the dose groups. There was 100% survival in all dose groups. No test article-related changes were observed in hematology, coagulation, clinical chemistry, or urinalysis. An unscheduled euthanization on day 75 for high-dose linezolid groups showed decreased red cell mass, absolute reticulocyte and neutrophil counts in rats dosed with 100 mg/kg/day linezolid. This is correlated with decreased cellularity of sternal and femoral bone marrow.

Table 4 shows the calculated safety margin of radezolid based on these data.

**Table 4. Calculated Safety Margin of Radezolid**

| | |
|---|---|
| Exposure (AUC 0 - 24): male rats at 200 mg/kg on Day 29 | 101.9 μg*hr/mL |
| Exposure (AUC 0 - 24): human @ 300 mg dose | 3.33 μg*hr/mL |
| Safety margin (Rat NOAEL AUC / Human efficacious AUC) | 30.8x |

**EXAMPLE 3: Uptake of Radezolid Versus Comparator Agent**

Approximately 60% of the radezolid accumulates in the cytosol of cells while approximately 40% accumulates in the lysosome. Radezolid accumulates in mammalian cells (*e.g.*, defense cells, macrophages, lung cells, and non-phagocytic cells) to a 17-fold greater extent than linezolid. Radezolid kills intracellular *S. aureus* (including linezolid-resistant), *Listeria monocytogenes* and *Legionella pneumophila,* organisms that reside in different cellular compartments. Accumulation affords once-daily dosing at doses lower than those predicted by plasma levels. Accumulation offers a wider safety window for radezolid as compared to linezolid. *See* Lemaire et al. AAC 2010, 54(6): 6549-59.

Levels of radezolid remain high in granuloma pouch even as they decline in the plasma, contributing to efficacy at the site of infection *(see* **FIGs. 3-4).**

**EXAMPLE 4: Synthesis of (5*S*)-N-(3-{2-fluoro-4'- [(3-fluoro-propylamino)-methyl]-biphenyl-4-yl}-2-oxo-oxazolidin-5-ylmethyl)-acetamide mono hydrochloride salt (Compound 11)**

Scheme 1 below depicts synthesis of aryl boronic acid **120,** which is coupled to aryl iodide **108** to yield compound **11.**

**Scheme 1. Synthesis of Compound 11**

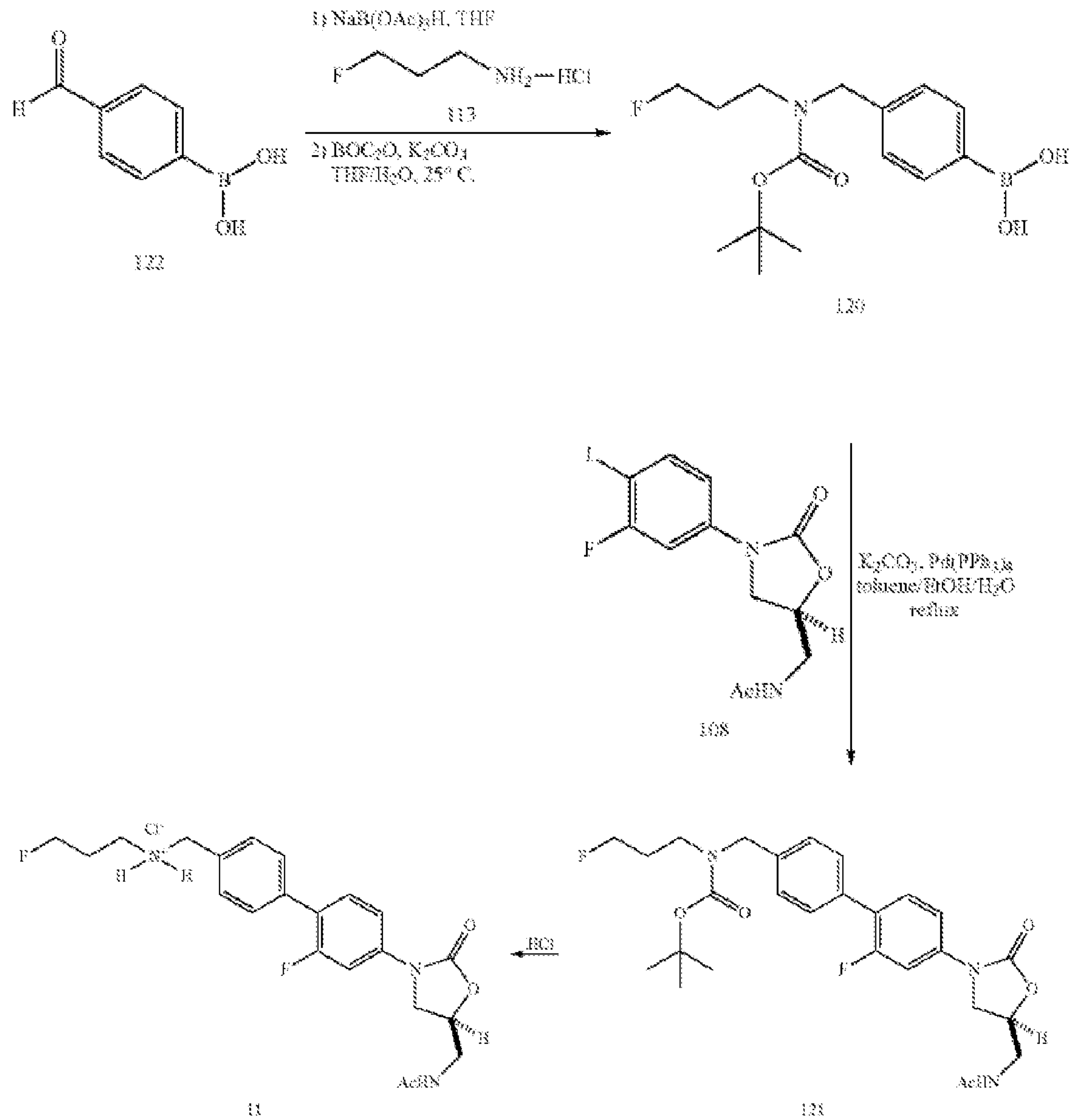


A solution of 4-formylphenyl boronic acid **122** (10.0 g, 66.69 mmol) in anhydrous DMF (150 mL) was treated with 3-fluoropropylamine hydrochloride salt **113** (8.70 g, 76.70 mmol, 1.15 equiv) at room temperature. The resulting mixture was treated with $NaB(OAc)_3H$ (28.30 g, 133.39 mmol, 2.0 equiv) at room temperature and stirred for 3 h. When TLC and HPLC/MS showed the reaction was complete, the reaction mixture was treated with water (150 mL), solid $Na_2CO_3$ (14.14 g, 133.39 mmol, 2.0 equiv), and $BOC_2O$ (22.05 g, 100.04 mmol, 1.5 equiv). The resulting reaction mixture was stirred at room temperature for 3 h. When TLC and HPLC/MS showed the reaction was complete, the reaction mixture was poured into water (500 mL) and EtOAc (500 mL). The two layers were separated and the aqueous layer was treated with a 2 N aqueous HCl (130 mL) to pH 4. The aqueous layer was then extracted with EtOAc (160 mL), and the combined organic layers were washed with water (2×100 mL) and saturated aqueous NaCl (2× 100 mL), dried over $Na_2SO_4$, and concentrated *in vacuo.* The residue was further dried *in vacuo* to afford the desired 4-(N-tert-butylcarbonyl-3-fluoropropylaminomethyl) phenyl boronic acid **120** (25.0 g) as a pale yellow oil. This product was directly used in the subsequent reaction without further purification.

A suspension of aryl boronic acid **120** (25.0 g, 64.30 mmol, 1.45 equiv) in a mixture of toluene (120 mL), EtOH (40 mL), and water (40 mL) was treated with (5*S*)-N-[3-(3-fluoro-4-iodo-phenyl)-2-oxo-oxazolidin-5-ylmethyl]-acetamide **108** (16.80 g, 44.44 mmol) and solid $K_2CO_3$ (18.40 g, 133.4 mmol, 3.0 equiv) at room temperature. The resulting reaction mixture was degassed three times under a steady stream of argon before being treated with $Pd(PPh_3)_4$ (2.57 g, 2.23 mmol, 0.05 equiv). The resulting reaction mixture was degassed three times under a steady stream of argon before

being warmed to reflux for 8 h. When TLC and HPLC/MS showed the reaction was complete, the reaction mixture was cooled to room temperature before being poured into water (300 mL) and ethyl acetate (EtOAc, 300 mL). The two layers were separated, and the organic phase was washed with water (60 mL) and saturated aqueous NaCl (2x50 mL), dried over anhydrous $Na_2SO_4$, and concentrated *in vacuo.* The product was recrystallized from EtOAc/hexanes and dried *in vacuo* to afford the desired (5*S*)-{4'-[5-(acetylamino-methyl)-2-oxo-oxazolidin-3-yl]-2'-fluoro-biphenyl-4-ylmethyl}-(3-fluoro-propyl)-carbamic acid tert-butyl ester **121** (21.2 g, 61.5% yield for three steps) as an off-white powder.

BOC-protected amine **121** was subsequently treated with 4 N hydrogen chloride in 1,4-dioxane to afford compound **11**. $^1H$ NMR (300 MHz, DMSO-$d_6$) $\delta$ 1.90 (s, 3H, $COCH_3$), 2.11-2.20 (m, 2H), 3.10 (m, 2H), 3.50 (t, 2H, J = 5.4 Hz), 3.87 (dd, 1H, J = 6.4, 9.2 Hz), 4.24 (t, 1H, J = 9.1 Hz), 4.27 (s, 2H, $ARCH_2$), 4.54 (t, 1H, J = 5.8 Hz), 4.70 (t, 1H, J = 5.8 Hz), 4.83 (m, 1H), 7.50 (dd, 1H, J = 2.2, 8.6 Hz), 7.65-7.74 (m, 6H, aromatic-H), 8.37 (t, 1H, J = 5.8 Hz, $NHCOCH_3$), 9.43 (br. s, 2H, $RArN^+H_2$). $C_{22}H_{25}F_2N_3O_3HCl$, LCMS (EI) *m/e* 418 ($M^+$+H).

**EXAMPLE 5: Synthesis of Radezolid**

Radezolid (shown as Compound 1) and its hydrochloride salt are synthesized according to Scheme 2 below.

**Scheme 2. Synthesis of Radezolid (Compound 1) and its Hydrochloride Salt**

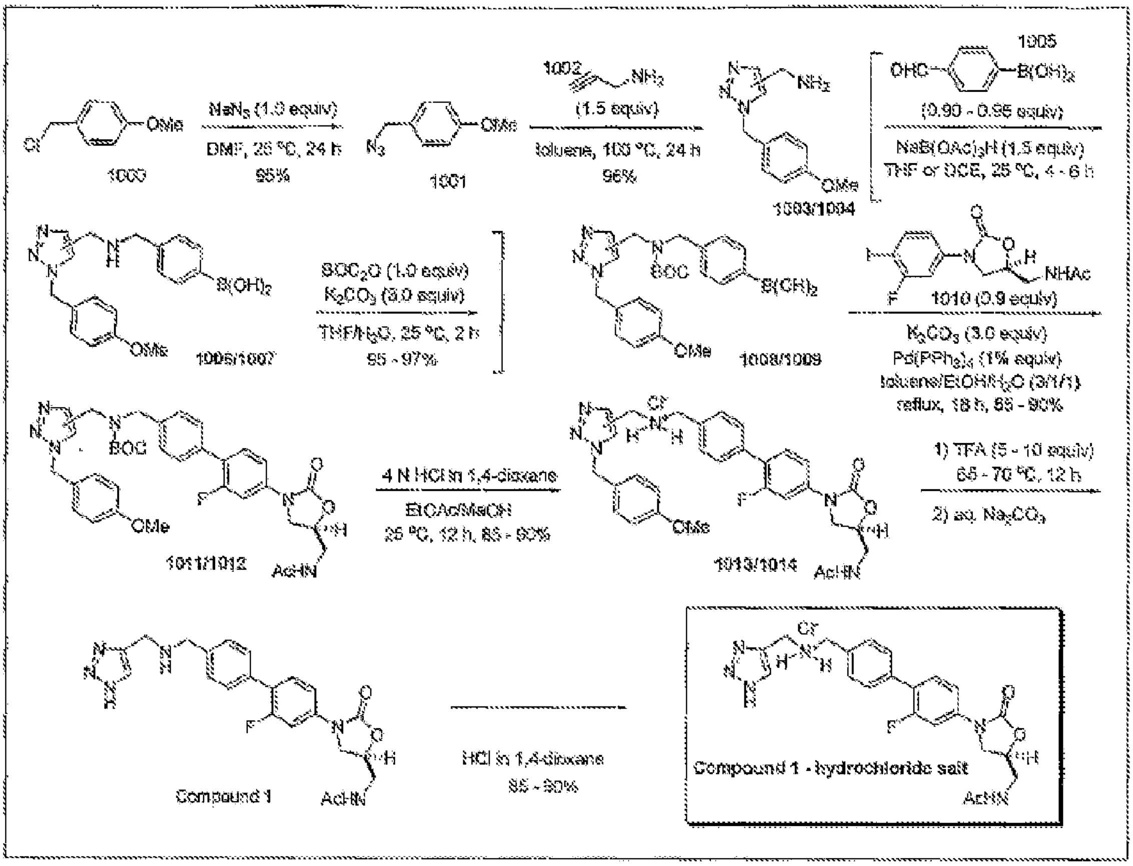


***Synthesis of Oxazolidinone Compound 1010***

Oxazolidinone compound **1010** is prepared according to Scheme 3 below.

**Scheme 3. Synthesis of Oxazolidinone Compound 1010**

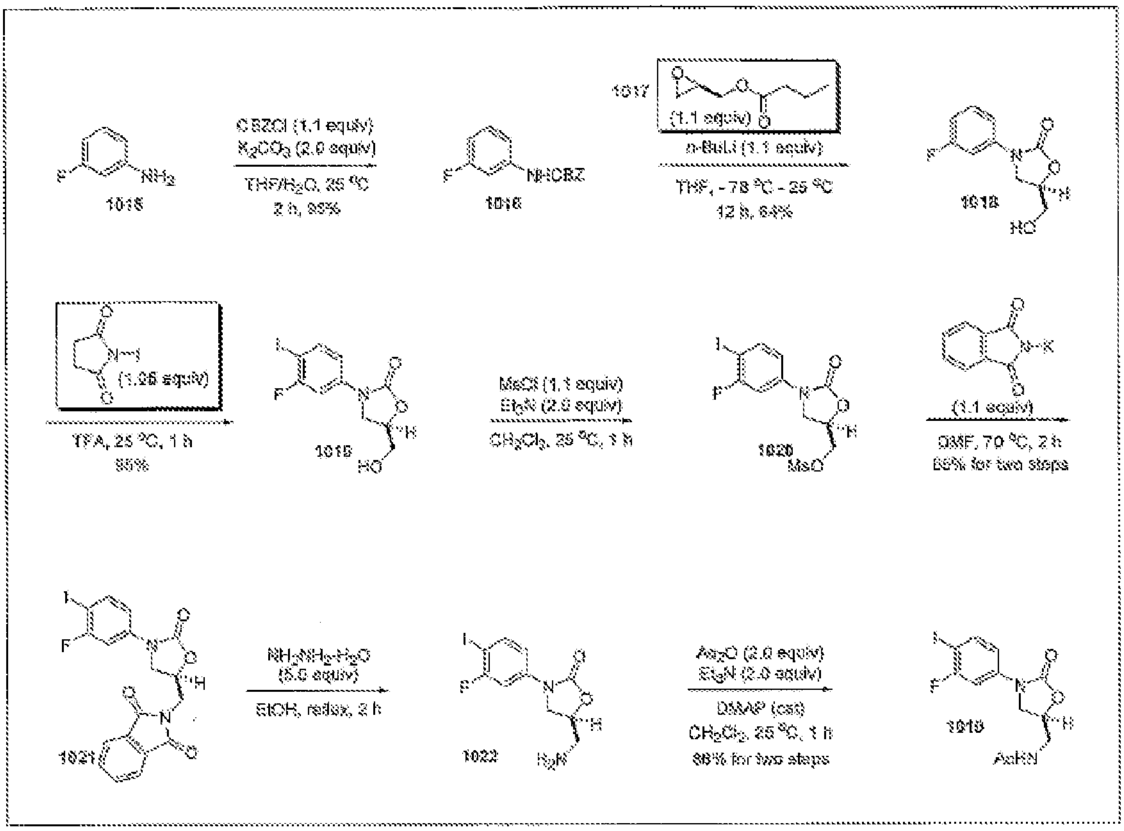


**(3-Fluoro-phenyl)-carbamic acid benzyl ester (1016).** A solution of the 3-fluorophenylamine **(1015,** which is commercially available under the names 3-fluoroaniline or 1-amino-3-fluorobenzene, 18.7 g, 168.3 mmol) in tetrahydrofuran (THF, 150 mL) was treated with potassium carbonate ($K_2CO_3$, 46.45 g, 336.6 mmol, 2.0 equiv) and water (150 mL) before a solution of benzyl chloroformate (CBZCl, 31.58 g, 185.1 mmol, 26.1 mL, 1.1 equiv) in THF (50 mL) was added dropwise into the reaction mixture at room temperature under nitrogen. The resulting reaction mixture was stirred at room temperature for 2 h. When TLC showed that the reaction was complete, the reaction mixture was treated with water (100 mL) and ethyl acetate (EtOAc, 100 mL). The two layers were separated, and the aqueous layer was extracted with EtOAc (2 $\times$ 100 mL). The combined organic extracts were washed with water (2 $\times$ 100 mL) and saturated NaCl aqueous solution (100 mL), dried over $MgSO_4$, and concentrated *in vacuo*. The residue was further dried *in vacuo* to afford the crude, (3-fluoro-phenyl)-carbamic acid benzyl ester (2, 39.2 g, 41.23 g theoretical, 95%) as pale-yellow oil, which was found to be essentially pure and was directly used in the subsequent reactions without further purifications. For 1016: $^1$H NMR (300 MHz, $CDCl_3$) $\delta$ 5.23 (s, 2H, $OCH_2Ph$), 6.75 - 6.82 (m, 2H), 7.05 (dd, 1H, J= 1.4, 8.2 Hz), 7.22 - 7.45 (m, 6H); $C_{14}H_{12}FNO_2$, LCMS (EI) *m/e* 246 ($M^+$ + H).

**(5*R*)-3-(3-Fluoro-phenyl)-5-hydroxymethyl-oxazolidin-2-one (1018).** A solution of (3-fluorophenyl)-carbamic acid benzyl ester **(1016,** 39.2 g, 160.0 mmol) in anhydrous tetrahydrofuran (THF, 300 mL) was cooled dowel to -78 °C in a dry-ice-acetone bath before a solution of *n*-butyllithium (*n*-BuLi, 2.5 M solution in hexanes, 70.4 mL, 176 mmol, 1.1 equiv) was added dropwise at -78 °C under nitrogen. The resulting reaction mixture was subsequently stirred at -78 °C for 1 h before a solution of (*R*)-(-)-glycidyl butyrate 1017 (25.37 g, 24.6 mL, 176 mmol, 1.1 equiv) in anhydrous THF (100 mL) was added dropwise into the reaction mixture at-78 °C under nitrogen. The resulting reaction mixture was stirred at -78 °C for 30 min before being gradually warmed up to room temperature for 12 h under nitrogen. When TLC and HPLC/MS showed that the reaction was complete, the reaction mixture was quenched with water (200 mL), and the resulting mixture was stirred at room temperature for 1 h before ethyl acetate (EtOAc, 200 mL) was added. The two layers were separated, and the aqueous layer was extracted with EtOAc (2 $\times$ 100 mL). The combined organic extracts were washed with water (2 $\times$ 100 mL) and saturated NaCl aqueous solution (100 mL), dried over $MgSO_4$, and concentrated *in vacuo.* The white crystals were precipitated out from the concentrated solution when most of the solvents were evaporated. The residue was then treated with 20% EtOAc/hexanes (100 mL) and the resulting slurry was further stirred at room temperature for 30 min. The solids were then collected by filtration and washed with 20% EtOAc/hexanes (2 $\times$ 50 mL) to afford the crude, (5*R*)-(3-(3-fluoro-phenyl)-5-hydroxymethyl-oxazolidin-2-one (**1018,** 24.4 g, 33.76 g theoretical,

72.3%) as white crystals, which were found to be essentially pure and was directly used in the subsequent reactions without further purifications. For **1018:** $^1$H NMR (300 MHz, DMSO-$d_6$) δ 3.34 - 3.72 (m, 2H), 3.83 (dd, 1H, J= 6.2, 9.0 Hz), 4.09 (t, 1H, J= 12.0 Hz), 4.68 -4.75 (m, 1H), 5.23 (t, 1H, J = 5.6 Hz, OH), 6.96 (m, 1H), 7.32 - 7.56 (m, 3H); $C_{10}H_{10}FNO_3$, LCMS (EI) *m/e* 212 ($M^+$ + H).

**(5*R*)-3-(3-Fluoro-4-iodo-phenyl)-5-hydroxymethyl-oxazolidin-2-one (1019).** A solution of (5*R*)-(3-(3-fluoro-phenyl)-5-hydroxymethyl-oxazolidin-2-one (**1018,** 10.74 g, 50.9 mmol) in trifluoroacetic acid (TFA, 50 mL) was treated with N-iodosuccinimide (NIS, 12.03 g, 53.45 mmol, 1.05 equiv) at 25 °C, and the resulting reaction mixture was stirred at 25 °C for 2 h. When TLC and HPLC/MS showed that the reaction was complete, the reaction mixture was concentrated *in vacuo.* The residue was then treated with water (100 mL) and 20% EtOAc/hexanes (100 mL) at 25 °C, and the resulting mixture was stirred at 25 °C for 30 min before being cooled down to 0 - 5 °C for 2 h. The white solids were collected by filtration, washed with water (2 × 25 mL) and 20% EtOAc/hexanes (2 × 25 mL), and dried *in vacuo* to afford the crude, (5*R*)-3-(3-fluoro-4-iodo-phenyl)-5-hydroxymethyl-oxazolidin-2-one **(1019,** 15.1 g, 17.15 g theoretical, 88%) as off-white powders, which were found to be essentially pure and were directly used in the subsequent reactions without further purifications. For **1019:** $^1$H NMR (300 MHz, DMSO-$d_6$) δ 3.58 (dd, 1H, J= 4.2, 12.6 Hz), 3.67 (dd, 1H, J= 3.0, 12.6 Hz), 3.67 (dd, 1H, J= 6.3, 9.0 Hz), 4.07 (t, 1H, J = 9.0 Hz), 4.72 (m, 1H), 5.21 (br. s, 1H, OH), 7.22 (dd, 1H, J= 2.4, 8.4 Hz), 7.58 (dd, 1H, J= 2.4, 11.1 Hz), 7.81 (dd, 1H, J= 7.8, 8.7 Hz); $C_{10}H_9FINO_3$, LCMS (EI) *m/e* 338 ($M^+$ + H).

**(5*R*)-Methanesulfonic acid 3-(3-fluoro-4-iodo-phenyl)-2-oxo-oxazolidin-5-ylmethyl ester (1020).** A solution of (5*R*)-3-(3-fluoro-4-iodo-phenyl)-5-hydroxymethyl-oxazolidin-2-one **(1019,** 25.2 g, 74.8 mmol) in methylene chloride ($CH_2Cl_2$, 150 mL) was treated with trimethylamine (TEA, 15.15 g, 20.9 mL, 150 mmol, 2.0 equiv) at 25 °C, and the resulting mixture was cooled down to 0 - 5 °C before methanesulfonyl chloride (MsCl, 10.28 g, 6.95 mL, 89.7 mmol, 1.2 equiv) was dropwise introduced into the reaction mixture at 0 - 5 °C under nitrogen. The resulting reaction mixture was subsequently stirred at 0 - 5 °C for 1 h under nitrogen. When TLC and HPLC/MS showed that the reaction was complete, the reaction mixture was quenched with water (100 mL) and $CH_2Cl_2$ (100 mL). The two layers were separated, and the aqueous layer was extracted with $CH_2Cl_2$ (100 mL). The combined organic extracts were washed with water (2 × 100 mL) and saturated NaCl aqueous solution (100 mL), dried over $MgSO_4$, and concentrated *in vacuo.* The residue was further dried *in vacuo* to afford the crude, (5*R*)-methanesulfonic acid 3-(3-fluoro-4-iodo-phenyl)-2-oxo-oxazolidin-5-ylmethyl ester **(1020,** 30.71 g, 31.04 g theoretical, 98.9%) as an off-white powder, which was found to be essentially pure and was directly used in the subsequent reactions without further purifications. For **1020:** $C_{11}H_{11}FINO_5S$, LCMS (EI) *m/e* 416 ($M^+$ + H).

**(5*R*)-2-[3-(3-Fluoro-4-iodo-phenyl)-2-oxo-oxazolidin-5-ylmethyl]-isoindole-1,3-dione (1021).** A solution of (5*R*)-methanesulfonic acid 3-(3-fluoro-4-iodo-phenyl)-2-oxo-oxazolidin-5-ylmethyl ester **(1020,** 26.38 g, 63.57 mmol) in anhydrous N,N-dimethylformamide (DMF, 120 mL) was treated with solid potassium phthalimide (12.95 g, 70.0 mmol, 1.1 equiv) at 25 °C, and the resulting reaction mixture was warmed up to 70 °C for 2 h. When TLC and HPLC showed that the reaction was complete, the reaction mixture was cooled down to room temperature before being quenched with water (400 mL), and the resulting mixture was stirred at room temperature for 10 min before being cooled down to 0 - 5 °C for 1 h. The white precipitates were then collected by filtration, washed with water (3 × 100 mL), and dried *in vacuo* to afford the crude, (5*R*)-2-[3-(3-fluoro-4-iodo-phenyl)-2-oxo-oxazolidin-5-ylmethyl]-isoindole-1,3-dione (**1021,** 27.85 g, 29.64 g theoretical, 94%) as an off-white powder, which was found to be essentially pure and was directly used in the subsequent reactions without further purifications. For **1021:** $C_{18}H_{12}FIN_2O_4$, LCMS (EI) *m/e* 467 ($M^+$ + H).

**(5*S*)-5-Aminomethyl-3-(3-fluoro-4-iodo-phenyl)-oxazolidin-2-one (1022).** A solution of (5*R*)-2-[3-(3-fiuoro-4-iodo-phenyl)-2-oxo-oxazolidin-5-ylmethyl]-isoindole-1,3-dione (1021, 23.3 g, 50.0 mmol) in ethanol (EtOH, 150 mL) was treated with hydrazine monohydrate (12.52 g, 12.1 mL, 250 mmol, 5.0 equiv) at 25 °C, and the resulting reaction mixture was warmed up to reflux for 2 h. White precipitates were formed while the reaction mixture was refluxed. When TLC and HPLC showed that the reaction was complete, the reaction mixture was cooled down to room temperature before being quenched with water (100 mL). The white precipitates were totally dissolved when water was introduced into the reaction mixture and a homogeneous solution was generated. The aqueous solution was then extracted with $CH_2Cl_2$ (3 × 200 mL), and the combined organic extracts were washed with water (2 × 100 mL) and saturated NaCl aqueous solution (100 mL), dried over $MgSO_4$, and concentrated *in vacuo.* The residue was further dried *in vacuo* to afford the crude (5*S*)-5-aminomethyl-3-(3-fluoro-4-iodo-phenyl)-oxazolidin-2-one (**1022,** 16.0 g, 16.8 g theoretical, 95.2%) as a white powder, which was found to be essentially pure and was directly used in the subsequent reactions without further purifications. For **1022:** $C_{10}H_{10}FIN_2O_2$, LCMS (EI) *m/e* 337 ($M^+$ + H).

**(5*S*)-N-[3-(3-Fluoro-4-iodo-phenyl)-2-oxo-oxazolidin-5-ylmethyl]-acetamide** (1010). A suspension of (5*S*)-5-aminomethyl-3-(3-fluoro-4-iodo-phenyl)-oxazolidin-2-one (1022, 16.0 g, 47.6 mmol) in $CH_2Cl_2$ (150 mL) was treated with triethylamine (TEA, 9.62 g, 13.2 mL, 95.2 mmol, 2.0 equiv) at 25 °C, and the resulting reaction mixture was cooled down to 0 - 5 °C before being treated with acetic anhydride ($Ac_2O$, 7.29 g, 6.75 mL, 71.4 mmol, 1.5 equiv) and 4-*N,N*-dimethylaminopyridine (DMAP, 58 mg, 0.5 mmol, 0.01 equiv) at 0 - 5 °C under nitrogen. The resulting reaction mixture was subsequently stirred at 0 - 5 °C for 2 h. When TLC and HPLC showed that the reaction was complete, the reaction mixture was quenched with water (100 mL). The two layers were separated, and the aqueous layer was then

extracted with $CH_2Cl_2$ (2 × 50 mL), and the combined organic extracts were washed with water (2 × 100 mL) and saturated NaCl aqueous solution (100 mL), dried over $MgSO_4$, and concentrated *in vacuo.* The residue was further dried *in vacuo* to afford the crude, (5*S*)-N-[3-(3-fluoro-4-iodo-phenyl)-2-oxo-oxazolidin-5-ylmethyl]-acetamide (**1010,** 17.36 g, 17.99 g theoretical, 96.5%) as white powders, which were found to be essentially pure and were directly used in the subsequent reactions without further purifications. For **1010:** $^1$H NMR (300 MHz, DMSO-$d_6$) δ 1.63 (s, 3H, $NHCOCH_3$), 3.25 (t, 2H, J= 5.4 Hz), 3.56 (dd, 1H, J= 6.4, 9.2 Hz), 3.95 (t, 1H, J= 9.1 Hz), 4.58 (m, 1H), 5.16 (t, 1H, J= 5.7 Hz, OH), 7.02 (dd, 1H, J= 2.4, 8.2 Hz), 7.38 (dd, 1H, J= 2.4, 10.8 Hz), 7.66 (t, 1H, J= 7.5, 8.4 Hz), 8.08 (t, 1H, J= 5.8 Hz, $NHCOCH_3$); $C_{12}H_{12}FIN_2O_3$, LCMS (EI) *m*/*e* 379 ($M^+$ + H).

***Synthesis of Radezolid* (*Compound 1*)**

**4-Methoxybenzyl Azide 1001.** A solution of 4-methoxybenzyl chloride **1000** (51.8 g, 331.0 mmol) in anhydrous DMF (200 mL) was treated with solid sodium azide (21.5 g, 331.0 mmol, 1.0 equiv) at 25 °C, and the resulting mixture was stirred at 25 °C for 24 h. When TLC and HPLC/MS showed that the reaction was complete, the reaction mixture was quenched with water (400 mL) and ethyl acetate (EtOAc, 400 mL) at room temperature. The two layers were separated, and the aqueous layer was extracted with EtOAc (200 mL). The combined organic extracts were washed with water (2 × 200 mL) and saturated NaCl aqueous solution (100 mL), dried over $MgSO_4$, and concentrated *in vacuo.* The crude 4-methoxybenzyl amide (51.2 g, 53.95 g theoretical, 94.9% yield) was obtained as a colorless oil, which by HPLC and $^1$H NMR was found to be essentially pure and was directly used in the subsequent reaction without further purifications. For 4-methoxybenzyl azide 1001: $^1$H NMR (300 MHz, $CDCl_3$) δ 3.84 (s, 3H, $ArOCH_3$), 4.29 (s, 2H, Ar-$CH_2$), 6.96 (d, 2H, J = 8.7 Hz), 7.28 (d, 2H, J= 7.8 Hz).

**C-[1-(4-Methoxy-benzyl)-1H-[1,2,3]triazol-4-yl]-methylamine and C-[3-(4-Methoxybenzyl)-3H-[1,2,3]triazol-4-yl]-methylamine (1003 and 1004).** A solution of 4-methoxybenzylamide **1001** (61.2 g, 375.5 mmol) in toluene (188 mL) was treated with propargylamine **1002** (commercially available, 30.97 g, 38.6 mL, 563.0 mmol, 1.5 equiv) at 25 °C, and the resulting reaction mixture was warmed up to gentle reflux at 100 - 110 °C for 21 h. When TLC and HPLC/MS showed that the reaction was complete, the reaction mixture was cooled down to room temperature before being concentrated *in vacuo* to remove the excess amount of propargylamine and solvent. The oily residue was then treated with 30% ethyl acetate/hexanes (v/v, 260 mL), and the resulting mixture was warmed up to reflux and stirred at reflux for 30 min before being cooled down to room temperature for 1 h. The pale-yellow solids were then collected by filtration, washed with 30% ethyl acetate/hexanes (v/v, 2 × 100 mL), and dried *in vacuo* at 40 °C for overnight to afford the crude, cycloaddition product (78.8 g, 81.75 g theoretical, 96.4%) as a mixture of two regioisomers, C-[1-(4-methoxy-benzyl)-1H-[1,2,3]triazol-4-yl]-methylamine and C-[3-(4-methoxy-benzyl)-3*H*-[1,2,3]triazol-4-yl]-methylamine (**1003** and **1004**), in a ratio of 1.2 to 1 by $^1$H NMR. The crude cycloaddition product was found to be essentially pure and the two regioisomers were not separated before being used directly in the subsequent reaction without further purification. For **1003** and **1004**: $^1$H NMR (300 MHz, DMSO-$d_6$) δ 1.82 (br. s, 2H, $NH_2$), 3.72 and 3.73 (two s, 3H, Ar-$OCH_3$), 5.47 and 5.53 (two s, 2H, $ArCH_2$), 6.89 and 6.94 (two d, 2H, J= 8.7Hz, Ar-H), 7.17 and 7.29 (two d, 2H, J= 8.7 Hz, Ar-H), 7.58 and 7.87 (two br. s, 1H, triazole-CH); $C_{11}H_{14}N_40$, LCMS (EI) *m*/*e* 219 ($M^+$ + H) and 241 ($M^+$ + Na).

**4-({*tert*-Butoxycarbonyl- [1-(4-methoxy-benzyl)-1H-[1,2,3]triazol-4-ylmethyl]-amino}-methyl)-phenylboronic acid and 4-({*tert*-Butoxycarbonyl-[3-(4-methoxy-benzyl)-3*H*-[1,2,3]triazol-4-ylmethyl]-amino}-methyl)-phenylboronic acid (1008** and **1009).** *Method A*. A solution of the regioisomeric C-[1-(4-methoxy-benzyl)-1H-[1,2,3]triazol-4-yl]-methylamine and C-[3-(4-methoxy-benzyl)-3*H*-[1,2,3]triazol-4-yl]-methylamine (**1003** and **1004,** 20.0 g, 91.74 mmol) in 1,2-dichloroethane (DCE, 280 mL) was treated with 4-formylphenylboronic acid **1005** (commercially available, 12.39 g, 82.57 mmol, 0.9 equiv) at room temperature, and the resulting reaction mixture was stirred at room temperature for 10 min. Sodium triacetoxyborohydride ($NaB(OAc)_3H$, 29.2 g, 137.6 mmol, 1.5 equiv) was then added to the reaction mixture in three portions over the period of 1.5 h at room temperature, and the resulting reaction mixture was stirred at room temperature for an additional 3.5 h. When TLC and HPLC/MS showed that the reductive amination reaction was complete, the reaction mixture was concentrated *in vacuo.* The residue, which contained a regioisomeric mixture of 4-({[1-(4-methoxy-benzyl)- 1H-[ 1,2,3]triazol-4-ylmethyl]-amino}-methyl)-phenylboronic acid and 4-({[3-(4-methoxy-benzyl)-3*H*-[1,2,3]triazol-4-ylmethyl]-amino}-methyl)-phenylboronic acid as the reductive amination products (**1006** and **1007**), was then treated with tetrahydrofuran (THF, 100 mL) and water (water, 100 mL). The resulting solution was subsequently treated with solid potassium carbonate ($K_2CO_3$, 37.98 g, 275.2 mmol, 3.0 equiv) and di-*tert*-butyl dicarbonate ($BOC_2O$, 20.02 g, 91.74 mmol, 1.0 equiv) at room temperature and the reaction mixture was stirred at room temperature for 2 h. When TLC and HPLC/MS showed that the N-BOC protection reaction was complete, the reaction mixture was treated with ethyl acetate (EtOAc, 150 mL) and water (water, 100 mL). The two layers were separated, and the aqueous layer was extracted with ethyl acetate (50 mL). The combined organic extracts were washed with water (50 mL), 1.5 N aqueous HCl solution (2 × 100 mL), water (100 mL), and saturated aqueous NaCl solution (100 mL), dried over $MgSO_4$, and concentrated *in vacuo.* The crude, regioisomeric 4-({*tert*-butoxycarbonyl-[1-(4-methoxy-benzyl)-1*H*-[1,2,3]triazol-4-ylmethyl] -amino } -methyl)-phenylboronic acid and 4-({*tert*-butoxycarbonyl-[3-(4-methoxy-benzyl)-3*H*-[ 1,2,3]triazol-4-ylmethyl]-amino}-methyl)-phenylboronic acid **(1008** and **1009,** 35.98 g, 37.32 g, 96.4%) was obtained as a pale-yellow oil, which solidified upon standing at room temperature *in vacuo.* This crude material was directly used in the subsequent reaction without further purification. For **1008** and **1009:** $^1H$ NMR (300 MHz, DMSO-$d_6$) $\delta$ 1.32 and 1.37 (two br. s, 9H, $COOC(CH_3)_3$), 3.70, 3.73 and 3.74 (three s, 3H, Ar-$OCH_3$), 4.07 - 4.39 (m, 4H), 5.49 and 5.52 (two s, 2H), 6.70 - 8.04 (m, 9H, Ar-H and triazole-CH); $C_{23}H_{29}BN_4O_5$, LCMS (EI) *m/e* 453 ($M^+$ + H) and 475 ($M^+$+ Na).

Method B. A solution of the regioisomeric C-[1-(4-methoxy-benzyl)-1*H*-[1,2,3]triazol-4-yl]-methylamine and C-[3-(4-methoxy-benzyl)-3*H*-[1,2,3]triazol-4-yl]-methylamine (**1003** and **1004,** 20.06 g, 92.0 mmol) in tetrahydrofuran (THF, 300 mL) was treated with 4-formylphenylboronic acid (13.11 g, 87.4 mmol, 0.95 equiv) at room temperature, and the resulting reaction mixture was stirred at room temperature for 10 min. Sodium triacetoxyborohydride ($NaB(OAc)_3H$, 29.25 g, 138.0 mmol, 1.5 equiv) was then added to the reaction mixture in three portions over the period of 1.5 h at room temperature, and the resulting reaction mixture was stirred at room temperature for an additional 3.5 h. When TLC and HPLC/MS showed that the reductive amination reaction was complete, the reaction mixture, which contained a regioisomeric mixture of 4-({[1-(4-methoxy-benzyl)-1H-[1,2,3]triazol-4-ylmethyl]-amino}-methyl)-phenylboronic acid and 4-({[3-(4-methoxy-benzyl)-3*H*-[1,2,3]triazol-4-ylmethyl]-amino}-methyl)-phenylboronic acid as the reductive amination products (1006 and 1007), was then treated with water (water, 200 mL). The resulting aqueous solution was subsequently treated with solid potassium carbonate ($K_2CO_3$, 38.0 g, 276 mmol, 3.0 equiv) and di-*tert*-butyl dicarbonate ($BOC_2O$, 20.08 g, 92 mmol, 1.0 equiv) at room temperature and the reaction mixture was stirred at room temperature for 2 h. When TLC and HPLC/MS showed that the N-BOC protection reaction was complete, the reaction mixture was treated with ethyl acetate (EtOAc, 150 mL) and water (water, 100 mL). The two layers were separated, and the aqueous layer was extracted with ethyl acetate (50 mL). The combined organic extracts were washed with water (50 mL), 1.5 N aqueous HCl solution (2 $\times$ 100 mL), water (100 mL), and saturated aqueous NaCl solution (100 mL), dried over $MgSO_4$, and concentrated *in vacuo.* The crude, 4-({*tert*-butoxycarbonyl-[1 -(4-methoxy-benzyl)-1*H*-[1,2,3 ]triazol-4-ylmethyl]-amino}-methyl)-phenylboronic acid and 4-({*tert*-butoxycarbonyl-[3-(4-methoxy-benzyl)-3*H*-[1,2,3]triazol-4-ylmethyl]-amino}-methyl)-phenylboronic acid (**1008** and **1009,** 38.45 g, 39.50 g, 97.3%) was obtained as a pale-yellow oil, which solidified upon standing at room temperature *in vacuo.* This crude material was found to be essentially identical in every comparable aspect as the material obtained from Method A and was directly used in the subsequent reaction without further purification.

**(5*S*)-{4'-[5-(Acertylamino-methyl)-2-oxo-oxazolidin-3-yl-2'-fluoro-biphenyl-4-ylmethyl}-[1-(4-methoxy-benzyl)-1H-[1,2,3]triazol-4-ylmethyl]-carbamic acid *tert*-butyl ester and (5*S*)-{4'-[5-(Acetylamino-methyl)-2-oxo-oxazolidin-3-yl]-2'-fluoro-biphenyl-4-ylmethyl}-[1-(4-methoxy-benzyl)-1H-[1,2,3]triazol-5-ylmethyl]-carbamic acid *tert*-butyl ester** (**1011** and **1012**). A suspension of the crude regioisomeric mixture of 4-({*tert*-butoxycarbonyl-[1-(4-methoxybenzyl)-1*H*-[1,2,3]triazol-4-ylmethyl]-amino}-methyl)-phenylboronic acid and 4-({*tert*-butoxycarbonyl-[3-(4-methoxy-benzyl)-3*H*-[ 1,2,3]triazol-4-ylmethyl]-amino}-methyl)-phenylboronic acid (**1008** and **1009**, 37.62 g, 83.23 mmol) and N-[3-(3-fluoro-4-iodo-phenyl)-2-oxo-oxazolidin-5-ylmethyl]-acetamide (**1010**, 28.32 g, 74.9 mmol, 0.90 equiv) in toluene (150 mL) was treated with powder $K_2CO_3$ (34.45 g, 249.7 mol, 3.0 equiv), EtOH (50 mL), and water (50 mL) at 25 °C, and the resulting mixture was degassed three times under a steady stream of Argon at 25 °C. $Pd(PPh_3)_4$ (866 mg, 0.749 mmol, 0.01 equiv) was subsequently added to the reaction mixture, and the resulting reaction mixture was degassed three times again under a steady stream of Argon at 25 °C before being warmed up to gentle reflux for 18 h. When TLC and HPLC/MS showed the coupling reaction was complete, the reaction mixture was cooled down to room temperature before being treated with water (100 mL) and ethyl acetate (100 mL). The two layers were then separated, and the aqueous layer was extracted with EtOAc (100 mL). The combined organic extracts were washed with water (50 mL), 1.5 N aqueous HCl solution (2 $\times$ 150 mL), water (100 mL), and the saturated aqueous NaCl solution (100 mL), dried over $MgSO_4$, and concentrated *in vacuo.* The residual oil was solidified upon standing at room temperature *in vacuo* to afford the crude, (5*S*)-{4'-[5-(acetylamino-methyl)-2-oxo-oxazolidin-3-yl]-2'-fluorobiphenyl-4-ylmethyl}-[1-(4-methoxy-benzyl)-1*H*-[ 1,2,3]triazol-4-ylmethyl]-carbamic acid *tert*-butylester (1011) and (5*S*)-{4'-[5-(acetylamino-methyl)-2-oxo-oxazolidin-3-yl]-2'-fluorobiphenyl-4-ylmethyl}-[1-(4-methoxy-benzyl)-1H-[1,2,3]triazol-5-ylmethyl]-carbamic acid *tert*-butylester (**1012**) as a regioisomeric mixture. This crude product (43.36 g, 49.28 g theoretical, 88%) was used directly in the subsequent reaction without further purification. For the mixture of **1011** and **1012:** $^1H$ NMR (300 MHz, DMSO-$d_6$) $\delta$ 1.35 and 1.38 (two br. s, 9H, $COO(CH_3)_3$), 1.85 (s, 3H, $COCH_3$), 3.45 (t, 2H, J= 5.4 Hz), 3.73 and 3.76 (two s, 3H, Ar-$OCH_3$), 3.79 (dd, 1H, J= 6.6, 9.1 Hz), 4.18 (t, 1H, J= 9.1 Hz), 4.35 - 4.43 (m, 4H), 4.73 - 4.81 (m, 1H), 5.50 (br. s, 2H), 6.90 and 6.98 (two d, 2H, J= 8.7 Hz), 7.28 and 7.32 (two d, 2H, J= 8.7 Hz), 7.35 (dd, 2H, J= 2.2, 8.6 Hz), 7.42 (dd, 1H, J= 2.2, 8.6 Hz), 7.49 - 7.63 (m, 4H, aromatic-H), 7.90 and 7.99 (two br. s, 1H, triazole-CH), 8.29 (t, 1H, J= 5.8 Hz, $NHCOCH_3$); $C_{35}H_{39}FN_6O_6$, LCMS (EI) m/e 659 ($M^+$ + H) and 681 ($M^+$ + Na).

**(5*S*)-N-{3-[2-Fluoro-4'-({[1-(4-methoxy-benzyl)-1*H*-[1,2,3]triazol-4-ylmethyl]-amino}-methyl)-biphenyl-4-yl]-2-oxo-oxazolidin-5-ylmethyl}-acetamide Hydrochloride (1013) and (5*S*)-N-{3-[2-Fluoro-4'-({[1-(4-methoxy-benzyl)-1H-[1,2,3]triazol-5-ylmethyl]-amino}-methyl)-biphenyl-4-yl]-2-oxo-oxazolidin-5-ylmethyl}-acetamide Hydrochloride (1014).** A solution of a regioisomeric mixture of (5*S*)-{4'-[5-(acetylamino-methyl)-2-oxo-oxazolidin-3-yl]-2'-fluoro-biphenyl-4-methyl}-[1-(4-methoxy-benzyl)-1*H*-[1,2,3]triazol-4-ylmethyl]-carbamic acid *tert-butyl* ester and

(5*S*)-{4'-[5-(acetylamino-methyl)-2-oxo-oxazolidin-3-yl]-2'-fluorobiphenyl-4-ylmethyl}-[1-(4-methoxy-benzyl)-1*H*-[1,2,3]triazol-5-ylmethyl] -carbamic acid *tert-butyl* ester (**1011** and **1012**, 37.28 g, 56.65 mmol) in ethyl acetate (EtOAc, 150 mL) and methanol (MeOH, 30 mL) was treated with a solution of 4 N hydrogen chloride in 1,4-dioxane (113.3 mL, 453.2 mmol, 8.0 equiv) at room temperature, and the resulting reaction mixture was stirred at room temperature for 12 h. When TLC and HPLC/MS showed that the N-BOC deprotection reaction was complete, the solvents were removed *in vacuo.* The residue was then suspended in 250 mL of 5% methanol (MeOH) in acetonitrile ($CH_3CN$), and the resulting slurry was stirred at room temperature for 1 h. The solids were then collected by filtration, washed with toluene (2 × 100 mL) and 5% methanol in acetonitrile (2 × 50 mL), and dried *in vacuo* to afford a regioisomeric mixture of the crude, (5*S*)-N-{3-[2-fluoro-4'-({[1-(4-methoxybenzyl)-1H-[1,2,3]triazol-4-ylmethyl]-amino}-methyl)-biphenyl-4-yl]-2-oxo-oxazolidin-5-ylmethyl}-acetamide hydrochloride and (5*S*)-N-{3-[2-fluoro-4'-({[1-(4-methoxy-benzyl)-1*H*-[1,2,3]triazol-5-ylmethyl] -amino } -methyl)-biphenyl-4-yl] -2-oxo-oxazolidin-5-ylmethyl}-acetamide hydrochloride (**1013** and **1014**, 30.0 g, 33.68 g theoretical, 89.1% yield) as off-white crystals in a ratio of 1.2 to 1. This material was found by $^1H$ NMR and HPLC/MS to be essentially pure and was directly used in the subsequent reactions without further purification. For the regioisomeric mixture of **1013** and **1014**: $^1H$ NMR (300 MHz, DMSO-$d_6$) δ 1.84 (s, 3H, $COCH_3$), 3.44 (t, 2H, J = 5.4 Hz), 3.71 and 3.74 (two s, 3H, Ar-$OCH_3$), 3.80 (dd, 1H, J= 6.6, 9.1 Hz), 4.17 (t, 1H, J= 9.1 Hz), 4.23 -4.30 (m, 4H), 4.73 -4.80 (m, 1H), 5.58 and 5.70 (two s, 2H), 6.88 and 6.93 (two d, 2H, J= 8.7 Hz), 7.15 and 7.32 (two d, 2H, J = 8.7 Hz), 7.43 (dd, 2H, J= 2.2, 8.6 Hz), 7.52 - 7.62 (m, 6H, aromatic-H), 8.28 (s, 1H, triazole-CH), 8.32 (t, 1H, J= 5.8 Hz, $NHCOCH_3$), 9.91 and 10.32 (two br. s, 2H, $ArCH_2N^+H_2$) ; $C_{30}H_{31}FN_6O_4$, LCMS (EI) *m/e 559* ($M^+$ + H) and 581 ($M^+$ + Na).

**(5*S*)-N-[3-(2-Fluoro-4'-{[(1H-[1,2,3]triazol-4-ylmethyl)-amino]-methyl}-biphenyl-4-yl)-2-oxo-oxazolidin-5-ylmethyl]-acetamide (1) (radezolid).** A solution of the crude regioisomeric mixture of (5*S*)-N-{3-[2-fluoro-4'-({[1-(4-methoxy-benzyl)-1H-[1,2,3]triazol-4-ylmethyl]-amino}-methyl)-biphenyl-4-yl]-2-oxo-oxazolidin-5-ylmethyl}-acetamide hydrochloride and (5*S*)-N-{3-[2-fluoro-4'({[1-(4-methoxy-benzyl)-1H-[1,2,3]triazol-5-ylmethyl]-amino}-methyl)-biphenyl-4-yl]-2-oxo-oxazolidin-5-ylmethyl}-acetamide hydrochloride (**1013** and **1014**, 29.17 g, 49.07 mmol) in trifluoroacetic acid (TFA, 150 mL) was warmed up to 65 - 70 °C, and the resulting reaction mixture was stirred at 65 - 70 °C for 12 h. When TLC and HPLC/MS showed that the deprotection reaction was complete, the solvents were removed *in vacuo.* The residual solids were then treated with ethyl acetate (EtOAc, 100 mL) and water (150 mL) before being treated with a saturated aqueous solution of sodium carbonate (30 mL) at room temperature. The resulting mixture was then stirred at room temperature for 1 h before the solids were collected by filtration, washed with EtOAc (2 × 50 mL) and water (2 × 50 mL), and dried *in vacuo* at 40 - 45 °C to afford the crude, (5S)-N-[3-(2-fluoro-4'-{[(1*H*-[1,2,3]triazol-4-ylmethyl)-amino]-methyl}-biphenyl-4-yl)-2-oxo-oxazolidin-5-ylmethyl]-acetamide (1 as the free base, 18.9 g, 21.49 g theoretical, 87.9%) as an off-white powder, which by HPLC/MS and $^1H$ NMR was found to be one pure regioisomer and this regioisomer was found to be identical to the material obtained from deprotection of 1013 alone by the same method. For 1 as the free base: $^1H$ NMR (300 MHz, DMSO-$d_6$) δ 1.85 (s, 3H, $COCH_3$), 3.44 (t, 2H, J= 5.4 Hz), 3.74 (s, 2H), 3.77 (s, 2H), 3.79 (dd, 1H, J= 6.4, 9.2 Hz), 4.17 (t, 1H, J= 9.1 Hz), 4.72-4.81 (m, 1H), 7.39- 7.62 (m, 7H, aromatic-H), 7.73 (s, 1H, triazole-CH), 8.29 (t, 1H, J= 5.8 Hz, $NHCOCH_3$), 9.72 (br. s, 2H, $ArCH_2N^+H_2$), 15.20 (hr. s, 1H, triazole-NH); $C_{22}H_{23}FN_6O_3$, LCMS (EI) *m/e* 439 ($M^+$ + H) and 461 ($M^+$ +Na); DSC onset melt at 208.4 °C.

**(5*S*)-N-[3-(2-Fluoro-4'-{[(1H-[1,2,3]triazol-4-ylmethyl)-amino]-methyl}-biphenyl-4-yl)-2-oxo-oxazolidin-5-ylmethyl]-acetamide chloride (1 hydrochloride salt).** A suspension of **1** free base (18.0 g, 41.1 mmol) in ethyl acetate (EtOAc, 80 mL), and methanol (MeOH, 20 mL) was treated with a solution of 4.0 N hydrogen chloride in 1,4-dioxane (41.1 mL, 164.4 mmol, 4.0 equiv) at room temperature, and the resulting mixture was stirred at room temperature for 8 h. The solvents were then removed *in vacuo,* and the residue was further dried *in vacuo* before being treated with a mixture of 10% methanol in acetonitrile (80 mL). The solids were collected by filtration, washed with 10% MeOH/acetonitrile (2 × 40 mL), and dried *in vacuo* to afford 1 hydrochloride salt (18.13 g, 19.50 g theoretical, 93% yield) as off-white crystals; DSC endotherm at 266 °C; onset melt at 261 °C.

**Recrystallization of Radezolid Hydrochloride.** The crude 1 hydrochloride salt was recrystallized from acetonitrile and water according to the following procedure: A suspension of the crude 1 hydrochloride salt (50.0 g) in acetonitrile (1250 mL) was warmed up to reflux before the distilled water (water, 280 mL) was gradually introduced to the mixture. The resulting clear yellow to light brown solution was then stirred at reflux for 10 min before being cooled down to 45 - 55 °C. The solution was then filtered through a Celite bed at 45 - 55 °C, and the filtrates were gradually cooled down to room temperature before being further cooled down to 0 - 5 °C in an ice bath for 1 h. The solids were then collected by filtration, washed with acetonitrile (2 × 50 mL), and dried *in vacuo* at 40 °C for 24 h to afford the recrystallized 1 hydrochloride salt (42.5 g, 50.0 g theoretical, 85% recovery) as off-white crystals. For 1: $^1H$ NMR (300 MHz, DMSO-$d_6$) δ 1.86 (s, 3H, $COCH_3$), 3.45 (t, 2H, J= 5.4 Hz), 3.84 (dd, 1H, J= 6.4, 9.2 Hz), 4.19 (t, 1H, J= 9.1 Hz), 4.24 (br. s, 2H), 4.31(br. s, 2H), 4.74-4.79 (m, 1H), 7.44(dd, 1H, J= 2.2, 8.6 Hz), 7.57 - 7.66 (m, 6H, aromatic-H), 8.17 (s, 1H, triazole-CH), 8.30 (t, 1H, J= 5.8 Hz, $NHCOCH_3$), 9.72 (br. s, 2H, $ArCH_2N^+H_2$), 15.20 (br. s, 1H, triazole-NH); $^{13}C$ NMR (75 MHz, DMSO-$d_6$) δ 22.57, 40.69, 41.50, 47.36, 49.23, 71.85, 105.70 (d, J= 28.5 Hz), 114.14 (d, J = 2.9 Hz), 122.29 (d, J = 13.3 Hz), 128.82 (d, J= 3.0 Hz), 130.70, 130.94, 131.0, 131.22, 135.30, 137.92 (br. s), 139.66 (d, J=11.2 Hz), 154.11,159.13 (d, J= 243.5 Hz), 170.19; $C_{22}H_{23}FN_6O_3$ - HCl, LCMS (EI) *m/e* 439 ($M^+$ + H) and 461 ($M^+$ + Na);

FTIR $cm^{-1}$ 3300, ~3400-~2300, 3003, 2933, 2810, 2779, 1730, 1654, 1552, 1502, and 807; DSC endotherm at 266 °C; onset melt at 261 °C.

**Radezolid Sulfate Salt.** To approximately 1 g of radezolid free base was added 15 mL of acetonitrile/water (2/3 by volume) to generate a radezolid slurry. To the radezolid slurry was added 1 equivalent of sulfuric acid dissolved in water. The resulting clear solution was evaporated on a rotary evaporator with water bath setting at 50 °C. The solution formed a white wet gel. The wet gel was dried under a nitrogen stream. 10 mL of methanol was added to dissolve the gel. The gel remained undissolved. 35 mL of DCM was added subsequently, and the gel-like solid turned into a fluffy white solid. The mixture was further mixed on a slurry wheel overnight. The solid was collected by vacuum filtration on a paper filter, and dried under reduced pressure for approximately 30 min. Yielded approximately 0.7 g of crystalline (low crystallinity) salt. (Note: There was some loss during the rotary evaporation). DSC endotherm at 72 and 166 °C; onset melt at 158 °C.

**Radezolid Tosylate Salt.** To approximately 1 g of radezolid free base was added 20 mL of water to generate a radezolid slurry. To the radezolid slurry was added 1 equivalent of toluenesulfonic acid dissolved in methanol at ambient temperature. The radezolid dissolved upon mixing. Some tacky solid formed and attached to the wall of the glass vial. As another 0.5 equivalents of acid added to the mixture, more solid formed. Solid was scraped off the vial. The mixture was further mixed on a slurry wheel overnight. A white soft precipitation formed along the wall of the vial. A white chunky solid formed upon addition of 20 mL of acetone. A solid was collected by vacuum filtration. Yielded approximately 0.4 g of crystalline salt. DSC endotherm at 97 and 173 °C; onset melt at 168 °C.

**Radezolid Esylate Salt.** To approximately 1.1 g of radezolid free base was added 15 mL of acetonitrile/water (2/3 by volume) to generate a radezolid slurry. To the radezolid slurry was added approximately 1.2 equivalents of acid dissolved in methanol. The resulting clear solution was evaporated on a rotary evaporator with water bath setting at 50 °C. Glassy gel was generated. The resulting white foamy gel was resuspended in 35 mL of acetone. The mixture was further mixed on a slurry wheel overnight. A white solid was collected by vacuum filtration on a paper filter and dried under reduced pressure for approximately 15 min. Yielded approximately 1.3 g of crystalline salt. DSC endotherm at 57 and 222 °C; onset melt at 216 °C.

**Radezolid Ethanedisulfonate Salt.** To approximately 1.1 g of radezolid free base was added 15 mL of acetonitrile/water (2/3 by volume) to generate a radezolid slurry. To the radezolid slurry was added 0.74 equivalents of acid dissolved in methanol. The resulting clear solution was evaporated on a rotary evaporator with water bath setting at 50 °C. The resulting clear gel was resuspended in 8 mL of methanol, and sonicated. A white gel-like solid formed after sonication. 60 mL of DCM was added subsequently. A white solid formed. The mixture was further mixed on a slurry wheel overnight. A white precipitate was collected by vacuum filtration on a paper filter and dried under reduced pressure for approximately 20 min. Yielded approximately 1.4 g of crystalline salt. DSC endotherm at 204 °C; onset melt at 198 °C.

**Radezolid Pyroglutamate Salt.** To approximately 1.1 g of radezolid free base was added 15 mL of acetonitrile/water (2/3 by volume) to generate an API slurry. To the API slurry was added 1.5 equivalents of pyroglutamic acid dissolved in water. The resulting clear solution was evaporated on a rotary evaporator with water bath setting at 50 °C, resulted in a clear gel. The gel was resuspended in acetone in a final volume of approximately 35 mL. The mixture was further mixed on a slurry wheel overnight. The solid was collected by vacuum filtration on a paper filter, dried under reduced pressure for 30 min. Yielded approximately 1.3 g of amorphous salt. DSC endotherm at 80 and 118 °C; exotherm at 128 °C; $T_g$ of 79 °C.

**Radezolid Mesylate Salt.** To approximately 1.1 g of radezolid free base was added 15 mL of acetonitrile/water (2/3 by volume) to generate a radezolid slurry. To the radezolid slurry was added 1.4 equivalents of acid dissolved in water. The resulting clear solution was evaporated on a rotary evaporator with water bath setting at 50 °C, resulted in a mixture of clear gel and white solid. To the mixture was added 30 mL of acetone. The suspension was further mixed on a slurry wheel overnight. Solid was collected by vacuum filtration on a paper filter, rinsed briefly with acetone, and dried under reduced pressure for approximately 5 min. Yielded approximately 1.3 g of crystalline salt. DSC endotherm at 82 and 203 °C; onset melt at 198 °C.

**Radezolid Mesylate Salt (25 g scale).** A sample of radezolid free base (23.5 g, 53.6 mmol) was suspended in a mixture of water (200 mL) and acetonitrile (20 mL) at room temperature. Neat methanesulfonic acid (3.56 mL, 54.9 mmol) was added. As the acid was added, the slurry thinned and became clear with a few solid particles. Stirring was continued at room temperature for 1 h until all particles had dissolved. The solution was transferred to a rotovap with the water bath set at 50 °C, and approximately 22 mL of solvent was stripped at 100 mBar. The warm solution was cooled in an ice bath and a white precipitate formed. After holding at 0 °C for approximately 1 h, the precipitate was collected by filtration, and the filter cake was washed with cold water (20 mL). The filter cake was then dried in a vacuum oven for 48 h (house vacuum ca. 100 mBar, 65 °C). The radezolid mesylate salt was obtained as a white powder in 90 % yield (25.7 g). An estimate of 0.75 mol % water content was made by $^1H$ NMR in $d_6$-DMSO, controlling for the amount of water present in the deuterated solvent. DSC endotherm at 82 and 203 °C; onset melt at 198 °C.

*Characterization of Radezolid Salts*

The thermal properties were evaluated by differential scanning calorimetry (DSC) and thermogravimetric analysis (TGA) as shown in Tables 5-6 below.

DSC data were collected on a TA Instruments 2910 DSC. In general, samples in the mass range of 1 to 10 mg were crimped in aluminum sample pans and scanned from 25 to about 300 °C at 10 °C/minute using a nitrogen purge at 50 mL/min.

TGA data were collected on a TA Instruments 2950 TGA. In general, samples in the mass range of 5 to 15 mg were placed in an open, pre-tarred platinum sample pan and scanned from 25 to about 150 °C at 10 °C/minute using a nitrogen purge.

Dynamic vapor sorption/desorption (DVS) data were collected on a VTI SGA-100 Vapor Sorption Analyzer. Sorption and desorption data were collected over a range of 5% to 95% relative humidity (RH) at 10% RH intervals under a nitrogen purge. Samples were not dried prior to analysis. Equilibrium criteria used for analysis were less than 0.0100% weight change in 5 minutes, with a maximum equilibration time of 3 hours if the weight criterion was not met. Data were not corrected for the initial moisture content of the samples. Sodium chloride and polyvinylpyrrolidine (PVP) were used as calibration standards.

**Thermal Characterization of Radezolid Salts**

**Table 5. Differential Scanning Calorimetry of Radezolid Salts**

| Salt Form | Appearance | DSC Peak Temperature (°C) Desolvation | DSC Onset Temperature (°C) Melt |
|---|---|---|---|
| Free base | White solids | N/A | 208.4 |
| Acetate | White solids | N/A | 207.4 |
| Ascorbate | Yellow clear | N/A | N/A |
| Benzoate | White solids | 106.7 | 180.4 |
| Citrate | White solids | N/A | N/A |
| Fumarate | White solids | 155.7 | 217.5 |
| Hydrochloride | White solids | N/A | 245.8 |
| Lactate | White solids | 122.6 | 195.9 |
| Maleate | White solids | N/A | 147.3 |
| Mesylate | White solids | 100.6 | 189.3 |
| Phosphate | White solids | N/A | 178.4 |
| Salicylate | White solids | 82.1, 131.5 | 180.5 |
| Succinate | White solids | N/A | 166.7 |
| Sulfate | White solids | N/A | 180.5 |
| Tartrate | White solids | 88.7, 140.6 | 210.7 |

**Table 6. Thermal Characterization of Radezolid Salts**

| Salt | DSC Results | TGA Results | DVS Results |
|---|---|---|---|
| Esylate | Endo: 57 and 222 °C (onset: 216 °C) | 0.2933% weight loss up to 200 °C | Sorption: 0.22% weight loss upon equilibration at 5% RH; 2.930% weight gain from 5 to 95% RH<br>Desorption: 2.999% weight loss from 95 to 5% RH |

(continued)

| Salt | DSC Results | TGA Results | DVS Results |
|---|---|---|---|
| Ethane-1,2-disulfonate | Endo: 204 °C (onset: 198 °C) | 0.5052% weight loss up to 200 °C | Sorption: 0.017% weight loss upon equilibration at 5% RH; 2.355% weight gain from 5 to 95% RH<br>Desorption: 2.388% weight loss from 95 to 5% RH |
| Hydrochloride | Endo: 266 °C (onset: 261 °C) | 0.1759% weight loss up to 220 °C | Sorption: 0.03% weight gain upon equilibration at 5% RH; 0.064% weight gain from 5 to 95% RH<br>Desorption: 0.069% weight loss from 95 to 5% RH |
| Mesylate | Endo: 82 and 203 °C (onset 198 °C) | 2.774% weight loss up to 200 °C (corresponding to 0.8 moles of water) | Sorption: 0.114% weight loss upon equilibration at 5% RH; 1.025% weight gain from 5 to 95% RH<br>Desorption: 0.835% weight loss from 95 to 5% RH |
| Pyroglutamate | Endo: 80 and 118 °C; Exo: 128 °C; Tg: 79 °C | 3.262% weight loss up to 150 °C | Sorption: 2.801% weight loss upon equilibration at 5% RH; 23.962% weight gain from 5 to 95% RH<br>Desorption: 24.936% weight loss from 95 to 5% RH |
| Sulfate | Endo: 72 and 166 °C (onset: 158 °C) | 1.915% weight loss up to 140 °C (corresponding to 0.6 moles of water) | Sorption: 0.654% weight loss upon equilibration at 5% RH; 11.527% weight gain from 5 to 95% RH<br>Desorption: 11.504% weight loss from 95 to 5% RH |
| Tosylate | Endo: 97 and 173 °C (onset: 168 °C) | 5.464% weight loss up to 100 °C (corresponding to 2 moles of water) | Sorption: 0.070% weight loss upon equilibration at 5% RH; 0.435% weight gain from 5 to 95% RH<br>Desorption: 0.435% weight loss from 95 to 5% RH |

**Solubility of Radezolid Salts**

The solubilities of the various radezolid salts was determined and are shown in Table 7 below. Approximately 2-3.5 mg of each salt was weighed into a glass vessel at ambient temperature. HPLC grade water, 200 μL, was added to each and stirred with a stir bar for two hours. Samples were inspected for solubility both visually and/or microscopically using a Carl Zeiss SV8 stereomicroscope. Additional solvent was added periodically until the solute completely dissolved or the solubility became <0.04 mg/mL.

**Table 7. Solubility of Radezolid Primary Isolated Salts**

| Salt Form | Salt (mg) | Volume (mL) | Solubility (mg/mL) |
|---|---|---|---|
| Fumarate | 3.13 | 1.20 | 2.6 |
| Citrate | 2.56 | 1.00 | 2.6 |
| Maleate | 3.61 | 1.60 | 2.3 |
| Tartrate | 2.88 | 1.60 | 1.8 |
| Phosphate | 3.58 | 3.40 | 1.1 |
| Ascorbate | 2.51 | 75.80 | <0.03 |

(continued)

| Salt Form | Salt (mg) | Volume (mL) | Solubility (mg/mL) |
|---|---|---|---|
| Benzoate | 2.47 | 82.20 | <0.03 |
| Salicylate | 3.61 | 122.20 | <0.03 |
| Succinate | 2.36 | 82.20 | <0.03 |
| Hydrochloride | 2.38 | 82.20 | <0.03 |
| Acetate | 2.21 | 118.20 | <0.02 |
| Lactate | 2.61 | 118.20 | <0.02 |
| Free base | 2.26 | 116.20 | <0.02 |

Solubilities of the radezolid free base and radezolid HCl salt in various solvents were estimated and are shown in Tables 8 and 9, respectively, below. A weighed sample was treated with aliquots of test solvent at ambient temperature. Complete dissolution of the test material was determined by visual inspection. Solubility was estimated based on the total solvent used to provide complete dissolution. The actual solubility may be greater than the approximate solubility calculated because of the use of solvent aliquots that were too large or due to a slow rate of dissolution. The solubility is expressed as "less than" if dissolution did not occur during the experiment. If complete dissolution was achieved as a result of only one aliquot addition, the solubility is expressed as "greater than." The approximate solubilities were rounded to the nearest whole number.

**Table 8. Approximate Solubilities of Radezolid Free Base**

| Solvent | Approximate Solubility (mg/mL) |
|---|---|
| Acetone | <1 |
| Acetonitrile | <1 |
| Dichloromethane | <1 |
| Ethyl acetate | <1 |
| Ethyl ether | <1 |
| Hexafluoroisopropanol (HFIPA) | ≥245 |
| HFIPA/Methanol (1:3 by volume) | <1 |
| Hexamethyl-phosphoramide | <26 |
| Methanol | <1 |
| 2,2,2-trifluoroethanol (TFE) | ≥54 |
| Tetrahydrofuran (THF) | <1 |
| Water | <1 |

**Table 9. Approximate Solubilities of Radezolid HCl Salt**

| Solvent | Approximate Solubility (mg/mL) |
|---|---|
| 1-Butanol[b] | <2 |
| 2-Butanone[b] | <2 |
| Dioxane[b] | <2 |
| Ethanol[b] | <2 |
| Hexafluoroisopropanol (HFIPA) | ≥225 |
| Methanol[b] | <2 |

(continued)

| Solvent | Approximate Solubility (mg/mL) |
|---|---|
| Propionitrile[b] | <2 |
| 2-Propanol[b] | <2 |
| Tert-butanolb | <5 |
| Tetrahydrofuran (THF)[b] | <2 |
| 2,2,2-trifluoroethanol (TFE) | ≥6 |
| HFIPA/Dioxane (9:1) | ≥52 |
| HFIPA/Ethanol (5:1) | ≥80 |
| HFIPA/THF | ≥74 |
| [b] Material never went into solution. | |

In another set of experiments, solubility of various radezolid salts was determined by HPLC. The results are provided in Table 10 below. Approximately 1 mL of saturated solution (with excess of solid in solution) of each salt was prepared in water. The mixture was further mixed overnight on a slurry wheel then centrifuged on a desktop centrifuge at maximum speed for approximately 5 min. The supernatant was collected, and pH was measured. Solubility was determined at the native pH. Then the pH was adjusted to approximately 3.5 with sodium hydroxide solution or an acid solution in which salt was generated. The supernatant was filtered through a 0.2 μm nylon syringe filter and diluted into 0.1% formic acid solution immediately after filtration for HPLC.

All HPLC analyses were performed using an Agilent 1100 series liquid chromatograph equipped with a diode array detector, degasser, quaternary pump, and an autosampler. The chromatographic column was a 4.6 × 150 mm SymmetryShield RP18 column with 3.5 μm packing (Waters). The column temperature was set to 30 °C, and the detector wavelength was 270 nm with a bandwidth of 8 nm and a reference wavelength of 360 nm. The mobile phase A was 0.1% formic acid in water (HPLC grade), mobile phase B was 0.1 % formic acid in methanol (HPLC grade). Flow rate was 1.2 mL/min and the column was equilibrated with 85% mobile phase A and 15% mobile phase B. The injection volume was 5 μL. The elution program was as follows: After sample was injected, the initial gradient was run from 85% mobile phase A, 15 % mobile phase B to 100% mobile phase B in 25 min, followed by 3 min with 100% mobile phase B, then a reverse gradient was run from 100% mobile phase B to 15% mobile phase B in 2 min, then with another 10 min of equilibration run with 85% mobile phase A and 15% mobile phase B.

**Table 10. Solubility Determination of Radezolid Salts by HPLC at 23 °C**

| Salt | Solubility at Native pH (mg/mL) | Native pH | Solubility at Adjusted pH (mg/mL) | Adjusted pH |
|---|---|---|---|---|
| Esylate | 53.8 | 4.0 | 61.7 | 3.5 |
| Ethane-1,2-disulfonate[C] | >133.2 | 2.0 | >116.9 | 3.6 |
| HCl Salt | 1.7 | 4.6 | 2.0 | 3.5 |
| Mesylate | 18.6 | 4.1 | 18.0 | 3.5 |
| Pyroglutamate[C] | >250 | N/A | N/A | N/A |
| Sulfate | 4.0 | 1.9 | 3.3 | 3.6 |
| Tosylate | 0.9 | 5.1 | 0.4 | 3.4 |
| [C] Solution was not saturated. | | | | |

The solubility of radezolid free base in various oily excipients was measured at 19-21 °C by adding a known amount of the free base to a known amount of the oily excipients. Increasing amounts of the free base were added if the oily excipient was not saturated. The results are shown in Table 11.

**Table 11. Radezolid Free Base Solubility in Various Oily Excipients.**

| Oily Excipient | Chemistry | Solubility, % w/w |
|---|---|---|
| Diisopropyl adipate (DIA) | Ester | <1.5 |
| Benzyl benzoate (BB) | Aromatic and ester | <1.5 |
| Mineral oil | Hydrocarbon | <1 |
| Cyclomethicone | Silicon | <0.75 |
| Oleic acid | Acidic oil | <0.75 |

In some embodiments, facial acne formulations are disclosed herein. In some embodiments, the amount of oil in an emulsion base (cream or lotion) is between 10-15% w/w. In further embodiments, the amount of oil in an emulsion base (cream or lotion) is less than 10%. To obtain a 1% w/w formulation of the free base dissolved in a dispersed oil phase, its solubility in that oil phase needs to be approximately 10% w/w. Radezolid free base was screened in oily excipients at a level of approximately 5% w/w. If an oily excipient could not dissolve at least 5% w/w, it would be unlikely to contribute to higher solubility in other oily excipients (i.e. low potential for synergy). None of the hydrophobic solvents tested, which had a wide range of chemistries, had over 1.5% w/w radezolid free base.

The solubility of radezolid HCl salt in various approved topical solvents was measured at 19-21 °C by adding a known amount of the radezolid HCl salt to a known amount of the solvent. Increasing amounts of the radezolid HCl salt were added if the solvent was not saturated. The results are shown in Table 12.

**Table 12. Radezolid HCl Salt Solubility in Various Solvents/Solvent Blends.**

| Solvent/Solvent Blend | Solubility, % w/w |
|---|---|
| Ethanol | <0.2 |
| 50% Ethanol/50% water | <0.6 |
| 50% Ethanol/50% phosphate buffer (pH=7.4) | <0.2 |
| Phosphate buffer (pH=7.4) | <0.2 |
| Glycerin | <0.2 |
| Propylene glycol | <0.2 |
| Diethylene glycol monoethyl ether (Transcutol®) | <0.2 |
| Hexylene glycol | <0.2 |
| PEG 300 | <0.2 |

The solubility of radezolid esylate and mesylate salts in various approved topical solvents was measured at 19-21 °C by adding a known amount of the radezolid salts to a known amount of the solvent. Increasing amounts of the radezolid salts were added if the solvent was not saturated. The results are shown in Table 13.

**Table 13. Radezolid Esylate and Mesylate Salt Solubilities in Topical Solvents**

| Solvent | Esylate solubility, % w/w | Mesylate solubility, % w/w |
|---|---|---|
| Propylene glycol | 1.1-1.5% | 2.7-3.1 |
| Glycerin | <0.2 | <0.2 |
| Hexylene glycol | <0.1 | <0.2 |
| Transcutol | <0.1 | <0.2 |
| PEG 300 | 0.3-0.5 | 0.3-0.5 |

The radezolid esylate and mesylate salts were most soluble in propylene glycol. Propylene glycol can be used as a nonvolatile penetration enhancer. Without wishing to be bound by theory, the inventors posit that it may be able to maintain the salts in solution after the water evaporates and provide a good thermodynamic driving force for permeation.

## EXAMPLE 6: Topical Formulations of Radezolid

### *Preparation of Topical Formulations of Radezolid*

A topical composition is prepared by combining the components in Table 14 utilizing conventional mixing techniques.

**Table 14. Topical Radezolid Formulation**

| Ingredients | % Weight |
|---|---|
| Deionized Water | q.s. to 100 |
| Ethanol (SD 40B Alcohol) | 35.0 |
| Radezolid | 0.5-10 |
| Salicylic Acid (Optional) | 2.0 |
| Dexpanthenol (Optional) | 3.0 |

In a suitable vessel the antibiotic compound is dissolved in ethanol with stirring. Next, if being added, the optional salicylic acid is dissolved in this ethanol mixture with stirring. If being used, the dexpanthenol is dissolved in a separate vessel in the water with stirring. The water, or if being used, the dexpanthenol solution is combined with the alcohol mixture with mixing.

### Topical Gel

A topical composition is prepared by combining the components in Table 15 utilizing conventional mixing techniques.

**Table 15. Topical Radezolid Formulation**

| Ingredients | % Weight |
|---|---|
| Deionized Water | q.s. to 100 |
| Ethanol (SD 40B Alcohol) | 35.0 |
| Radezolid | 0.5-10 |
| Hydrophilic Gelling Agent | 3.0 |

In a suitable vessel the antibiotic compound is dissolved in ethanol with stirring. Next, the hydrophilic gelling agent is dissolved in a separate vessel in the water with stirring. The hydrophilic gelling agent solution is combined with the alcohol mixture with mixing.

This composition is useful for topical application for the treatment of acne or other skin infections caused or mediated by *Propionibacterium acnes, Gardnerella vaginalis,* or *Staphylococcus aureus.*

### Topical Cream

Topical creams can be prepared by combining the various components utilizing conventional mixing techniques. In exemplary embodiments, the mesylate creams described in Table 17 were prepared as follows:

- Mix methylparaben, propylparaben, and propylene glycol in one vessel.
- Mix cetostearyl alcohol, Cetomacrogol 1000, cyclomethicone, and dimethicone in a second vessel.
- Mix the water, citric acid, and glycerin in a third vessel.
- Mix the radezolid mesylate salt with the contents of the third vessel.
- Adjust the pH of the contents of the third vessel using NaOH.
- Add the contents of the first vessel to the third vessel.
- Add the contents of the second vessel to the third vessel.
- Mix until homogeneous.

## EXAMPLE 7: Topical Cream and Gel Formulations of Radezolid

### *Development of Topical Cream and Gel Formulations of Radezolid*

A radezolid mesylate salt topical formulation was prepared as a solution. The solubility of the radezolid mesylate salt is approximately 18 mg/mL, or 1.8% w/w, at pH 3.5. A 1% solution of the radezolid mesylate salt was slightly hazy and had a pH of 5.4. Adding citric acid to reduce the pH down to 4.0 produced a clear and homogeneous solution. A radezolid HCl salt topical formulation was prepared as a suspension.

Two vehicles were developed for the radezolid HCl salt: one with carbomer (an anionic polymer that requires a neutral-basic pH for thickening) and one with hydroxyethylcellulose (HEC, a polymer that has no net charge and is stable at low and high pHs). For the radezolid mesylate salt, a cream vehicle and a HEC gel vehicle were developed at pH 4.0. Cream formulation screening focused on using cyclomethicone and dimethicone as the oil phase to provide a smooth skin feel and some emollience. Cyclomethicone is volatile, so it can also contribute to a "dry" feel shortly after application. Both of these silicone-based excipients are considered to be low for comedogenicity (*i.e.,* tendency to clog pores and encourage the formation of blackheads). Several ratios of cyclomethicone and dimethicone were evaluated to produce optimal skin feel after application. All of the formulations contained 10% propylene glycol as a nonvolatile penetration enhancer and 2.0% glycerin as a moisturizing agent. Parabens were used for microbial preservation; however, radezolid does not require preservatives, and preservative-free formulations are also possible.

The following variables were screened for the radezolid formulations:

- Citrate buffer compositions for pH 4.0
- Trolamine buffer compositions for pH 7.5
- Cetostearyl alcohol and ceteareth-20 concentrations for creams
- Cyclomethicone and dimethicone concentrations for creams
- Hydroxyethylcellulose concentrations for gels
- Carbomer concentration and amount of trolamine for neutralization for gels

The vehicles for topical formulations in Table 16 were developed. The ingredients are shown in % w/w.

**Table 16. Vehicles for Topical Formulations**

| Ingredient | Role | Cellulose Gel 1 | Cellulose Gel 2 | Carbomer Gel | Cream 1 | Cream 2 |
|---|---|---|---|---|---|---|
| Glycerin | Moisturizing agent | 2.0 | 2.0 | 2.0 | 2.0 | 2.0 |
| Propylene Glycol | Penetration enhancer | 10.0 | 10.0 | 10.0 | 10.0 | 10.0 |
| Methylparaben | Preservative | 0.15 | 0.15 | 0.15 | 0.15 | 0.15 |
| Propylparaben | Preservative | 0.05 | 0.05 | 0.05 | 0.05 | 0.05 |
| Water | Solvent | q.s. to 100% | q.s. to 100% | q.s. to 100% | q.s. to 100% | q.s. to 100% |
| Trolamine | Buffering agent | N/A | 0.075 | 0.70 | N/A | N/A |
| 1 N HCl | pH modifier | N/A | 0.357 | N/A | N/A | N/A |
| Citric acid | Buffering agent | 0.105 | N/A | N/A | 0.105 | 0.105 |
| 1 N NaOH | pH modifier | 0.52 | N/A | N/A | 0.52 | 0.52 |
| Hydroxyethylcellulose | Thickener | 1.25 | 1.75 | N/A | N/A | N/A |
| Carbomer 980 | Thickener | N/A | N/A | 0.50 | N/A | N/A |
| Cetostearyl alcohol | Emulsion stabilizer | N/A | N/A | N/A | 5.0 | 4.0 |

(continued)

| Ingredient | Role | Cellulose Gel 1 | Cellulose Gel 2 | Carbomer Gel | Cream 1 | Cream 2 |
|---|---|---|---|---|---|---|
| Ceteareth-20 | Nonionic surfactant | N/A | N/A | N/A | 1.0 | 1.0 |
| Dimethicone | Cosmesis enhancer | N/A | N/A | N/A | 1.0 | 1.0 |
| Cyclomethicone | Cosmesis enhancer | N/A | N/A | N/A | 2.0 | 2.0 |

It was found that the pH does not affect the consistency of cellulose gels. Carbomer gels are not compatible with salts, such as NaCl or $CaCl_2$. All of the vehicles in Table 16 were found to be stable at 40 °C for at least 3.5 weeks and have shown no signs of syneresis (*i.e.,* separation of the liquid out of the formulation). The radezolid HCl salt was formulated with the Cellulose Gel 2 vehicle, with slight variations thereof. The radezolid mesylate salt was formulated with the Cellulose Gel 2 vehicle and the Cream 2 vehicle, with slight variations thereof. The resulting topical formulations of radezolid are shown in Table 17 (with ingredients shown in wt/wt %) were then developed.

**Table 17. Topical Formulations**

| Ingredient | Role | HCl Gel 1 | HCl Gel 2 | HCl Gel 3 | mesylate Gel 1 | mesylate Gel 2 | mesylate Cream |
|---|---|---|---|---|---|---|---|
| Radezolid HCl Salt | API | 1.08 | 1.08 | 1.08 | N/A | N/A | N/A |
| Radezolid mesylate salt | API | N/A | N/A | N/A | 1.22 | 1.22 | 1.22 |
| Trolamine | Buffering agent | 0.45 | 0.45 | 0.45 | N/A | N/A | N/A |
| Citric acid monohydrate | Buffering agent | N/A | N/A | N/A | 0.11 | 0.22 | 0.11 |
| Methylparaben | Preservative | 0.15 | 0.15 | 0.15 | 0.15 | 0.15 | 0.15 |
| Propylparaben | Preservative | 0.05 | 0.05 | 0.05 | 0.05 | 0.05 | 0.05 |
| Propylene glycol | Penetration enhancer | 10.0 | 10.0 | 10.0 | 10.0 | 10.0 | 10.0 |
| Glycerin | Moisturizing agent | 2.0 | 2.0 | 2.0 | 2.0 | 2.0 | 2.0 |
| Polysorbate 80 | Emulsifier | 0.01 | 0.01 | 0.01 | N/A | N/A | N/A |
| NaCl | Salt | 1.0 | 1.0 | 0.5 | N/A | N/A | N/A |
| Natrosol HXX | Thickener | 1.75 | 1.25 | 1.75 | 1.75 | 1.75 | N/A |
| 1.0 N HCl | pH modifier | q.s. to pH 7.5 | q.s. to pH 7.5 | q.s. to pH 7.5 | N/A | N/A | N/A |
| 1.0 N NaOH | pH modifier | N/A | N/A | N/A | q.s. to pH 4 | q.s. to pH 4 | q.s. to pH 4 |
| Cetostearyl alcohol | Emulsion stabilizer | N/A | N/A | N/A | N/A | N/A | 4.0 |
| Cetomacrogol 1000 | Nonionic surfactant | N/A | N/A | N/A | N/A | N/A | 1.0 |
| Cyclomethicone | Cosmesis enhancer | N/A | N/A | N/A | N/A | N/A | 2.0 |
| Dimethicone | Cosmesis enhancer | N/A | N/A | N/A | N/A | N/A | 1.0 |

(continued)

| Ingredient | Role | HCl Gel 1 | HCl Gel 2 | HCl Gel 3 | mesylate Gel 1 | mesylate Gel 2 | mesylate Cream |
|---|---|---|---|---|---|---|---|
| Purified water | Solvent | q.s. to 100 | q.s. to 100 | q.s. to 100 | q.s. to 100 | q.s. to 100 | q.s. to 100 |

***Stability Testing of Topical Formulations of Radezolid***

The formulations in Table 17 were compounded at the 250-300 g scale. Their stability was studied at (i) zero time, (ii) 3 months at 2-8 °C, (iii) 1 month at 25 °C, (iv) 3 months at 25 °C, (v) 6 months at 25 °C, (vi) 1 month at 40 °C, (vii) 3 months at 40 °C, and (viii) 6 months at 40 °C.. The results of the studies are shown in Figures 5 and 6.

There was no significant change in pH, viscosity, appearance, or paraben assays for all formulations at 1 month at 25 °C or 40 °C. No significant new peaks were observed in the radezolid mesylate salt formulations. There were some new peaks observed in the radezolid HCl salt formulations, with larger peaks at 40 °C, but these are most likely related to paraben degradation products at the higher pH as they were also seen in the vehicle formulations.

There were no significant changes in pH, viscosity, appearance, or paraben assays after 3 months at 25 °C or 40 °C. There may be a pH drop for radezolid HCl salt formulations with higher storage temperatures. No significant new peaks were observed in the radezolid mesylate salt formulations. The API Assay for the radezolid HCl salt formulations trended down with higher storage temperatures. Additional peaks were seen in the radezolid HCl salt formulations, but these are most likely related to paraben degradation products at the higher pH as they were also seen in the vehicle formulations. The general trend that higher storage temperatures result in a lower pH and lower API Assay values for the radezolid HCl salt formulations could be correlated: more of the radezolid HCl salt dissolving lowers the pH and presents more API in solution for degradation.

There was a slight drop in viscosity for the radezolid HCl salt formulations at 40 °C, but not at 25 °C. There could be some very slight degradation of the polymer at the higher pH and temperature. There was no significant change in viscosity for the radezolid mesylate salt gels. The radezolid mesylate salt cream showed an increase in viscosity, which is normal for creams on storage, as it is undesirable for their viscosities to drop. This process proceeded a little slower for the radezolid mesylate salt cream, which may be due to having silicon excipients in the dispersed phase *(i.e.,* it is oil-free). The cream was still soft and spreadable. There was no significant change in paraben assays. There were no significant new peaks observed in the API Assay for the radezolid mesylate salt formulations. The API Assay for the radezolid HCl salt formulations appeared mostly unchanged from the 3 month time point.

**EXAMPLE 8: Topical Cream Formulations of Radezolid**

***Development of Topical Cream Formulations of Radezolid***

The exemplary topical cream formulations of radezolid in Table 18 were developed and produced as follows:

- Mix required amounts of purified water, anhydrous citric acid, and glycerin in Main vessel.
- Mix requisite amount of radezolid mesylate into Main vessel until dissolved.
- Adjust pH of contents of Main vessel to between pH 3.8 and pH 4.2 slowly using IN sodium hydroxide solution while mixing.
- Mix requisite amounts of propylene glycol, methylparaben, and propylparaben in Paraben Phase vessel until parabens are dissolved.
- Add contents of Paraben Phase vessel to Main vessel.
- Begin heating Main vessel to 60 °C while mixing.
- Add requisite amounts of cetostearyl alcohol, polyoxyl 20 cetostearyl alcohol, cyclomethicone, and dimethicone to Oil Phase vessel.
- Mix and heat Oil Phase vessel to 60 °C.
- Add contents of Oil Phase vessel to Main vessel when both are at 60 °C.
- Begin mixing Main vessel with high shear homogenizer.
- Cool Main vessel to 40 °C while mixing with high shear homogenizer.
- Discontinue operation of high shear homogenizer.
- Cool to ≤ 30 °C while mixing.

Table 18. Topical Cream Formulations of Radezolid

| Ingredient | Grade | Function | 0.5% Mesylate Cream (% w/w) | 0.75% Mesylate Cream (% w/w) | 1% Mesylate Cream (% w/w) | 1.8% Mesylate Cream (% w/w) |
|---|---|---|---|---|---|---|
| Radezolid Mesylate | N/A | Active Ingredient | 0.61 | 0.91 | 1.22 | 2.20 |
| Propylene Glycol | USP | Penetration Enhancer | 10.00 | 10.00 | 10.00 | 10.00 |
| Cetostearyl Alcohol | NF | Emulsion Stabilizer | 4.00 | 4.00 | 4.00 | 4.00 |
| Glycerin | USP | Moisturizing Agent | 2.00 | 2.00 | 2.00 | 2.00 |
| Cyclomethicone | NF | Cosmesis Enhancer | 2.00 | 2.00 | 2.00 | 2.00 |
| Polyoxyl 20 Cetostearyl Ether | NF | Emulsion Stabilizer | 1.00 | 1.00 | 1.00 | 1.00 |
| Dimethicone | NF | Cosmesis Enhancer | 1.00 | 1.00 | 1.00 | 1.00 |
| Methylparaben | NF | Preservative | 0.15 | 0.15 | 0.15 | 0.15 |
| Anhydrous Citric Acid | USP | Buffering Agent | 0.10 | 0.10 | 0.10 | 0.10 |
| Propylparaben | NF | Preservative | 0.05 | 0.05 | 0.05 | 0.05 |
| Sodium Hydroxide | NF | pH Modifier | q.s. to pH 3.8 to 4.2 | q.s. to pH 3.8 to 4.2 | q.s. to pH 3.8 to 4.2 | q.s. to pH 3.8 to 4.2 |
| Purified Water | USP | Solvent | q.s. to 100% (79.09) | q.s. to 100% (78.79) | q.s. to 100% (78.48) | q.s. to 100% (77.50) |

***Stability Testing of Topical Cream Formulations of Radezolid***

The stability of the exemplary topical cream formulations of radezolid shown in Table 18 were studied as shown in Tables 19-27.

**Table 19. Stability Data for 0.5% Mesylate Cream (Non-Clinical GLP) Stored at 25 °C and 60% Relative Humidity in a 300 mL Amber Glass Jar**

| Test | Method | Timepoint | |
|---|---|---|---|
| | | Initial | 4 Months |
| Appearance | Visual | White to off-white smooth cream | White to off-white smooth cream |
| Radezolid Mesylate: Identification | TM-0732-01/C | Positive | Positive |
| Radezolid Mesylate: Assay | TM-0732-01/C | % w/w %Label Claim<br>Top 0.515 103.0<br>Mid 0.513 102.6<br>Bot 0.511 102.2<br>Avg 0.513 102.6 | % w/w %Label Claim<br>Top 0.5096 101.9<br>Mid 0.5014 100.3<br>Bot 0.4930 98.6<br>Avg 0.5013 100.3 |
| Uniformity in Container | TM-0732-01/C | 0.4% RSD | 1.7% RSD |
| Methylparaben: Assay | TM-0732-01/C | 101% Label Claim | 98% Label Claim |
| Propylparaben: Assay | TM-0732-01/C | 102% Label Claim | 98% Label Claim |
| Viscosity | DVIII Rheometer CP40 Spindle 10 RPM 2 minutes USP <911> | 340 cP | 361 cP |
| pH | QI-020; USP <791> | 3.9 | 4.0 |
| Total Aerobic Microbial Count | QM-105; USP <61> | < 100 CFU/g | < 100 CFU/g |
| Total Yeasts and Molds Count | QM-105; USP <61> | < 10 CFU/g | < 10 CFU/g |
| Microbial Limits | QM-110; USP <62> | Absence of *Staphylococcus aureus*, *Pseudomonas aeruginosa*, and clinically significant Gram negative bacilli and beta-hemolytic *Streptococcus spp.* | Absence of *Staphylococcus aureus*, *Pseudomonas aeruginosa*, and clinically significant Gram negative bacilli and beta-hemolytic *Streptococcus spp.* |

**Table 20. Stability Data for 0.75% Mesylate Cream (Non-Clinical GLP) Stored at 25 °C and 60% Relative Humidity in a 300 mL Amber Glass Jar**

| Test | Method | Timepoint | |
|---|---|---|---|
| | | **Initial** | **4 Months** |
| Appearance | Visual | White to off-white smooth cream | White to off-white smooth cream |
| Radezolid Mesylate: Identification | TM-0732-01/C | Positive | Positive |
| Radezolid Mesylate: Assay | TM-0732-01/C | % w/w %Label Claim<br>Top 0.772 102.9<br>Mid 0.760 101.3<br>Bot 0.767 102.3<br>Avg 0.766 102.2 | % w/w %Label Claim<br>Top 0.7507 100.1<br>Mid 0.7552 100.7<br>Bot 0.7490 99.9<br>Avg 0.7516 100.2 |
| Uniformity in Container | TM-0732-01/C | 0.8% RSD | 0.4% RSD |
| Methylparaben: Assay | TM-0732-01/C | 101% Label Claim | 96% Label Claim |
| Propylparaben: Assay | TM-0732-01/C | 101% Label Claim | 95% Label Claim |
| Viscosity | DVIII Rheometer<br>CP40 Spindle<br>10 RPM<br>2 minutes<br>USP <911> | 324 cP | 332 cP |
| pH | QI-020; USP <791> | 4.0 | 4.0 |
| Total Aerobic Microbial Count | QM-105; USP <61> | < 100 CFU/g | < 100 CFU/g |
| Total Yeasts and Molds Count | QM-105; USP <61> | < 10 CFU/g | < 10 CFU/g |
| Microbial Limits | QM-110; USP <62> | Absence of *Staphylococcus aureus*, *Pseudomonas aeruginosa*, and clinically significant Gram negative bacilli and beta-hemolytic *Streptococcus spp.* | Absence of *Staphylococcus aureus*, *Pseudomonas aeruginosa*, and clinically significant Gram negative bacilli and beta-hemolytic *Streptococcus spp.* |

**Table 21. Stability Data for 1% Mesylate Cream (Non-Clinical GLP) Stored at 25 °C and 60% Relative Humidity in a 300 mL Amber Glass Jar**

| Test | Method | Timepoint | |
|---|---|---|---|
| | | **Initial** | **4 Months** |
| Appearance | Visual | White to off-white smooth cream | White to off-white smooth cream |
| Radezolid Mesylate: Identification | TM-0732-01/C | Positive | Positive |
| Radezolid Mesylate: Assay | TM-0732-01/C | % w/w %Label Claim<br>Top 1.020 102.0<br>Mid 1.021 102.1<br>Bot 1.018 101.8<br>Avg 1.020 102.0 | % w/w %Label Claim<br>Top 0.9970 99.7<br>Mid 0.9706 97.1<br>Bot 0.9285 92.9<br>Avg 0.9654 96.6 |
| Uniformity in Container | TM-0732-01/C | 0.1% RSD | 3.6% RSD |
| Methylparaben: Assay | TM-0732-01/C | 101% Label Claim | 98% Label Claim |
| Propylparaben: Assay | TM-0732-01/C | 101% Label Claim | 99% Label Claim |
| Viscosity | DVIII Rheometer<br>CP40 Spindle<br>10 RPM<br>2 minutes<br>USP <911> | 335 cP | 364 cP |
| pH | QI-020; USP <791> | 3.7 | 3.8 |
| Total Aerobic Microbial Count | QM-105; USP <61> | < 100 CFU/g | < 100 CFU/g |
| Total Yeasts and Molds Count | QM-105; USP <61> | < 10 CFU/g | < 10 CFU/g |
| Microbial Limits | QM-110; USP <62> | Absence of *Staphylococcus aureus*, *Pseudomonas aeruginosa*, and clinically significant Gram negative bacilli and beta-hemolytic *Streptococcus spp.* | Absence of *Staphylococcus aureus*, *Pseudomonas aeruginosa*, and clinically significant Gram negative bacilli and beta-hemolytic *Streptococcus spp.* |

**Table 22. Stability Data for 1% Mesylate Cream (Non-Clinical GLP) Stored at 25 °C and 60% Relative Humidity in a 500 mL Amber Glass Jar**

| Test | Method | Timepoint | |
|---|---|---|---|
| | | **Initial** | **4 Months** |
| Appearance | Visual | White to off-white smooth cream | White to off-white smooth cream |
| Radezolid Mesylate: Identification | TM-0732-01/C | Positive | Positive |
| Radezolid Mesylate: Assay | TM-0732-01/C | % w/w %Label Claim<br>Top 0.955 95.5<br>Mid 1.013 101.3<br>Bot 1.004 100.4<br>Avg 0.991 99.1 | % w/w %Label Claim<br>Top 1.008 100.8<br>Mid 1.016 101.6<br>Bot 1.025 102.5<br>Avg 1.014 101.4 |
| Uniformity in Container | TM-0732-01/C | 3.1% RSD | 0.8% RSD |
| Methylparaben: Assay | TM-0732-01/C | 101% Label Claim | 103% Label Claim |
| Propylparaben: Assay | TM-0732-01/C | 100% Label Claim | 102% Label Claim |
| Viscosity | DVIII Rheometer<br>CP40 Spindle<br>10 RPM<br>2 minutes<br>USP <911> | 366 cP | 330 cP |
| pH | QI-020; USP <791> | 3.9 | 3.9 |
| Total Aerobic Microbial Count | QM-105; USP <61> | < 100 CFU/g | < 100 CFU/g |
| Total Yeasts and Molds Count | QM-105; USP <61> | < 10 CFU/g | < 10 CFU/g |
| Microbial Limits | QM-110; USP <62> | Absence of *Staphylococcus aureus*, *Pseudomonas aeruginosa*, and clinically significant Gram negative bacilli and beta-hemolytic *Streptococcus spp.* | Absence of *Staphylococcus aureus*, *Pseudomonas aeruginosa*, and clinically significant Gram negative bacilli and beta-hemolytic *Streptococcus spp.* |

**Table 23. Stability Data for 1% Mesylate Cream (Non-Clinical GLP) Stored at 25 °C and 60% Relative Humidity in a 500 mL Amber Glass Jar**

| Test | Method | Timepoint | |
|---|---|---|---|
| | | **Initial** | **4 Months** |
| Appearance | Visual | White to off-white smooth cream | White to off-white smooth cream |
| Radezolid Mesylate: Identification | TM-0732-01/C | Positive | Positive |
| Radezolid Mesylate: Assay | TM-0732-01/C | % w/w %Label Claim<br>Top 0.974 97.4<br>Mid 0.994 99.4<br>Bot 0.986 98.6<br>Avg 0.985 98.5 | % w/w %Label Claim<br>Top 1.016 101.6<br>Mid 1.010 101.0<br>Bot 1.019 101.9<br>Avg 1.015 101.5 |
| Uniformity in Container | TM-0732-01/C | 1.0% RSD | 0.5% RSD |
| Methylparaben: Assay | TM-0732-01/C | 101% Label Claim | 103% Label Claim |
| Propylparaben: Assay | TM-0732-01/C | 100% Label Claim | 104% Label Claim |
| Viscosity | DVIII Rheometer<br>CP40 Spindle<br>10 RPM<br>2 minutes<br>USP <911> | 285 cP | 306 cP |
| pH | QI-020; USP <791> | 3.8 | 3.8 |
| Total Aerobic Microbial Count | QM-105; USP <61> | < 100 CFU/g | < 100 CFU/g |
| Total Yeasts and Molds Count | QM-105; USP <61> | < 10 CFU/g | < 10 CFU/g |
| Microbial Limits | QM-110; USP <62> | Absence of *Staphylococcus aureus*, *Pseudomonas aeruginosa*, and clinically significant Gram negative bacilli and beta-hemolytic *Streptococcus spp.* | Absence of *Staphylococcus aureus*, *Pseudomonas aeruginosa*, and clinically significant Gram negative bacilli and beta-hemolytic *Streptococcus spp.* |

**Table 24. Stability Data for 1.8% Mesylate Cream (Non-Clinical GLP) Stored at 25 °C and 60% Relative Humidity in a 500 mL Amber Glass Jar**

| Test | Method | Timepoint | |
|---|---|---|---|
| | | **Initial** | **4 Months** |
| Appearance | Visual | White to off-white smooth cream | White to off-white smooth cream |
| Radezolid Mesylate: Identification | TM-0732-01/C | Positive | Positive |
| Radezolid Mesylate: Assay | TM-0732-01/C | % w/w %Label Claim<br>Top 1.822 101.2<br>Mid 1.823 101.3<br>Bot 1.820 101.1<br>Avg 1.822 101.2 | % w/w %Label Claim<br>Top 1.750 97.2<br>Mid 1.758 97.7<br>Bot 1.779 98.8<br>Avg 1.762 97.9 |
| Uniformity in Container | TM-0732-01/C | 0.1% RSD | 0.8% RSD |
| Methylparaben: Assay | TM-0732-01/C | 101% Label Claim | 102% Label Claim |
| Propylparaben: Assay | TM-0732-01/C | 99% Label Claim | 100% Label Claim |
| Viscosity | DVIII Rheometer<br>CP40 Spindle<br>10 RPM<br>2 minutes<br>USP <911> | 324 cP | 296 cP |
| pH | QI-020; USP <791> | 3.6 | 3.6 |
| Total Aerobic Microbial Count | QM-105; USP <61> | < 100 CFU/g | < 100 CFU/g |
| Total Yeasts and Molds Count | QM-105; USP <61> | < 10 CFU/g | < 10 CFU/g |
| Microbial Limits | QM-110; USP <62> | Absence of *Staphylococcus aureus*, *Pseudomonas aeruginosa*, and clinically significant Gram negative bacilli and beta-hemolytic *Streptococcus spp.* | Absence of *Staphylococcus aureus*, *Pseudomonas aeruginosa*, and clinically significant Gram negative bacilli and beta-hemolytic *Streptococcus spp.* |

**Table 25. Stability Data for 1.8% Mesylate Cream (Non-Clinical GLP) Stored at 25 °C and 60% Relative Humidity in a 500 mL Amber Glass Jar**

| Test | Method | Timepoint | |
|---|---|---|---|
| | | **Initial** | **4 Months** |
| Appearance | Visual | White to off-white smooth cream | White to off-white smooth cream |
| Radezolid Mesylate: Identification | TM-0732-01/C | Positive | Positive |
| Radezolid Mesylate: Assay | TM-0732-01/C | % w/w %Label Claim<br>Top 1.824 101.3<br>Mid 1.821 101.2<br>Bot 1.833 101.8<br>Avg 1.826 101.4 | % w/w %Label Claim<br>Top 1.744 96.9<br>Mid 1.740 96.7<br>Bot 1.734 96.3<br>Avg 1.739 96.6 |
| Uniformity in Container | TM-0732-01/C | 0.3% RSD | 0.3% RSD |
| Methylparaben: Assay | TM-0732-01/C | 101% Label Claim | 102% Label Claim |
| Propylparaben: Assay | TM-0732-01/C | 100% Label Claim | 102% Label Claim |
| Viscosity | DVIII Rheometer<br>CP40 Spindle<br>10 RPM<br>2 minutes<br>USP <911> | 309 cP | 303 cP |
| pH | QI-020; USP <791> | 3.4 | 3.4 |
| Total Aerobic Microbial Count | QM-105; USP <61> | < 100 CFU/g | < 100 CFU/g |
| Total Yeasts and Molds Count | QM-105; USP <61> | < 10 CFU/g | < 10 CFU/g |
| Microbial Limits | QM-110; USP <62> | Absence of *Staphylococcus aureus*, *Pseudomonas aeruginosa*, and clinically significant Gram negative bacilli and beta-hemolytic *Streptococcus spp.* | Absence of *Staphylococcus aureus*, *Pseudomonas aeruginosa*, and clinically significant Gram negative bacilli and beta-hemolytic *Streptococcus spp.* |

**Table 26. Stability Data for 0.75% Mesylate Cream (Non-Clinical GLP) Stored at 25 °C and 60% Relative Humidity in a 60 g Aluminum Laminate Tube**

| Test | Time (months) | | | |
|---|---|---|---|---|
| | Initial[a] | 1 | 2 | 3 |
| Appearance | White to off-white smooth cream | White to off-white smooth cream | White to off-white smooth cream | White to off-white smooth cream |
| Radezolid: Identification | Positive | Positive | Positive | Positive |
| Radezolid: Assay | Average: 102.2 % Label Claim<br>Individual: 80.0 to 120.0% Label Claim | Average: 99.9 % Label Claim<br>Individual: 80.0 to 120.0% Label Claim | Average: 105.5% Label Claim<br>Individual: 80.0 to 120.0% Label Claim | Average: 102.1% Label Claim<br>Individual: 80.0 to 120.0% of Label Claim |
| Radezolid: Related Substances | Not Tested | Not Tested | Not Tested | Not Tested |
| Uniformity in Container | 0.8 RSD | 2.6 RSD | 2.7 RSD | 0.2 RSD |
| Methy lparaben | 101 % Label Claim | 96 % Label Claim | 104 % Label Claim | 100 % Label Claim |
| Propy lparaben | 101 % Label Claim | 95 % Label Claim | 103 % Label Claim | 98 % Label Claim |
| Viscosity | 324 cP | 332 | 442 | 343 |
| pH | 4.0 | 4.0 | 4.0 | 4.1 |
| Total Aerobic Microbial Count | <100 CFU/g | Not Tested | Not Tested | Not Tested |
| Total Yeasts and Molds Count | <10 CFU/g | Not Tested | Not Tested | Not Tested |
| Microbial Limits | Absence of *S. aureus, P. aeruginosa,* and clinically significant Gram negative bacilli and beta-hemolytic *Streptococcus spp.* | Not Tested | Not Tested | Not Tested |

**Table 27. Stability Data for 0.75% Mesylate Cream (Non-Clinical GLP) Stored at 40 °C and 75% Relative Humidity in a 60 g Aluminum Laminate Tube**

| Test | Time (months) | | | |
|---|---|---|---|---|
| | Initial[a] | 1 | 2 | 3 |
| Appearance | White to off-white smooth cream | White to off-white smooth cream | White to off-white smooth cream | White to off-white smooth cream |
| Radezolid: Identification | Positive | Positive | Positive | Positive |

(continued)

| Test | Time (months) | | | |
|---|---|---|---|---|
| | Initial[a] | 1 | 2 | 3 |
| Radezolid: Assay | Average: 102.2 % Label Claim<br><br>Individual: 80.0 to 120.0% Label Claim | Average: 99.0 % Label Claim<br>Individual: 80.0 to 120.0% Label Claim | Average: 105.8 % Label Claim<br>Individual: 80.0 to 120.0% Label Claim | Average: 101.6 % Label Claim<br>Individual: 80.0 to 120.0% Label Claim |
| Radezolid: Related Substances | Not Tested | Not Tested | Not Tested | Not Tested |
| Uniformity in Container | 0.8 RSD | 1.8 RSD | 0.7 RSD | 0.6 RSD |
| Methylparaben | 101 % Label Claim | 94 % Label Claim | 103 % Label Claim | 99 % Label Claim |
| Propy Iparaben | 101 % Label Claim | 92 % Label Claim | 102 % Label Claim | 99 % Label Claim |
| Viscosity | 324 cP | 382 cP | 335 cP | 450 cP |
| pH | 4.0 | 4.0 | 4.0 | 4.0 |
| Total Aerobic Microbial Count | <100 CFU/g | Not Tested | Not Tested | Not Tested |
| Total Yeasts and Molds Count | <10 CFU/g | Not Tested | Not Tested | Not Tested |
| Microbial Limits | Absence of S. *aureus, P. aeruginosa,* and clinically significant Gram negative bacilli and beta-hemolytic *Streptococcus spp.* | Not Tested | Not Tested | Not Tested |

The results for the Radezolid: Assay, the Radezolid: Related Substances, the Uniformity in Container, the Methylparaben, and the Propylparaben tests for Tables 19-27 were obtained with the TM-0732-01/C method. The TM-0732-01/C method is an HPLC reversed-phase procedure at 30 °C using a C18 column (Waters Symmetry Shield RP18, 3.5 μm, 4.6 × 100 mm) and a binary mobile phase of (A) 0.1% formic acid in water and (B) 0.1% formic acid in methanol at a flow rate of 1.4 mL/min. The injection volume was 5 μL and samples were injected at ambient temperature. The gradient program linearly increased the amount of mobile phase B from 15% (initial) to 100% (final) over 16 minutes. The pump was then held at 100% mobile phase B for 2 minutes prior to returning to the initial isocratic conditions. After returning to the starting conditions, the system was allowed to equilibrate for at least 7 minutes before making the next injection. Detection for this method was by UV at 270 nm using a reference wavelength of 380 nm. The autosampler flush was 1:1 water:acetonitrile. The start time of the gradient may be adjusted based on the dwell volume of the HPLC system used and elution time of the radezolid mesylate peak. This method is capable of detecting and quantitating 0.02 and 0.05 w/w % impurities, respectively.

The diluent for the samples and standard was prepared by combining 850 mL water, 150 mL acetonitrile, and 1.0 mL formic acid. The radezolid HCl standard was 0.5 mg/mL in diluent. If not completely dissolved with mixing by inversion and sonication, a few drops of formic acid were added to facilitate dissolution. The radezolid LOQ standard was 0.05 w/w% (0.25 μg/mL) in diluent. Radezolid mesylate samples were prepared at concentrations of 0.56 mg/mL in diluent. If not completely dissolved with mixing by inversion and sonication, a few drops of formic acid were added to facilitate dissolution.

The identity of radezolid was confirmed if the principal peak in the chromatogram of the sample corresponded to the retention time of the peak produced by the reference material within ± 2.5%. The expected retention time for radezolid is 5.05 minutes and the relative retention time is 1.00.

Quantitation of radezolid mesylate was performed by external standard calibration. The calibration factor was

calculated as follows:

$$\text{Calibration Factor (CF)} = \frac{A_{Std}}{\text{Conc}_{Std} \text{ x } P_{Std}},$$

where $A_{Std}$ is the area of the radezolid peak in the standard solution, $Conc_{Std}$ is the concentration of radezolid (μg/mL) in the standard solution, and $P_{Std}$ is the purity of radezolid (%) in the standard. The radezolid content (% w/w) was then determined as follows:

$$\text{RX-1741 content (\% w/w)} = \frac{A_{RX-1741} \text{ x } 100}{\text{Conc}_{Smp} \text{ x } CF} \text{ x } \frac{MW_{RX-1741Mesylate}}{MW_{RX-1741HCl}},$$

where $A_{Rx-1741}$ is the area of the radezolid peak in the sample solution, Concsmp is the concentration of radezolid in the sample solution (μg/mL), CF is the calibration factor (area/μg), $MW_{RX-174Mesylate}$ is 534.56 g/mol, and $MW_{RX-1741HCl}$ is 474.92 g/mol. The radezolid content can be corrected to water- and solvent-free basis as follows:

$$\frac{RX-1741\text{ \%w/w (either mesylate or HCl, as is) x } 100}{100 - [\text{water content (\%w/w)} + \text{total solvent content (\%w/w)}]}.$$

Quantitation of related impurities was performed by relative response factors (RRFs) to radezolid mesylate external standard. The impurity content was determined as follows:

$$\text{Impurity Content (\%w/w)} = \frac{A_{imp} \text{ x} 100}{\text{Conc}_{smp} \text{ x RRF x CF}},$$

where $A_{imp}$ is the peak area of the impurity, $Conc_{smp}$ is the concentration of the sample (mg/mL), RRF is the Relative Response Factor for the impurity, and CF is the calibration factor of radezolid (area/mg).

The other tests in Tables 19-27 were conducted in accordance with the USP. For example, tests in Tables 19-27 were conducted in accordance with "Microbiological Examination of Nonsterile Products: Microbial Enumeration Tests" in 2016 USP <61>; "Microbiological Examination of Nonsterile Products: Tests for Specified Microorganisms" in 2016 USP <62>; "pH" in 2016 USP <791>; and "Viscosity - Rotational Methods" in 2016 USP <912>, the contents of all of the foregoing are hereby incorporated by reference in their entireties.

## EXAMPLE 9: Franz Cell Diffusion Studies for Topical Formulations of Radezolid

The rate and extent of *in vitro* skin permeation of the topical formulations from Table 17 into and through intact human cadaver skin was studied using a Franz diffusion cell system.

### *Experimental Overview*

The high pressure liquid chromatography ("HPLC") analytical conditions developed on an Agilent 1200 HPLC with a variable wavelength detector ("VWD") for analyzing radezolid were as described in Table 28.

**Table 28. HPLC Conditions Used For Detecting Radezolid**

| | |
|---|---|
| Instrument: | Agilent 1200 VWD with quadratic pump |
| Column: | Eclipse C8: 5μm pore size, 4.6 × 150mm dimensions |
| Mobile Phase: | A: Water (HPLC grade) with 0.1% Formic Acid<br>B: Methanol (HPLC grade) with 0.1% Formic Acid |

(continued)

| Gradient: | Time (minute): | %B |
|---|---|---|
| | 0 | 10% |
| | 2 | 10% |
| | 6 | 95% |
| | 6.5 | 95% |
| Flow Rate: | 1.0 mL/min | |
| Column Temp: | 30 °C | |
| UV Detection: | 270nm | |
| Injection Vol: | 30μL | |
| Retention Times: | HCL and mesylate salt: approximately 5.55 minutes | |

The reagents in Table 29 were used during the course of the study.

**Table 29. Reagents Used During the Study**

| | Supplier | Catalog # | Lot # |
|---|---|---|---|
| Phosphate buffered saline (PBS) | Quality Biological | 119-069-491 | 721143 |
| Dimethyl sulfoxide (DMSO) | Macron | 2969-08 | 0000095948 |
| Methanol (MeOH) | Alfa Aesar | 22909 | X09A921 |
| Ultimata Gold XR | Perkin Elmer | 6013119 | 79-12511 |
| Tritiated water | Perkin Elmer | Net001B001 | 2046527 |
| Water (HPLC) | EMD | WX0008-1 | 55183 |
| Formic acid | Fluka | 56302 | BCBM7881V |

Intact human cadaver skin was purchased from New York Firefighters Tissue Bank (NY, NY). The skin was dermatomed by the tissue bank to a thickness of approximately 500μm and collected from the posterior torso. The donor information supplied by the tissue bank was: White, male, age: 59, COD: cardiomegaly, donor site: posterior torso. The donor ID# from the tissue bank was: WM092114. Upon receipt of the skin from the tissue bank, the skin was stored frozen at -20 °C until the morning of the experiment. Prior to use, the skin was removed from the freezer and allowed to fully thaw at room temperature. Only areas of the skin that were visually intact were used during the experiment.

Based on the results of solubility studies, a receptor fluid of phosphate buffered saline ("PBS") at pH 5.5 with 0.01wt% $NaN_3$ (added as a preservative) was chosen. The solubility of the actives in the receptor fluid was shown to be approximately 15 μg/mL for the mesylate salt and >100 μg/mL for the HCl salt, both of which are sufficient to maintain sink conditions for the flux study. The receptor solution was prepared at an appropriate pH and degassing was carried out by filtering the receptor fluid through a ZapCap CR 0.2 μm membrane while pulling vacuum.

Custom made Franz diffusion cells with a receptor volume of 3.3 mL were used for the experiment. The available diffusional surface area of the skin for each cell is $0.55cm^2$. The receptor fluid is maintained at 32 °C during the experiment using a stirring dry block heater and the fluid continuously agitated with a stir bar. The steps for assembling the diffusion cells are outlined below:

1. The cadaver skin was removed from the freezer and allowed to defrost in a bio-safety hood for 30 minutes. The skin was thoroughly defrosted prior to opening the package.
2. The cadaver skin was removed from the package and placed on the bio-safety hood countertop with the stratum corneum side up. The skin was patted dry with a Kimwipe, then sprayed with fresh PBS and patted dry again. This process was repeated 3 more times to remove any residues present on the skin.
3. The receptor wells were then filled with the degassed receptor fluid and a Teflon coated stir bar was added to each receptor well.
4. The defrosted cadaver skin was examined and only areas with even thickness and no visible damage to the

surface were used.

5. The skin was cut into approximately 2 cm × 2 cm squares.
6. The skin piece was centered on the donor cells, stratum corneum (SC) side up.
7. The skin was centered again and the edges flattened out. The donor and receptor wells were then aligned and clamped together with a pinch clamp.
8. Additional receptor fluid was added where necessary. Any air bubbles present were removed by tilting the cell, allowing air to escape along the sample port.
9. Diffusion cells were then placed in to the stirring dry block heaters and allowed to rehydrate for 20 minutes from the receptor fluid. The block heaters were maintained at 32 °C throughout the experiment with continuous stirring.
10. After 20 minutes, the surface of the skin was examined. If the skin is wet or shows signs of "sweating", the SC is considered compromised. No compromised skin pieces were identified.

Once the cells had been assembled and the skin allowed to hydrate for 20 minutes, the barrier integrity of each skin section was tested using a tritiated water test prior to the dosing of the formulation to the skin.

1. An aliquot of 150 μL of tritiated water (spiked with 25 μCi water/10 mL water) was added to each FDC donor well.
2. After 5 minutes, the tritiated water from the donor wells was removed and the skin tapped dry using a Kimwipe.
3. The receptor wells were agitated for an additional 1 hour after the tritiated donor fluid was removed.
4. After 1 hour of agitation, a 300 μL aliquot sample was taken from each receptor well. The remaining receptor fluid was discarded and replaced with fresh PBS (membrane integrity study uses only PBS in receptor fluid).
5. 600 μL of scintillation cocktail (Ultima Gold XR) was added to each sample aliquot.
6. The tritium content of the receptor-well aliquot was then measured using a liquid scintillation counter ("LSC").
7. After LSC analysis was complete, results were analyzed. Any FDCs showing anomalously high water flux were discarded. All cells were below this cut-off value for this experiment.
8. The FDCs were then ranked according to $H^3$ water flux *(i.e.,* how much tritium passed into the receptor-well). The FDCs were then distributed such that each formulation was assigned FDCs with nearly equivalent average tritiated water flux values.
9. Once the membrane integrity check study was complete, the entire receptor chamber volume was replaced with the receptor fluid.

After the membrane integrity test was complete, and the cells appropriately sorted, the formulations were applied to the stratum corneum of the skin. A one-time dosing regimen was used for this study. The test articles were applied as 5 μL doses to the skin using a positive displacement Nichiryo pipetter. The formulations were then spread across the surface of the skin using a glass rod. Donor cells were left uncapped during the experiment. The dose of radezolid per cell is shown in Table 30. The radezolid dose assumes a specific gravity of 1.0 for the formulation.

**Table 30. Radezolid Salt Concentration and Radezolid Dose Per Cell for Each Formulation**

| Formulation | wt/wt% Radezolid salt | Nominal formulation dose per cell | Radezolid salt dose per cell |
|---|---|---|---|
| HCl Gel 1 | 1.08 | 5 μL | 98.2 μg/cm$^2$ |
| HCl Gel 2 | 1.08 | 5 μL | 98.2 μg/cm$^2$ |
| HCl Gel 3 | 1.08 | 5 μL | 98.2 μg/cm$^2$ |
| mesylate Gel 1 | 1.22 | 5 μL | 110.91 μg/cm$^2$ |
| mesylate Gel 2 | 1.22 | 5 μL | 110.91 μg/cm$^2$ |
| mesylate Cream | 1.22 | 5 μL | 110.91 μg/cm$^2$ |

At 4, 8, and 24 hours, a 300 μL sample aliquot was drawn from the receptor wells using a graduated Hamilton type injector syringe. Fresh receptor medium was added to replace the 300 μL sample aliquot. The samples were then filtered with a 0.2 μm GHP filter membrane plate.

At 24 hours, the skin was washed with PBS/ethanol, then wiped cleaned using PBS/ethanol soaked KimWipes. After the residual formulation was wiped off and the skin tapped dry with KimWipes, the stratum corneum was tape stripped three times - each tape stripping consisting of applying cellophane tape to the skin with uniform pressure and peeling the tape off.

After the skin was tape stripped, the epidermis of each piece was then separated from the underlying dermal

tissue using tweezers. The epidermal and dermal tissues were collected and placed in 4 mL borosilicate glass vials. After all the skin pieces were separated, 2 mL of the extraction solvent (pure DMSO) was added to each vial. The vials were then allowed to incubate for 24 hours at 32 °C with gentle shaking. After 24 hours, sample aliquots were taken and filtered with the 0.20 μm GHP membrane filter plate.

Sample aliquots were analyzed using the analytical method as outlined above. Samples were stored frozen (at -20 °C) prior to analysis to prevent any unwanted degradation of the actives.

***Results***

The delivered dose results using all six replicates, including outliers, are shown in Table 31.

**Table 31. Delivered Doses of Radezolid (μg/cm²) (Including Outliers)**

| Time | HCl Gel 1 | HCl Gel 2 | HCl Gel 3 | mesylate Gel 1 | mesylate Gel 2 | mesylate Cream |
|---|---|---|---|---|---|---|
| 4 hours | 0.846 ± 0.148 | 0.726 ± 0.155 | 0.462 ± 0.156 | 0.461 ± 0.134 | 0.378 ± 0.066 | 0.660 ± 0.175 |
| 8 hours | 1.691 ± 0.361 | 1.245 ± 0.216 | 0.798 ± 0.272 | 0.788 ± 0.261 | 0.657 ± 0.152 | 1.081 ± 0.368 |
| 24 hours | 3.389 ± 0.776 | 2.781 ± 0.743 | 1.602 ± 0.587 | 1.744 ± 0.687 | 1.274 ± 0.307 | 1.825 ± 0.589 |
| Epidermis | 0.124 ± 0.015 | 0.106 ± 0.021 | 0.125 ± 0.018 | 0.467 ± 0.057 | 0.545 ± 0.087 | 2.631 ± 0.466 |
| Dermis | 0.332 ± 0.034 | 0.310 ± 0.027 | 0.296 ± 0.039 | 0.400 ± 0.027 | 0.352 ± 0.027 | 0.771 ± 0.127 |

Figure 7 graphically shows the comparative delivered dose from Table 31 of the different formulations for transdermal and dermal delivery.

The delivered dose results using all six replicates, excluding outliers, are shown in Table 32.

**Table 32. Delivered Doses of Radezolid (μg/cm²) (Excluding Outliers)**

| Time | HCl Gel 1 | HCl Gel 2 | HCl Gel 3 | mesylate Gel 1 | mesylate Gel 2 | mesylate Cream |
|---|---|---|---|---|---|---|
| 4 hours | 0.846 ± 0.148 | 0.726 ± 0.155 | 0.315 ± 0.062 | 0.461 ± 0.134 | 0.378 ± 0.066 | 0.660 ± 0.175 |
| 8 hours | 1.691 ± 0.361 | 1.245 ± 0.216 | 0.548 ± 0.126 | 0.788 ± 0.261 | 0.657 ± 0.152 | 1.082 ± 0.368 |
| 24 hours | 3.389 ± 0.776 | 2.781 ± 0.743 | 1.067 ± 0.285 | 1.744 ± 0.687 | 1.274 ± 0.307 | 1.825 ± 0.589 |
| Epidermis | 0.124 ± 0.015 | 0.086 ± 0.005 | 0.125 ± 0.018 | 0.467 ± 0.057 | 0.545 ± 0.087 | 2.631 ± 0.466 |
| Dermis | 0.332 ± 0.034 | 0.310 ± 0.027 | 0.296 ± 0.039 | 0.400 ± 0.027 | 0.352 ± 0.027 | 0.771 ± 0.127 |

Figure 8 graphically shows the comparative delivered dose from Table 32 of the different formulations for transdermal and dermal delivery.

In general, it is preferable to have some but not too much bioavailability in the receptor fluid to minimize systemic (*i.e.,* plasma) load. In general, drug in the dermis layer demonstrates the ability of the drug to reach the site of action. HCl Gel 1 and HCl Gel 2 delivered more radezolid transdermally than HCl Gel 3. The HCl formulations had significant levels of drug in the receptor phase and little in the dermis or epidermis, potentially due to follicular transport, which could present a problem for transport into blocked follicles. Although mesylate Gel 1, mesylate Gel 2 and the mesylate Cream all statistically delivered similar amounts of radezolid transdermally, the mesylate Cream delivered more radezolid into the skin than mesylate Gel 1 and mesylate Gel 2. The mesylate Cream demonstrates a good balance between the amount of drug delivered in the receptor phase compared to the dermis and epidermis.

## EXAMPLE 10: Use of Radezolid for Treating Skin Infections

Treatment with a variety of topical radezolid formulations can be used as a method to treat, prevent, or reduce the risk of various conditions caused or mediated by various radezolid-sensitive microbial pathogens. Conditions include skin and soft tissue infections (e.g., impetigo, rosacea, bacterial conjunctivitis, otitis externa, folliculitis, and local wound infections), acne, nosocomial or staph carrier nasal prophylaxis, and other bacterial infections such as bacterial vaginosis. Radezolid-sensitive microbial pathogens include *Propionibacterium acnes, Gardnerella vaginalis,* and *Staphylococcus aureus* (including Methicillin-resistant *Staphylococcus aureus).*

### *Proposed Formulations*

For uncomplicated skin and soft tissues infections, nasal colonization and acne, radezolid is formulated as a topical semi-solid dosage form (*e.g.,* ointment, cream, lotion, solution, foam or gel) for topical cutaneous applications.
Radezolid is formulated in creams or foams for intravaginal application to treat bacterial vaginosis caused by *Gardnerella vaginalis.*
Typical compositions that can be used include from 0.1 to 1500 mg of radezolid.

### *Mode of Administration and Duration of Treatment (Anticipated Ranges Based on Possible Effectiveness)*

For acne, topical application 1-2 times daily for 4 - 12 weeks is envisioned.
For uncomplicated skin and soft tissues infections caused by S. *aureus*/*MRSA (e.g.,* impetigo, rosacea, bacterial conjunctivitis, otitis externa, folliculitis, and local wound infections), topical application 1-2 times daily for days to 2 weeks is envisioned.
For nosocomial or staph carrier nasal prophylaxis including MRSA, topical application 1-2 times daily for 3-7 days is envisioned.
For bacterial vaginosis/vaginitis, intravaginal administration 1-2 times daily for 3-7 days is envisioned.

### *Endpoint of Treatment*

For acne, the endpoint is clearance or material reduction of inflammatory and/or non-inflammatory lesions.
For uncomplicated skin and soft tissues, the endpoint is resolution of all clinical signs and symptoms of infection (*e.g*., erythema, crusting, burning/stinging, pruritus etc.).
For nosocomial or staph carrier nasal prophylaxis including MRSA, the endpoint is a negative culture.
For bacterial vaginosis/vaginitis, the endpoint is resolution of all clinical signs and symptoms of infection (*e.g*., reversal of AMSEL criteria, vaginal discharge, odor, burning/stinging, pH ≤ 4.5, and elimination of clue cell(s)).
The disclosed methods are sufficient to meet the proposed endpoints.

### EQUIVALENTS

Scope of the invention is thus indicated by the appended claims rather than by the foregoing description, and all changes that come within the meaning and range of equivalency of the claims are intended to be embraced therein.

## Claims

**1.** A topical formulation comprising:

radezolid, or a pharmaceutically acceptable salt or tautomer thereof; and
a penetration enhancer.

**2.** The topical formulation of claim 1, wherein the penetration enhancer is selected from the group consisting of propylene glycol, hexylene glycol, diethylene glycol monoethyl ether, dimethyl sulfoxide, ethanol, isopropanol, oleic acid, laurocapram, d-limonene, ethyl acetate, and mixtures thereof.

**3.** The topical formulation of claim 1 further comprising:

an emulsion stabilizer;
a moisturizing agent;

a cosmesis enhancer;
a preservative;
a buffering agent;
a pH modifier; and
water.

**4.** The topical formulation of claim 1, further comprising a moisturizing agent.

**5.** The topical formulation of claim 4, wherein the moisturizing agent is selected from the group consisting of glycerin, urea, lactic acid, glycolic acid, hyaluronic acid, pyrrolidone carboxylic acid, and mixtures thereof.

**6.** The topical formulation of claim 1, further comprising a preservative.

**7.** The topical formulation of claim 6, wherein the preservative is selected from the group consisting of methylparaben, propyl paraben, benzyl alcohol, benzalkonium chloride, cetylpyridinium chloride, benzoic acid, sodium benzoate, potassium sorbate, and mixtures thereof.

**8.** The topical formulation of claim 1, further comprising a pH modifier.

**9.** The topical formulation of claim 1, further comprising a buffering agent.

**10.** The topical formulation of claim 9, wherein the buffering agent is selected from the group consisting of citric acid, tartaric acid, lactic acid, phosphoric acid, acetic acid, boric acid, trolamine, ammonia, Tris(hydroxymethyl)aminomethane (TRIS), and pharmaceutically acceptable salts thereof.

**11.** The topical formulation of claim 1, further comprising a cosmesis enhancer.

**12.** The topical formulation of claim 11, wherein the cosmesis enhancer is a silicone-based cosmesis enhancer selected from the group consisting of cyclomethicone, dimethicone, and mixtures thereof.

**13.** The topical formulation of claim 1, further comprising an emulsion stabilizer.

**14.** The topical formulation of claim 13, wherein the emulsion stabilizer is selected from the group consisting of cetostearyl alcohol, cetyl alcohol, stearyl alcohol, myristyl alcohol, glyceryl monostearate, propylene glycol stearate, polyoxyl 20 cetostearyl ether, and mixtures thereof.

**15.** The topical formulation of claim 1, wherein the topical formulation has a pH of about 3.5 to about 4.5.

**16.** The topical formulation of claim 1, wherein the radezolid is a radezolid mesylate salt.

**17.** A topical formulation comprising about 0.2 to about 5 wt.% radezolid mesylate salt, about 7.5 to about 15 wt.% propylene glycol, about 0.1 to about 15 wt.% cetostearyl alcohol, about 1 to about 15 wt.% glycerin, about 0.5 to about 3.0 wt.% cyclomethicone, about 0.1 to about 15 wt.% polyoxyl 20 cetostearyl ether, about 0.5 to about 3.0 wt.% dimethicone, about 0.01 to about 1 wt.% methylparaben, about 0.01 to about 1.0 wt.% anhydrous citric acid, about 0.01 to about 1 wt.% propylparaben, q.s. NaOH to about pH 3.8 to 4.2, and q.s. purified water to 100%.

**18.** The topical formulation of claim 1, wherein the topical formulation is in the form of a cream.

**19.** The topical formulation of any one of claims 1-18 for use in treating a skin infection caused or mediated by *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* or *Staphylococcus aureus* (including *Methicillin-resistant Staphylococcus aureus* (MRSA)).

**20.** The topical formulation of claim 19 for use in treating a skin infection, wherein the skin infection is selected from acne vulgaris, rosacea, impetigo, otitis externa, bacterial conjunctivitis, and bacterial vaginosis.

## Patentansprüche

1. Topische Formulierung, umfassend:

   Radezolid oder ein pharamzeutisch unbedenkliches Salz oder Tautomer davon und
   einen Penetrationsverstärker.

2. Topische Formulierung nach Anspruch 1, wobei der Penetrationsverstärker ausgewählt ist aus der Gruppe bestehend aus Propylenglykol, Hexylenglykol, Diethylenglykolmonoethylether, Dimethylsulfoxid, Ethanol, Isopropanol, Ölsäure, Laurocapram, d-Limonen, Essigsäureethylester und Mischungen davon.

3. Topische Formulierung nach Anspruch 1, weiterhin umfassend:

   einen Emulsionsstabilisator,
   ein feuchtigkeitsspendendes Mittel,
   einen Cosmeseverstärker,
   einen Konservierungsstoff,
   einen Puffer,
   ein den pH-Wert modifizierendes Mittel und
   Wasser.

4. Topische Formulierung nach Anspruch 1, weiterhin umfassend ein feuchtigkeitsspendendes Mittel.

5. Topische Formulierung nach Anspruch 4, wobei das feuchtigkeitsspendende Mittel ausgewählt ist aus der Gruppe bestehend aus Glycerin, Harnstoff, Milchsäure, Glykolsäure, Hyaluronsäure, Pyrrolidoncarbonsäure und Mischungen davon.

6. Topische Formulierung nach Anspruch 1, weiterhin umfassend einen Konservierungsstoff.

7. Topische Formulierung nach Anspruch 6, wobei der Konservierungsstoff ausgewählt ist aus der Gruppe bestehend aus Methylparaben, Propylparaben, Benzylalkohol, Benzalkoniumchlorid, Cetylpyridiniumchlorid, Benzoesäure, Natriumbenzoat, Kaliumsorbat und Mischungen davon.

8. Topische Formulierung nach Anspruch 1, weiterhin umfassend ein en pH-Wert modifizierendes Mittel.

9. Topische Formulierung nach Anspruch 1, weiterhin umfassend einen Puffer.

10. Topische Formulierung nach Anspruch 9, wobei der Puffer ausgewählt ist aus der Gruppe bestehend aus Citronensäure, Weinsäure, Milchsäure, Phosphorsäure, Essigsäure, Borsäure, Trolamin, Ammoniak, Tris(hydroxymethyl)aminomethan (TRIS) und pharmazeutisch unbedenklichen Salzen davon.

11. Topische Formulierung nach Anspruch 1, weiterhin umfassend einen Cosmeseverstärker.

12. Topische Formulierung nach Anspruch 11, wobei der Cosmeseverstärker ausgewählt ist aus der Gruppe bestehend aus Cyclomethicon, Dimethicon und Mischungen davon.

13. Topische Formulierung nach Anspruch 1, weiterhin umfassend einen Emulsionsstabilisator.

14. Topische Formulierung nach Anspruch 13, wobei der Emulsionsstabilisator ausgewählt ist aus der Gruppe bestehend aus Cetostearylalkohol, Cetylalkohol, Stearylalkohol, Myristylalkohol, Glycerylmonostearat, Propylenglykolstearate, Polyoxyl-20-cetostearylether und Mischungen davon.

15. Topische Formulierung nach Anspruch 1, die topische Formulierung einen pH-Wert von etwa 3,5 bis etwa 4,5 aufweist.

16. Topische Formulierung nach Anspruch 1, wobei es sich bei dem Radezolid um ein Radezolidmesylatsalz handelt.

17. Topische Formulierung, umfassend etwa 0,2 bis etwa 5 Gew.-% Radezolidmesylatsalz, etwa 7,5 bis etwa 15 Gew.-

% Propylenglykol, etwa 0,1 bis etwa 15 Gew.-% Cetostearylalkohol, etwa 1 bis etwa 15 Gew.-% Glycerin, etwa 0,5 bis etwa 3,0 Gew.-% Cyclomethicon, etwa 0,1 bis etwa 15 Gew.-% Polyoxyl-20-cetostearylether, etwa 0,5 bis etwa 3,0 Gew.-% Dimethicon, etwa 0,01 bis etwa 1 Gew.-% Methylparaben, etwa 0,01 bis etwa 1,0 Gew.-% wasserfreie Citronensäure, etwa 0,01 bis etwa 1 Gew.-% Propylparaben, q.s. NaOH bis etwa pH 3,8 bis 4,5, und q.s. gereinigtes Wasser ad 100%.

**18.** Topische Formulierung nach Anspruch 1, wobei die topische Formulierung in Form einer Creme vorliegt.

**19.** Topische Formulierung nach einem der Ansprüche 1-18 zur Verwendung bei der Behandlung einer durch *Streptococcus pyogenes, Streptococcus agalactiae, Haemophilus influenza, Trichomonas vaginalis, Klebsiella* sp., *Enterobacter* sp., *Proteus* sp., *Propionibacterium acnes, Gardnerella vaginalis* oder *Staphylococcus aureus* (einschließlich methicillinresistentem *Staphylococcus aureus* (MRSA)) verursachten oder vermittelten Hautinfektion.

**20.** Topische Formulierung nach Anspruch 19 zur Verwendung bei der Behandlung einer Hautinfektion, wobei die Hautinfektion ausgewählt ist aus Acne vulgaris, Rosazea, Impetigo, Otitis externa, bakterieller Bindehautentzündung und bakterieller Vaginose.

## Revendications

**1.** Formulation topique comprenant :

du radézolide, ou un sel ou une forme tautomère pharmaceutiquement acceptable correspondant(e) ; et
un agent d'amélioration de la pénétration.

**2.** Formulation topique selon la revendication 1, l'agent d'amélioration de la pénétration étant choisi dans le groupe constitué par le propylèneglycol, l'hexylèneglycol, le monoéthyléther de diéthylèneglycol, le diméthylsulfoxyde, l'éthanol, l'isopropanol, l'acide oléique, le laurocaprame, le d-limonène, l'acétate d'éthyle, et des mélanges correspondants.

**3.** Formulation topique selon la revendication 1 comprenant en outre :

un stabilisateur d'émulsion ;
un agent hydratant ;
un agent d'amélioration de l'esthétique ;
un conservateur ;
un agent tampon ;
un agent de modification du pH ; et
de l'eau.

**4.** Formulation topique selon la revendication 1 comprenant en outre un agent hydratant.

**5.** Formulation topique selon la revendication 4, l'agent hydratant étant choisi dans le groupe constitué par la glycérine, l'urée, l'acide lactique, l'acide glycolique, l'acide hyaluronique, l'acide pyrrolidone carboxylique, et des mélanges correspondants.

**6.** Formulation topique selon la revendication 1, comprenant en outre un conservateur.

**7.** Formulation topique selon la revendication 6, le conservateur étant choisi dans le groupe constitué par le méthylparabène, le propylparabène, l'alcool benzylique, le chlorure de benzalkonium, le chlorure de cétylpyridinium, l'acide benzoïque, le benzoate de sodium, le sorbate de potassium et des mélanges correspondants.

**8.** Formulation topique selon la revendication 1, comprenant en outre un agent de modification du pH.

**9.** Formulation topique selon la revendication 1, comprenant en outre un agent tampon.

**10.** Formulation topique selon la revendication 9, l'agent tampon étant choisi dans le groupe constitué par l'acide citrique, l'acide tartrique, l'acide lactique, l'acide phosphorique, l'acide acétique, l'acide borique, la trolamine, l'ammoniaque,

le Tris(hydroxyméthyl)aminométhane (TRIS), et des sels pharmaceutiquement acceptables correspondants.

**11.** Formulation topique selon la revendication 1, comprenant en outre un agent d'amélioration de l'esthétique.

**12.** Formulation topique selon la revendication 11, l'agent d'amélioration de l'esthétique étant un agent d'amélioration de l'esthétique à base de silicone choisi dans le groupe constitué par une cyclométhicone, une diméthicone et des mélanges correspondants.

**13.** Formulation topique selon la revendication 1, comprenant en outre un stabilisateur d'émulsion.

**14.** Formulation topique selon la revendication 13, le stabilisateur d'émulsion étant choisi dans le groupe constitué par l'alcool cétostéarylique, l'alcool cétylique, l'alcool stéarylique, l'alcool myristylique, le monostéarate de glycéryle, le stéarate de propylèneglycol, l'éther cétostéarylique de polyoxyle 20, et des mélanges correspondants.

**15.** Formulation topique selon la revendication 1, la formulation topique possédant un pH d'environ 3,5 à environ 4,5.

**16.** Formulation topique selon la revendication 1, le radézolide étant un sel de mésylate de radézolide.

**17.** Formulation topique comprenant environ 0,2 à environ 5 % en poids de sel de mésylate de radézolide, environ 7,5 à environ 15 % en poids de propylène glycol, environ 0,1 à environ 15 % en poids d'alcool cétostéarylique, environ 1 à environ 15 % en poids de glycérine, environ 0,5 à environ 3,0 % en poids de cyclométhicone, environ 0,1 à environ 15 % en poids d'éther cétostéarylique de polyoxyle 20, environ 0,5 à environ 3,0 % en poids de diméthicone, environ 0,01 à environ 1 % en poids de méthylparabène, environ 0,01 à environ 1 % en poids d'acide citrique anhydre, environ 0,01 à environ 1 % en poids de propylparabène, q.s. de NaOH à environ pH 3,8 à 4,2, et q.s. d'eau purifiée jusqu'à 100 %.

**18.** Formulation topique selon la revendication 1, la formulation topique étant sous la forme d'une crème.

**19.** Formulation topique selon l'une quelconque des revendications 1 à 18, pour une utilisation dans le traitement d'une infection cutanée causée ou médiée par *Streptococcus pyogenes, Streptococcus agalactiae, Haemophilus influenza, Trichomonas vaginalis, Klebsiella sp., Enterobacter sp., Proteus sp., Propionibacterium acnes, Gardnerella vaginalis,* ou *Staphylococcus aureus* (y compris *Staphylococcus aureus résistant à la Méticilline* (MRSA)).

**20.** Formulation topique selon la revendication 19 pour une utilisation dans le traitement d'une infection cutanée, l'infection cutanée étant choisie parmi l'acné vulgaire, la rosacée, l'impétigo, l'otite externe, la conjonctivite bactérienne et la vaginose bactérienne.

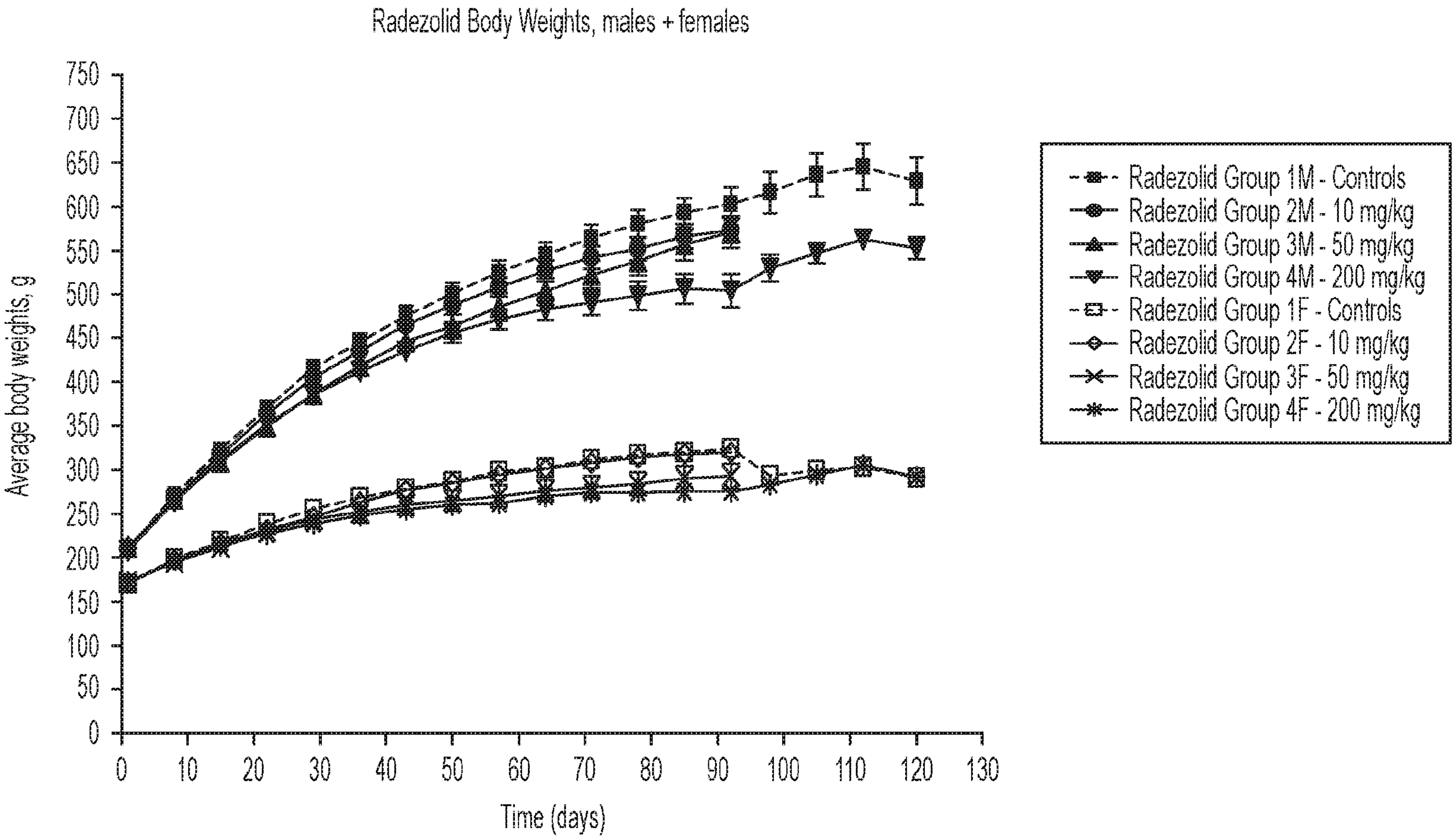
Radezolid Body Weights, males + females
Average body weights, g
Time (days)
0
50
100
150
200
250
300
350
400
450
500
550
600
650
700
750
0
10
20
30
40
50
60
70
80
90
100
110
120
130
Radezolid Group 1M - Controls
Radezolid Group 2M - 10 mg/kg
Radezolid Group 3M - 50 mg/kg
Radezolid Group 4M - 200 mg/kg
Radezolid Group 1F - Controls
Radezolid Group 2F - 10 mg/kg
Radezolid Group 3F - 50 mg/kg
Radezolid Group 4F - 200 mg/kg

FIG. 1

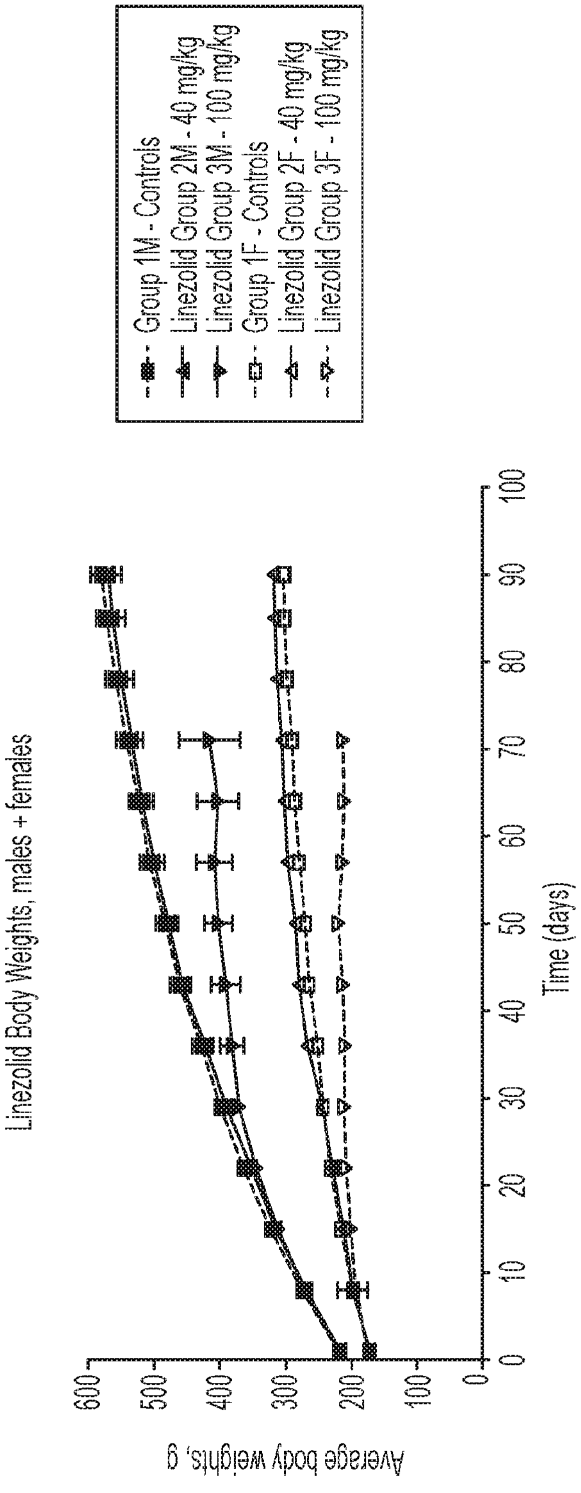
Linezolid Body Weights, males + females
Average body weights, g
600
500
400
300
200
100
0
0
10
20
30
40
50
60
70
80
90
100
Time (days)
Group 1M - Controls
Linezolid Group 2M - 40 mg/kg
Linezolid Group 3M - 100 mg/kg
Group 1F - Controls
Linezolid Group 2F - 40 mg/kg
Linezolid Group 3F - 100 mg/kg

FIG. 2

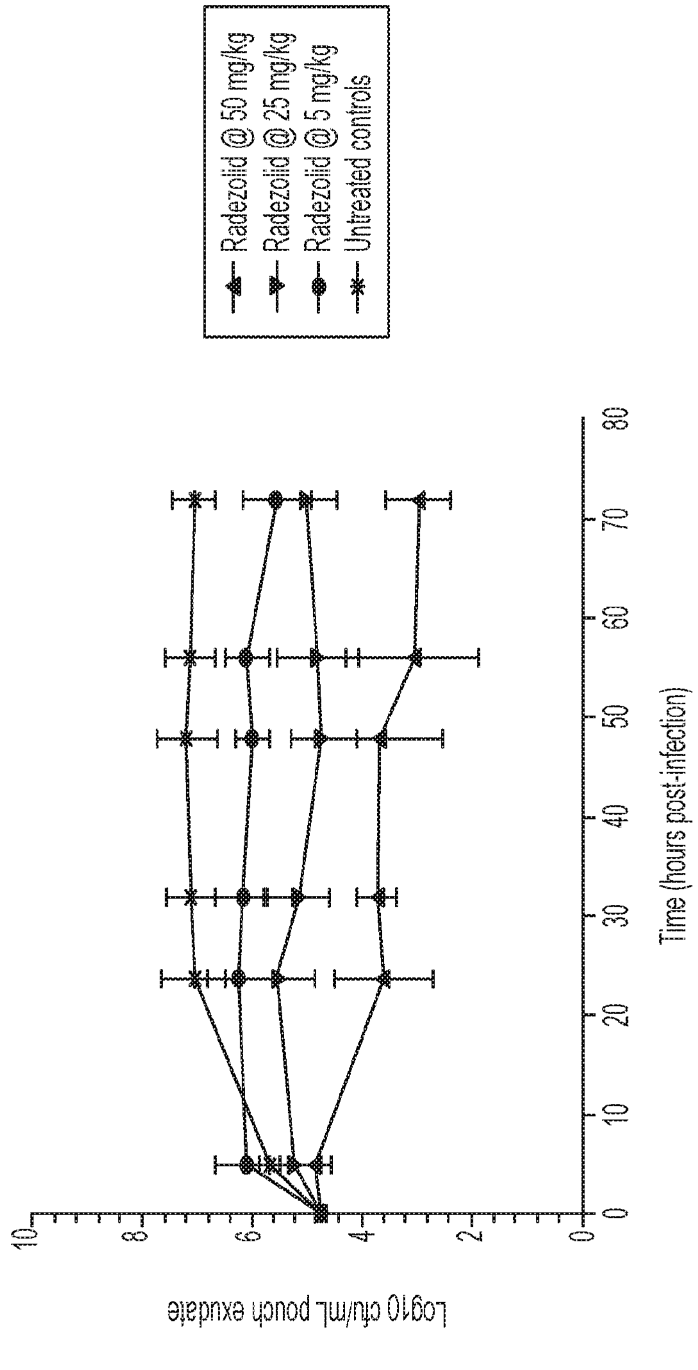
Log10 cfu/mL pouch exudate
Time (hours post-infection)
Radezolid @ 50 mg/kg
Radezolid @ 25 mg/kg
Radezolid @ 5 mg/kg
Untreated controls
0
2
4
6
8
10
0
10
20
30
40
50
60
70
80

FIG. 3

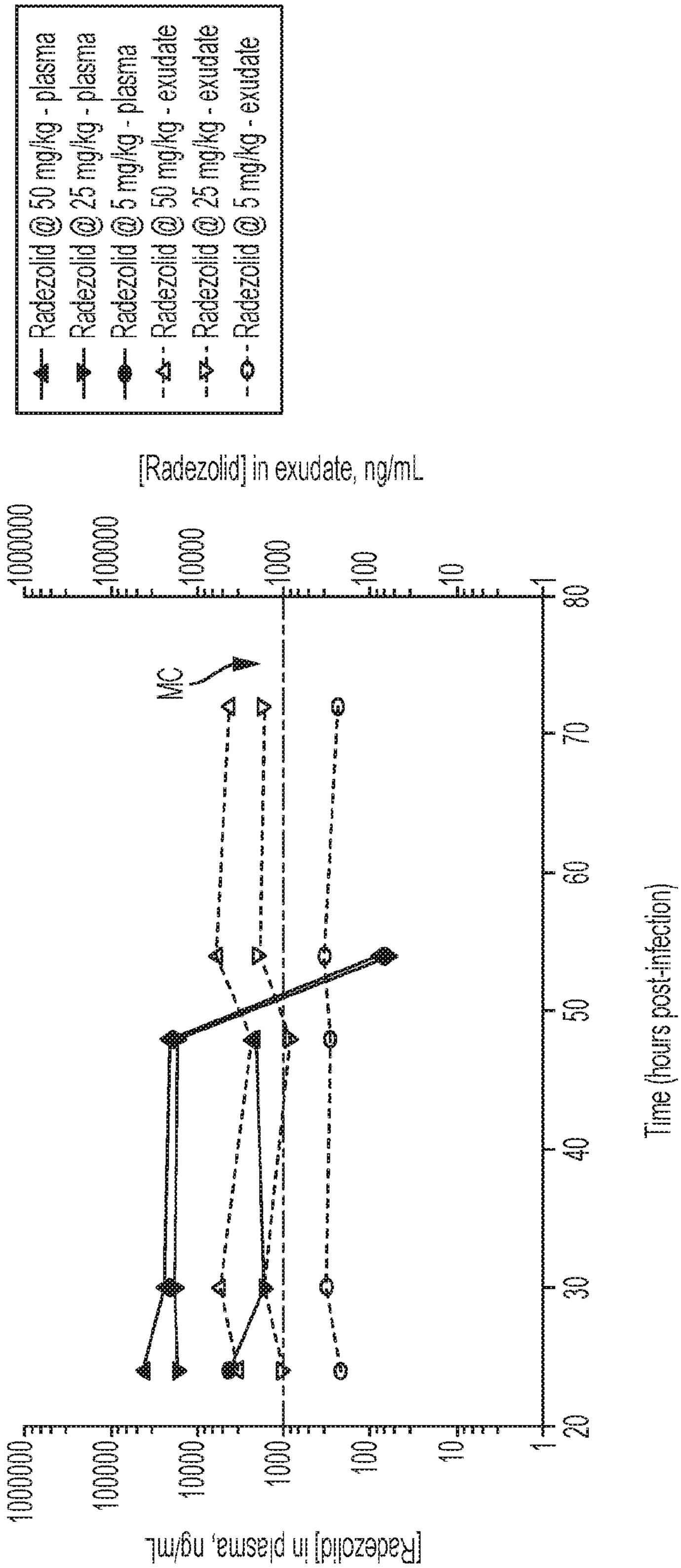
Radezolid @ 50 mg/kg - plasma
Radezolid @ 25 mg/kg - plasma
Radezolid @ 5 mg/kg - plasma
Radezolid @ 50 mg/kg - exudate
Radezolid @ 25 mg/kg - exudate
Radezolid @ 5 mg/kg - exudate
[Radezolid] in exudate, ng/mL
[Radezolid] in plasma, ng/mL
Time (hours post-infection)
MIC
1000000
100000
10000
1000
100
10
1
20
30
40
50
60
70
80

FIG. 4

| | HCl Gel 1 | | | | HCl Gel 2 | | | | HCl Gel 3 | | | |
|---|---|---|---|---|---|---|---|---|---|---|---|---|
| Conditions | API % | pH | Viscosity (cP) | Appearance | API % | pH | Viscosity (cP) | Appearance | API % | pH | Viscosity (cP) | Appearance |
| Zero time | 101.1 | 7.41 | 28,400 | White to off-white homo-geneous gel | 100.0 | 7.26 | 9,200 | White to off-white homo-geneous gel | 99.9 | 7.27 | 28,600 | White to off-white homo-geneous gel |
| 3 months, 2-8 °C | 100.1 | 7.41 | N/A | No change. | 100.2 | 7.40 | N/A | No change. | 99.7 | 7.35 | N/A | No change. |
| 1 month, 25 °C | 98.5* | 7.40 | 27,500 | No change. | 99.0* | 7.29 | 9,400 | No change. | 98.9* | 7.31 | 28,800 | No change. |
| 3 months, 25 °C | 98.2 | 7.35 | 26,100 | No change. | 98.9 | 7.27 | 9,900 | No change. | 98.7 | 7.26 | 26,500 | No change. |
| 6 months, 25 °C | 98.1 | 7.36 | 26,800 | No change. | 98.5 | 7.24 | 9,200 | No change. | 99.1 | 7.28 | 26,800 | No change. |
| 1 month, 40 °C | 98.5* | 7.36 | 26,400 | No change. | 99.0* | 7.25 | 9,700 | No change. | 98.9* | 7.21 | 27,900 | No change. |
| 3 months, 40 °C | 96.9 | 7.15 | 27,700 | No change. | 96.5 | 7.19 | 9,600 | No change. | 96.6 | 7.14 | 27,800 | No change. |
| 6 months, 40 °C | 97.1 | 7.25 | 24,300 | No change. | 96.4 | 7.20 | 8,500 | No change. | 98.2 | 7.13 | 24,900 | No change. |

*New peaks observed at approximately 0.24-0.60 area % with respect to main peak

FIG. 5

| | Mesylate Gel 1 | | | | Mesylate Gel 2 | | | | Mesylate Cream | | | |
|---|---|---|---|---|---|---|---|---|---|---|---|---|
| Conditions | API % | pH | Viscosity (cP) | Appearance | API % | pH | Viscosity (cP) | Appearance | API % | pH | Viscosity (cP) | Appearance |
| Zero time | 100.2 | 3.84 | 24,800 | Clear to translucent homo-geneous gel | 100.3 | 3.85 | 24,600 | Clear to translucent homo-geneous gel | 98.3 | 4.16 | 66,000 | White to off-white homo-geneous cream |
| 1 month, 25 °C | 101.2 | 3.93 | 24,900 | No change. | 99.9 | 3.94 | 24,700 | No change. | 99.8 | 4.15 | 66,200 | No change. |
| 3 months, 25 °C | 99.5 | 3.88 | 24,900 | No change. | 99.3 | 3.94 | 24,700 | No change. | 99.9 | 4.00 | 67,500 | No change. |
| 6 months, 25 °C | 99.7 | 3.93 | 24,800 | No change. | 99.7 | 3.99 | 24,200 | No change. | 99.5 | 3.92 | 76,000 | No change. |
| 1 month, 40 °C | 102.9 | 3.89 | 24,900 | No change. | 100.1 | 3.86 | 23,800 | No change. | 99.7 | 4.05 | 67,500 | No change. |
| 3 months, 40 °C | 99.1 | 3.85 | 24,900 | No change. | 99.5 | 3.96 | 23,800 | No change. | 100.1 | 3.99 | 66,000 | No change. |
| 6 months, 40 °C | 99.7 | 3.91 | 24,400 | No change. | 100.1 | 3.92 | 24,300 | No change. | 101.0 | 4.02 | 75,100 | No change. |

FIG. 6

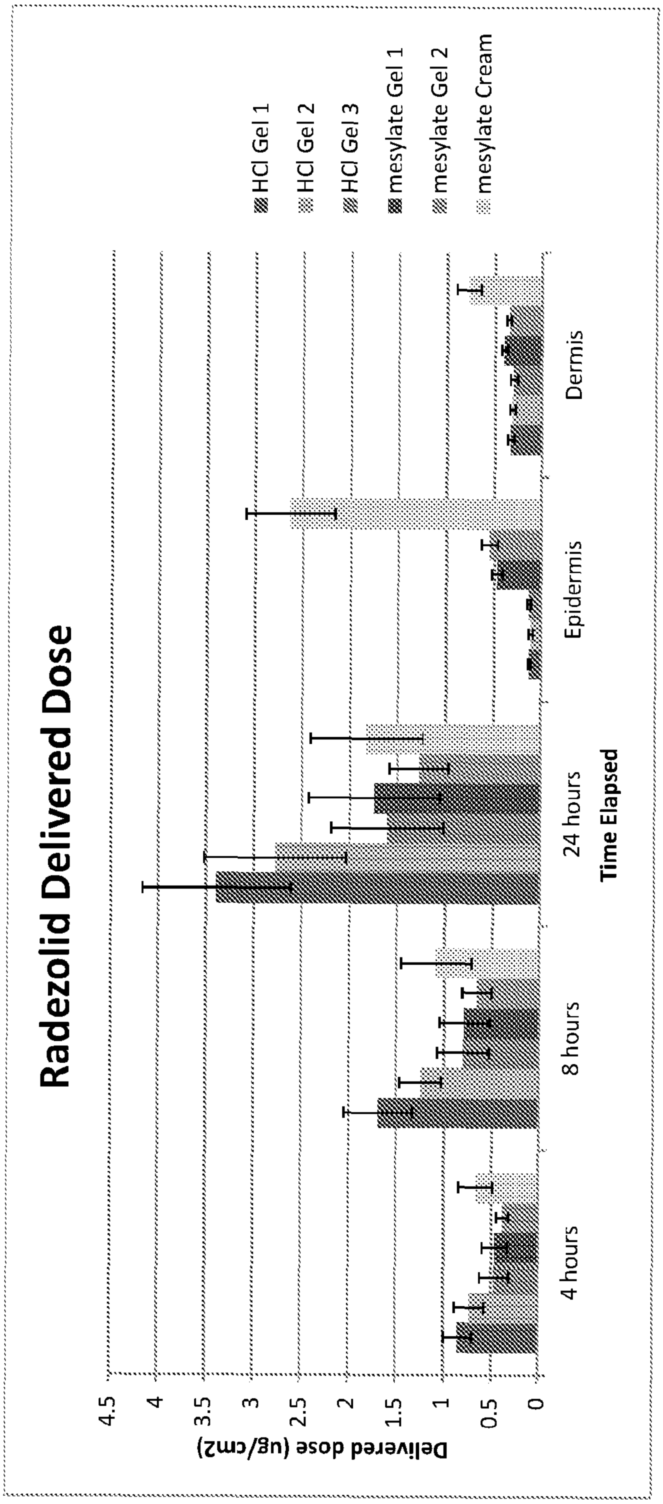
Radezolid Delivered Dose
Delivered dose (ug/cm2)
4.5
4
3.5
3
2.5
2
1.5
1
0.5
0
4 hours
8 hours
24 hours
Epidermis
Dermis
Time Elapsed
HCl Gel 1
HCl Gel 2
HCl Gel 3
mesylate Gel 1
mesylate Gel 2
mesylate Cream

FIG. 7

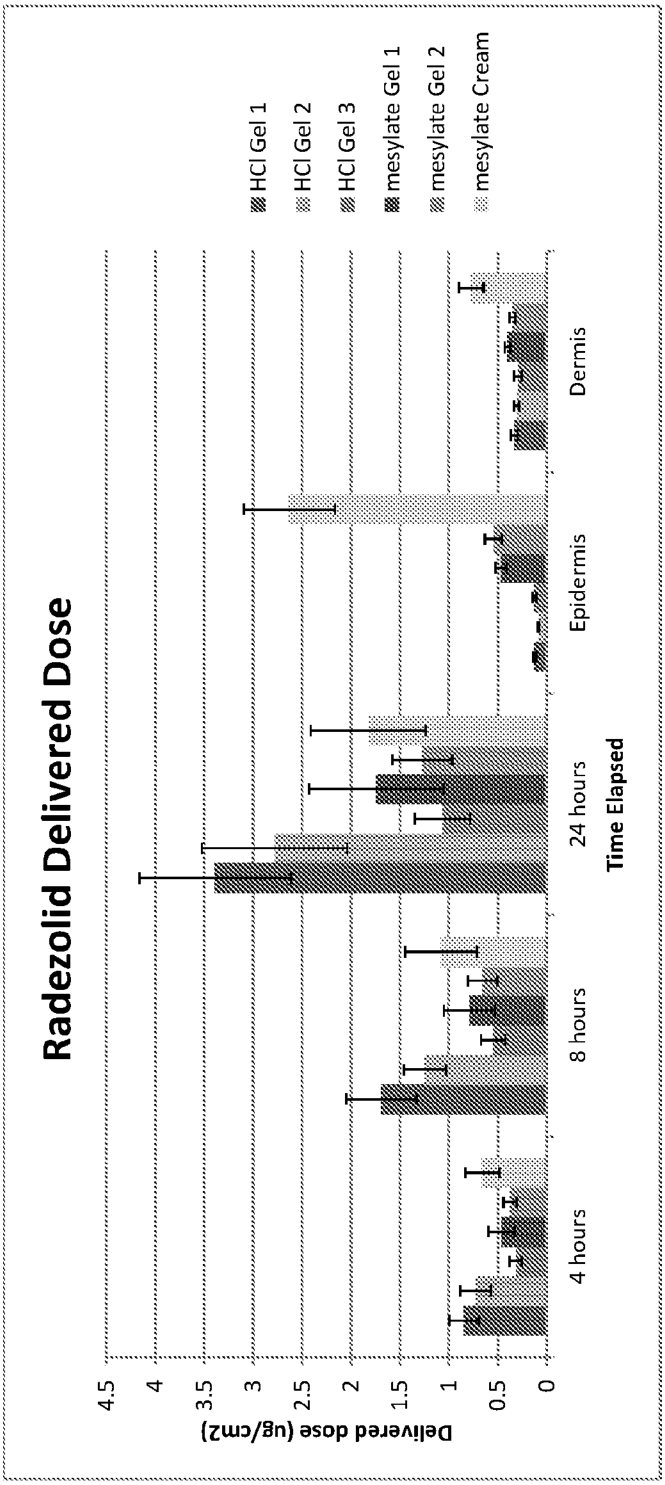

Radezolid Delivered Dose
Delivered dose (ug/cm2)
4.5
4
3.5
3
2.5
2
1.5
1
0.5
0
4 hours
8 hours
24 hours
Epidermis
Dermis
Time Elapsed
HCl Gel 1
HCl Gel 2
HCl Gel 3
mesylate Gel 1
mesylate Gel 2
mesylate Cream


FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62337636
- US 3535422 A, Cox
- GB 1185685 A, Fisher
- GB 1163004 A
- GB 1407937 A
- US 4960764 A, Figueroa
- US 4254105 A, Fukuda
- US 4985459 A, Sunshine
- US 4800197 A, Kowcz
- US 4919934 A, Deckner
- US 5087445 A, Haffey
- US 5073372 A, Turner
- US 5073371 A, Turner
- US 4937370 A, Sabatelli
- US 4999186 A, Sabatelli
- US 4891228 A, Thaman
- US 4891227 A, Thaman
- US 4693623 A, Schwartzman
- US 4620648 A, Schwartzman
- US 3669323 A, Harker
- US 3418055 A, Schwartzman
- US 3410645 A, Schwartzman


### Non-patent literature cited in the description


- **C. HUBER et al.** *Arch. Derm. Res.,* 1977, vol. 257, 293-297
- **LEVINE et al.** *Ohio State Med. J.,* 1969, vol. 65, 492
- **BROGDNE et al.** *Drugs,* 1974, vol. 4, 417
- **POOLE et al.** *Arch Derm.,* 1972, vol. 102, 400
- **EAGLSTEIN.** *Arch Derm.,* 1968, vol. 97, 527
- **PACE.** *Can. Med. Assoc. J.,* 1965, vol. 93, 252
- **VASARINSH.** *Arch. Derm.,* 1968, vol. 98, 183
- **MYSLIBORSKI et al.** *AFP,* 1977, vol. 15, 86
- **NARE.** *Br. J. Clin. Prac,* 1975, vol. 29, 63
- **FULTON et al.** *Arch. Derm.,* 1974, vol. 10, 83
- **WILKINSON et al.** *Can. Med. Assoc. J.,* 1966, vol. 95, 28
- **EADY E.A. et al.** The effects of acne treatment with a combination of benzoyl peroxide and erythromycin on skin carriage of erythromycinresistant propionibacteria. *Br. J. Dermatol.,* 1996, vol. 134, 107-113
- **MCLEAN, N. W. ; MCGROARTY, J. A.** *Appl. Environ. Microbiol.,* 1996, vol. 62 (3), 1089-1092
- **NAGARAJA, P.** *Indian J. Med. Microbiol.,* 2008, vol. 26 (2), 155-157
- **TOMUSIAK, A. et al.** *Ginekol. Pol.,* 2011, vol. 82 (12), 900-904
- **ESCHENBACH, D. A.** *Clin. Infectious Dis.,* 2007, vol. 44 (2), 220-221
- **TAN, A.W. ; TAN, H.H.** Acne vulgaris,: a review of antibiotic therapy. *Expert Opin. Pharmacother.,* March 2005, vol. 6 (3), 409-418
- **OPRICA, C. et al.** European surveillance study on the antibiotic susceptibility of Propionibacterium acnes. *Clinical Microbiology and Infection,* March 2005, vol. 11 (3), 204-312
- **GOLDSTEIN, E. et al.** Comparative In Vitro Activities of retapamulin (SB-275833) against 141 Clinical isolates of propionibacterium spp., Including 117 P. acnes Isolates. *Antimicrobial Agents and Chemotherapy,* January 2006, vol. 50 (1), 379-381
- **CHOU ; TALALAY.** *Adv. Enzyme Regul.,* 1984, vol. 22, 27-55
- Remington: The Science and Practice of Pharmacy. Pharmaceutical Press, 2012
- **DECKER, A. ; GRABER, E. M.** *J. Clin. Aesthet. Dermatol.,* 2012, vol. 5 (5), 32-40
- Vo1. 1 Compressed Solid Products, Vol. 2 Uncompressed Drug Products, Vol. 3 Liquid Products, Vol. 4 Semi-Solid Products, Vol. 5 Over the Counter Products, and Vol. 6 Sterile Products. Handbook of Pharmaceutical Formulations. CRC Press, 27 April 2004, vol. 1-6
- Sun Products Formulary. *Cosmetics & Toiletries,* December 1990, vol. 105, 122-139
- Sun Products Formulary. *Cosmetics & Toiletries,* March 1987, vol. 102, 117-136
- McCutcheon's, Detergents and Emulsifiers. Allured Publishing Corporation, 1986
- **SEGARIN et al.** Cosmetics Science and Technology. 189
- *Federal Register,* 25 August 1978, vol. 43 (166), 38206-38269
- **LEMAIRE et al.** *AAC,* 2010, vol. 54 (6), 6549-59